# EUROPEAN PATENT SPECIFICATION

(11) **EP 3 539 990 B1**
(45) Date of publication and mention of the grant of the patent: **08.09.2021**
(21) Application number: 19155820.4
(22) Date of filing: 15.07.2015
(51) Int. Cl.: C07K 16/32, A61P 35/00

(54) **HER3 INHIBITION IN LOW-GRADE SEROUS CANCERS**
HER3-INHIBITION BEI SERÖSEM LOW-GRADE OVARIALKARZINOM
INHIBITION DE HER3 DANS LES CANCERS SÉREUX À ÉVOLUTION LENTE

(30) Priority: 16.07.2014 US 201462025321 P
(43) Date of publication of application: 18.09.2019
(62) Divisional of application: 15747648.2
(73) Proprietor: Dana-Farber Cancer Institute, Inc., Boston, MA 02215 (US)
(72) Inventor: LIVINGSTONE, David, Brookline, MA 02446 (US); LIU, Joyce, Newton, MA 02459 (US); DRAPKIN, Ronny, Merion Station, PA 19066 (US)
(74) Representative: D Young & Co LLP

(56) References cited:
- WO-A1-2012/022814
- WO-A2-2013/003037
- JOHN FARLEY ET AL: "Selumetinib in women with recurrent low-grade serous carcinoma of the ovary or peritoneum: an open-label, single-arm, phase 2 study", THE LANCET ONCOLOGY, vol. 14, no. 2, 1 February 2013 (2013-02-01), pages 134-140, XP055213332, ISSN: 1470-2045, DOI: 10.1016/S1470-2045(12)70572-7
- RACHEL N. GRISHAM ET AL: "Bevacizumab Shows Activity in Patients With Low-Grade Serous Ovarian and Primary Peritoneal Cancer", INTERNATIONAL JOURNAL OF GYNECOLOGICAL CANCER, vol. 24, no. 6, 1 July 2014 (2014-07-01), pages 1010-1014, XP055213199, ISSN: 1048-891X, DOI: 10.1097/IGC.0000000000000190
- RUSSELL VANG ET AL: "Ovarian Low-grade and High-grade Serous Carcinoma", ADVANCES IN ANATOMIC PATHOLOGY, vol. 16, no. 5, 1 September 2009 (2009-09-01), pages 267-282, XP055213006, ISSN: 1072-4109, DOI: 10.1097/PAP.0b013e3181b4fffa
- David M Gershenson: "The life and times of low-grade serous carcinoma of the ovary", American Society of Clinical Oncology educational book / ASCO. American Society of Clinical Oncology. Meeting, 1 January 2013 (2013-01-01), XP055213325, United States Retrieved from the Internet: URL:http://www.ncbi.nlm.nih.gov/pubmed/237 14500

## Description

### BACKGROUND

### 1. Technical Field

The invention relates to an agent for use in treating low-grade serous ovarian cancers by inhibiting signaling of an EGFR family member.

### 2. Background Information

Ovarian cancer is the fifth leading cause of cancer death in women in the United States, with an estimated 22,000 cases and 14,000 deaths occurring in 2014 (Siegel et al. Cancer Statistics, 2014. CA Cancer J Clin. 2014 Jan-Feb; 64(1):9-29. Doi:10.3322/caac.21208. Epub 2014 Jan 7). Although primary treatment with surgery and platinum-based chemotherapy can be effective, the disease frequently recurs and becomes increasingly resistant to therapy (Modesitt, 2007, Expert Opin Pharmacother, 2293). To date, identification of successful targeted therapies in ovarian cancer has been limited.

### BRIEF SUMMARY

The invention relates to an agent for use in treating low-grade serous ovarian cancer as defined in claim 1. Also disclosed herein are methods of treating cancer. The methods can include administering to the subject a therapeutically effective amount of an antibody or fragment thereof that specifically binds to an Epidermal Growth Factor Receptor (EGFR) family member, wherein the cancer is low-grade serous ovarian cancer. In one embodiment, the EGFR family member is HER3 and the antibody or fragment thereof is a Her3 antibody or fragment thereof. In one embodiment, the Her3 antibody or fragment thereof binds to a conformational epitope comprising amino acid residues within domain 2 and domain 4 of HER3 and blocks both ligand-dependent and ligand-independent signal transduction.

In some aspects, methods of treating cancer include administering to the subject a therapeutically effective amount of an agent that inhibits signaling of an EGFR family member in the cancer, wherein the cancer is low-grade serous ovarian cancer. The agent can include a small molecule, a polypeptide, an antibody, an antisense oligonucleotide, or an siRNA molecule.

Also disclosed herein is use of an agent that inhibits signaling of an EGFR family member the manufacture of a medicament for use in the treatment of low-grade serous ovarian cancer. Farley et al,(2013) The Lancet Oncology,14 (2): 134-140 describes the use of Selumetinib, a MEK inhibitor, to treat LGSC with possible previous chemotherapy or treatment with MEK inhibitors.

### BRIEF DESCRIPTION OF THE DRAWINGS

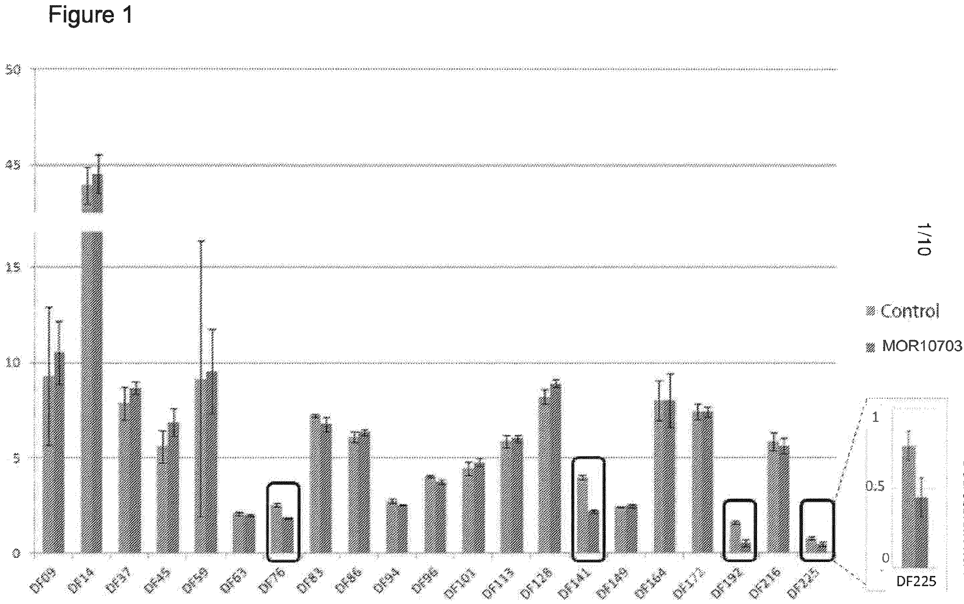

Figure 1 illustrates the effect of the anti-Her3 monoclonal antibody MOR10703 on 21 different primary ovarian cultures. Four primary cell packs (DF76, DF141, DF192, and DF225) had significant decrease in proliferation after 6 days of continuous exposure to MOR10703 at a concentration of 1:1000.

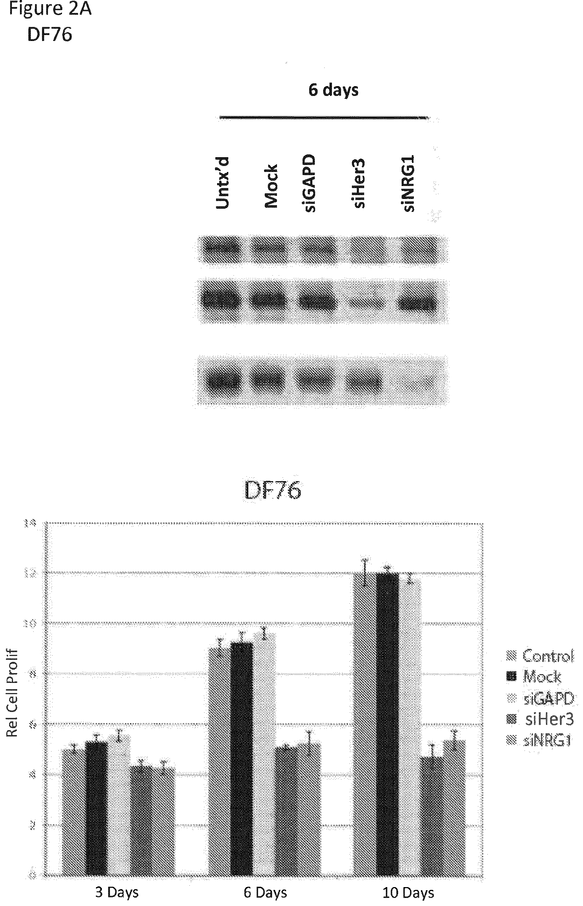

Figures 2A-2B illustrate the effect of RNAi (siRNA) directed against Her3 and NRG1 in (2A) DF76 and (2B) DF141 cells. (2A) 6 days after initial transfection, siHer3 resulted in significant decrease in Her3 protein expression and decreased Her3 phosphorylation, while siNRG significantly decreased NRG1 protein expression and had a moderate effect on Her3 phosphorylation. siRNA against either Her3 or NRG1 resulted in significant proliferation decrease in DF76 cells. (2B) 3 days after initial transfection, siHer3 resulted in decrease in Her3 protein expression and slight decrease in Her3 phosphorylation in DF141 cells. 6 days after initial transfection, siHer3 resulted in significant decrease in Her3 protein expression and significant decrease in Her3 phosphorylation in DF141 cells. siRNA also resulted in significant decrease in NRG1 expression at 3 days. Effects on Her3 phosphorylation at 3 days and 6 days by siNRG1 could not be observed due to insufficient lysate at those time points due to toxicity.

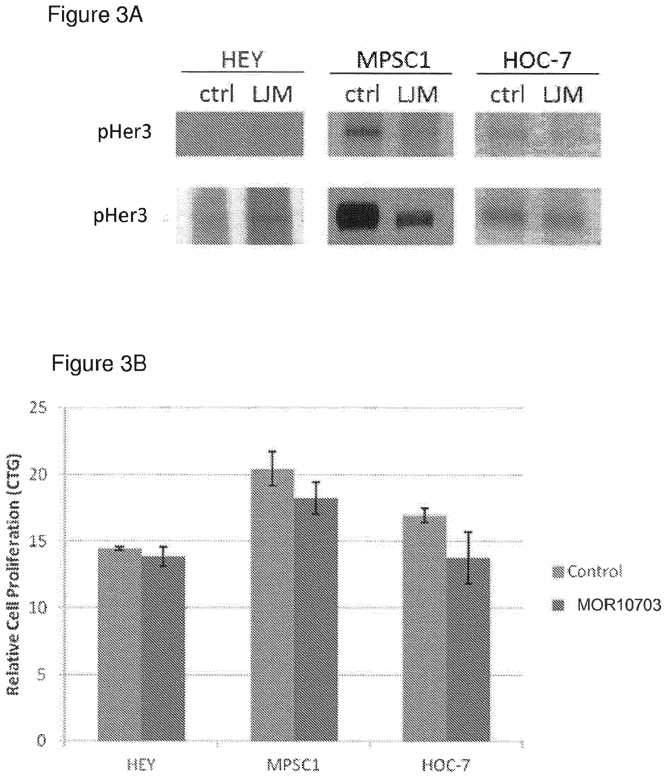

Figures 3A-3B illustrate the effect on Her3 expression and phosphorylation in low grade serous cell lines by MOR10703. (3A) MOR10703 reduced Her3 expression and phosphorylation in both MPSC1 and HOC-7 cells. HEY cells had very low amounts of Her3 expression and no evidence of Her3 phosphorylation. (3B) MOR10703 had no effect on proliferation of HEY, MPSC1, or HOC-7 cells.

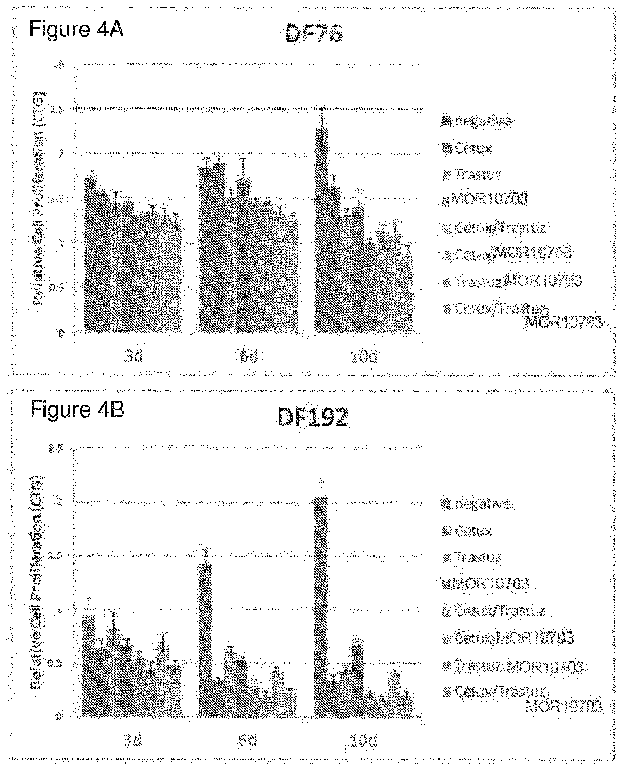

Figures 4A-4D illustrates the effect of monoclonal antibodies directed against EGFR, Her-2, and Her3 in primary DF low-grade serous cell packs. Although variability exists, each of the low-grade DF cell packs exhibits some degree of sensitivity against cetuximab, trastuzumab, and MOR10703. Greater effect was seen when antibodies were combined than with any given antibody in isolation.

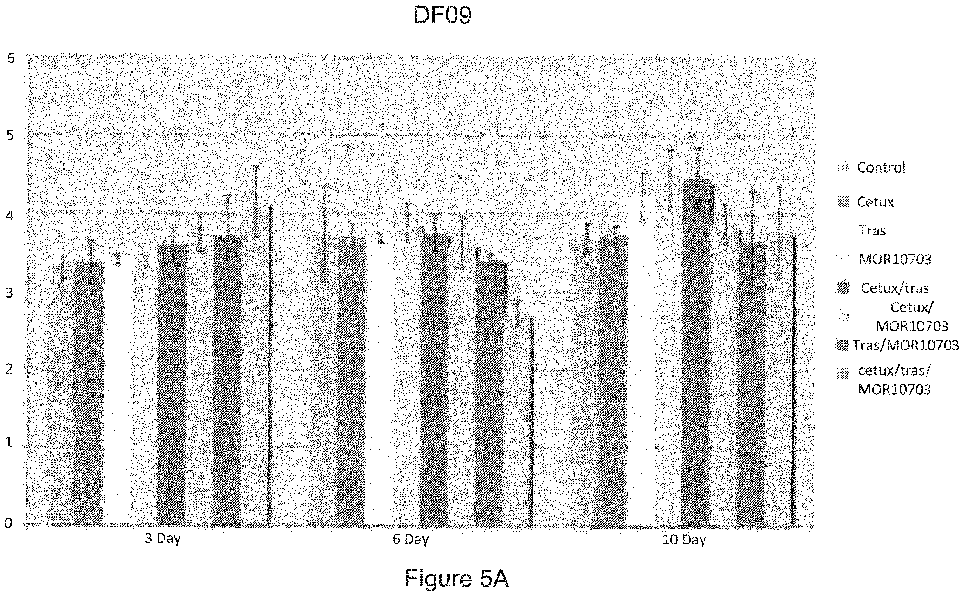

Figures 5A-5D illustrates the effect of monoclonal antibodies directed against EGFR, Her-2 and Her3 in two high grade primary ovarian cancer cells (DF09, DF14) and in the low-grade serous ovarian cancer cell lines (HEY, MPSC1).

### DETAILED DESCRIPTION

The present invention relates to the finding that a particular subset of ovarian cancer, low grade serous ovarian cancer, can be treated with an agent that inhibits signaling of an EGFR family member. In particular it was found that interference in HER3 signaling can be used to treat low-grade serous ovarian cancer.

The most common type of ovarian cancer arises from epithelial cells that line the surface of the ovary. Approximately 50% of epithelial ovarian tumors are classified as serous, or tumors with glandular features, and make up approximately 80% of all ovarian tumors. Other types of ovarian cancers can arise from germ cells (e.g., cancer of the ovarian egg-making cells) and sarcomas. High-grade serous tumors denote highly aggressive, invasive tumors as compared to low malignant potential (LMP) tumors. Whether an invasive serous tumor is classified as either high or low grade is based on the clinical course of the disease. For example, high grade serous tumors were found to over express genes that control various cellular functions associated with cancer cells, for example genes that control cell growth, DNA stability (or lack thereof) and genes that silence other genes. Conversely, LMP tumors were not found to overexpress these types of genes and LMP tumors were alternatively characterized by expression of growth control pathways, such as tumor protein 53 (TP53 or p53) pathways.

More recently, a two-tiered system of characterizing serous ovarian tumors has been described (Vang, et al, 2009, Adv Anat Pathol 16:267-282) based on studies performed Johns Hopkins Hospital and M.D. Anderson Cancer Center. Briefly, low grade serous ovarian tumors are characterized based in a number of criteria, for example low grade serous tumors have low to no chromosomal instability, typically have mutated KRAS, BRAF and HER2 genes, demonstrate slow tumor development, typically have cell nuclei that are uniform, small and round and generally have low mitotic index. Conversely, a high grade serous tumor has a high degree of chromosomal instability, has mutated TP53 gene, demonstrates very fast tumor development, typically has nuclei that are non-uniform, enlarged and irregularly shaped and has high mitotic index.

The human epidermal growth factor receptor 3 (ErbB3, also known as HER3) is a receptor protein tyrosine kinase and belongs to the epidermal growth factor receptor (EGFR) subfamily of receptor protein tyrosine kinases, which also includes EGFR (HER1, ErbBI), HER2 (ErbB2, Neu), and HER4 (ErbB4) (Plowman et al, (1990) Proc. Natl. Acad. Sci. U.S.A. 87:4905-4909; Kraus et al, (1989) Proc. Natl. Acad. Sci. U.S.A. 86:9193-9197; and Kraus et al, (1993) Proc. Natl. Acad. Sci. U.S.A. 90:2900-2904). Like the prototypical epidermal growth factor receptor, the transmembrane receptor HER3 consists of an extracellular ligand- binding domain (ECD), a dimerization domain within the ECD, a transmembrane domain, an intracellular protein tyrosine kinase-like domain (TKD) and a C-terminal phosphorylation domain. Unlike the other EGFR family members, the kinase domain of HER3 displays very low intrinsic kinase activity.

The ligands neuregulin 1 (NRG) or neuregulin 2 bind to the extracellular domain of HER3 and activate receptor-mediated signaling pathway by promoting dimerization with other dimerization partners such as HER2. Heterodimerization results in activation and transphosphorylation of HER3's intracellular domain and is a means not only for signal diversification but also signal amplification. In addition, HER3 heterodimerization can also occur in the absence of activating ligands and this is commonly termed ligand-independent HER3 activation. For example, when HER2 is expressed at high levels as a result of gene amplification (e.g. in breast, lung, ovarian or gastric cancer) spontaneous HER2/ HER3 dimers can be formed. In this situation the HER2/ HER3 is considered the most active ErbB signaling dimer and is therefore highly transforming.

The terms "EGFR family member" refers to a subfamily of epidermal growth factor receptor (EGFR) family members which include the human epidermal growth factor receptor 3 (ErbB3, also known as HER3), EGFR (HER1, ErbBI), HER2 (ErbB2, Neu), and HER4 (ErbB4).

The terms "HER1," "ErbB1," "epidermal growth factor receptor" and "EGFR" are used interchangeably herein and refer to EGFR as disclosed, for example, in Carpenter et al. Ann. Rev. Biochem. 56:881-914 (1987), including naturally occurring mutant forms thereof (e.g. a deletion mutant EGFR as in Humphrey et al, (1990) PNAS (USA) 87:4207-4211). egfr refers to the gene encoding the EGFR protein product.

The terms "HER2" and "ErbB2" and are used interchangeably herein and refer to human HER2 protein described, for example, in Semba et al, (1985) PNAS (USA) 82:6497-6501 and Yamamoto et a/.(1986) Nature 319:230-234 (Genebank accession number X03363). The term "her2" refers to the gene encoding human HER2 and "neu" refers to the-gene encoding rat pI85^{neu}.

The terms "HER4" and "ErbB4" herein refer to the receptor polypeptide as disclosed, for example, in EP Publication No 0,599,274; Plowman et al, (1993) Proc. Natl. Acad. Sci. USA, 90: 1746-1750; and Plowman et al, (1993) Nature, 366:473-475, including isoforms thereof, e.g., as disclosed in WO99/19488, published Apr. 22, 1999.

The term "HER3" or "HER3 receptor" also known as "ErbB3" as used herein refers to mammalian HER3 protein and "her3" or "erbB3" refers to mammalian her3 gene. The preferred HER3 protein is human HER3 protein present in the cell membrane of a cell. The human her3 gene is described in U.S. Pat. No. 5,480,968 and Plowman et al, (1990) Proc. Natl. Acad. Sci. USA, 87:4905-4909. Human HER3 as defined in Accession No. NP 001973 (human) and represented below as SEQ ID NO: 1. All nomenclature is for full length, immature HER3 (amino acids 1-1342). The immature HER3 is cleaved between positions 19 and 20, resulting in the mature HER3 protein (20-1342 amino acids).

MRANDALQVL GLLFSLARGS EVGNSQAVCP GTLNGLSVTG
DAENQYQTLY KLYERCEWM GNLEIVLTGH NADLSFLQWI REVTGYVLVA
MNEFSTLPLP NLRWRGTQV YDGKFAIFVM LNYNTNSSHA LRQLRLTQLT
EILSGGVYIE KNDKLCHMDT IDWRDIVRDR DAEIWKDNG RSCPPCHEVC
KGRCWGPGSE DCQTLTKTIC APQCNGHCFG PNPNQCCHDE CAGGCSGPQD
TDCFACRHFN DSGACVPRCP QPLVYNKLTF QLEPNPHTKY QYGGVCVASC
PHNFWDQTS CVRACPPDKM EVDKNGLKMC EPCGGLCPKA CEGTGSGSRF
QTVDSSNIDG FVNCTKILGN LDFLITGLNG DPWHKIPALD PEKLNVFRTV
REITGYLNIQ SWPPHMHNFS VFSNLTTIGG RSLYNRGFSL LIMKNLNVTS
LGFRSLKEIS AGRIYISANR QLCYHHSLNW TKVLRGPTEE RLDIKHNRPR
RDCVAEGKVC DPLCSSGGCW GPGPGQCLSC RNYSRGGVCV THCNFLNGEP
REFAHEAECF SCHPECQPME GTATCNGSGS DTCAQCAHFR DGPHCVSSCP
HGVLGAKGPI YKYPDVQNEC RPCHENCTQG CKGPELQDCL GQTLVLIGKT
HLTMALTVIA GLWIFMMLG GTFLYWRGRR IQNKRAMRRY LERGESIEPL
DPSEKANKVL ARIFKETELR KLKVLGSGVF GTVHKGVWIP EGESIKIPVC
IKVIEDKSGR QSFQAVTDHM LAIGSLDHAH IVRLLGLCPG SSLQLVTQYL
PLGSLLDHVR QHRGALGPQL LLNWGVQIAK GMYYLEEHGM VHRNLAARNV
LLKSPSQVQV ADFGVADLLP PDDKQLLYSE AKTPIKWMAL ESIHFGKYTH
QSDVWSYGVT VWELMTFGAE PYAGLRLAEV PDLLEKGERL AQPQICTIDV
YMVMVKCWMI DENIRPTFKE LANEFTRMAR DPPRYLVIKR ESGPGIAPGP
EPHGLTNKKL EEVELEPELD LDLDLEAEED NLATTTLGSA LSLPVGTLNR
PRGSQSLLSP SSGYMPMNQG NLGESCQESA VSGSSERCPR PVSLHPMPRG
CLASESSEGH VTGSEAELQE KVSMCRSRSR SRSPRPRGDS AYHSQRHSLL
TPVTPLSPPG LEEEDVNGYV MPDTHLKGTP SSREGTLSSV GLSSVLGTEE
EDEDEEYEYM NRRRRHSPPH PPRPSSLEEL GYEYMDVGSD LSASLGSTQS
CPLHPVPIMP TAGTTPDEDY EYMNRQRDGG GPGGDYAAMG ACPASEQGYE
EMRAFQGPGH QAPHVHYARL KTLRSLEATD SAFDNPDYWH SRLFPKANAQ RT
(SEQ ID NO: 1)

The term "HER ligand" or "ErbB ligand" as used herein refers to polypeptides which bind and activate HER receptors such as HER1, HER2, HER3 and HER4. Examples of HER ligands include, but are not limited to neuregulin 1 (NRG), neuregulin 2, neuregulin 3, neuregulin 4, betacellulin, heparin-binding epidermal growth factor, epiregulin, epidermal growth factor, amphiregulin, and transforming growth factor alpha. The term includes biologically active fragments and/or variants of a naturally occurring polypeptide.

The term "HER3 ligand" as used herein refers to polypeptides which bind and activate HER3. Examples of HER3 ligands include, but are not limited to neuregulin 1 (NRG) and neuregulin 2, betacellulin, heparin-binding epidermal growth factor, and epiregulin. The term includes biologically active fragments and/or variants of a naturally occurring polypeptide.

The "HER-HER protein complex" is a noncovalently associated oligomer containing a HER co- receptors in any combination (e.g., HER1-HER2, HER1-HER3, HER1-HER4, HER2- HER3, HER3- HER4, and the like). This complex can form when a cell expressing both of these receptors is exposed to a HER ligand e.g., NRG, or when a HER receptor is active or overexpressed.

The "HER2-HER3 protein complex" is a noncovalently associated oligomer containing HER2 receptor and the HER3 receptor. This complex can form when a cell expressing both of these receptors is exposed to a HER3 ligand e.g., NRG or when HER2 is active/overexpressed The phrase "HER3 activity" or "HER3 activation" as used herein refers to an increase in oligomerization (e.g. an increase in HER3 containing complexes), HER3 phosphorylation, conformational rearrangements (for example those induced by ligands), and HER3 mediated downstream signaling.

The term "stabilization" or "stabilized" used in the context of HER3 refers to an antibody or fragment thereof that directly maintains (locks, tethers, holds, preferentially binds, favors) the inactive state or conformation of Her3 without blocking ligand binding to HER3, such that ligand binding is no longer able to activate HER3. Assays can be used to measure ligand binding to a stabilized HER3 receptor, e.g., Biacore assay.

The term "ligand-dependent signaling" as used herein refers to the activation of ErbB (e.g., HER3) via ligand. HER3 activation is evidenced by increased oligomerization (e.g. heterodimerization) and/or HER3 phosphorylation such that downstream signaling pathways (e.g. PI3K) are activated. The antibody or fragment thereof can statistically significantly reduce the amount of phosphorylated HER3 in a stimulated cell exposed to the antigen binding protein (e.g., an antibody) relative to an untreated (control) cell, as measured using the assays described in the Examples. The cell which expresses HER3 can be a naturally occurring cell line (e.g. MCF7) or can be recombinantly produced by introducing nucleic acids encoding HER3 protein into a host cell. Cell stimulation can occur either via the exogenous addition of an activating HER3 ligand or by the endogenous expression of an activating ligand. The antibody or fragment thereof which "reduces neregulin-induced HER3 activation in a cell" is one which statistically significantly reduces HER3 tyrosine phosphorylation relative to an untreated (control) cell. This can be determined based on HER3 phosphotyrosine levels following exposure of HER3 to NRG and the antibody of interest. The cell which expresses HER3 protein can be a naturally occurring cell or cell line (e.g. MCF7) or can be recombinantly produced.

The term "ligand-independent signaling" as used herein refers to cellular HER3 activity (e.g. phosphorylation) in the absence of a requirement for ligand binding. For example, ligand- independent HER3 activation can be a result of HER2 overexpression or activating mutations in HER3 heterodimer partners such as EGFR and HER2. The antibody or fragment thereof can statistically significantly reduce the amount of phosphorylated HER3 in a cell exposed to the antigen binding protein (e.g., an antibody) relative to an untreated (control) cell. The cell which expresses HER3 can be a naturally occurring cell line (e.g. SK-Br-3) or can be recombinantly produced by introducing nucleic acids encoding HER3 protein into a host cell.

The term "blocks" as used herein refers to stopping or preventing an interaction or a process, e.g., stopping ligand-dependent or ligand-independent signaling.

The term "recognize" as used herein refers to an antibody or fragment thereof that finds and interacts (e.g., binds) with its conformational epitope.

The phrase "concurrently binds" as used herein refers to an ErbB ligand that can bind to a ligand binding site on the ErbB receptor along with the ErbB antibody. This means that both the antibody and antibody can bind to the HER receptor together. For the sake of illustration only, the HER3 ligand NRG, can bind to the HER3 receptor along with the HER3 antibody.

The term "fails" as used herein refers to an antibody or fragment thereof that does not do a particular event. For example, an antibody or fragment thereof that "fails to activate signal transduction" is one that does not trigger signal transduction; an antibody or fragment thereof that "fails to induce a conformational change" is one that does not cause a structural alteration in the ErbB receptor; an antibody or fragment thereof that stabilizes the ErbB receptor in an inactive state such that the ErbB receptor "fails to dimerize" is one that does not form protein- protein complexes.

The term "antibody" as used herein refers to whole antibodies that interact with (e.g., by binding, steric hindrance, stabilizing/destabilizing, spatial distribution) an HER3 epitope and inhibit signal transduction. A naturally occurring "antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CHI, CH2 and CH3. Each light chain is comprised of a light chain variable region (abbreviated herein as VL) and a light chain constant region. The light chain constant region is comprised of one domain, CL. The VH and V_{L} regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. The term "antibody" includes for example, monoclonal antibodies, human antibodies, humanized antibodies, camelised antibodies, chimeric antibodies, single-chain Fvs (scFv), disulfide-linked Fvs (sdFv), Fab fragments, F (ab') fragments, and anti-idiotypic (anti-Id) antibodies (including, e.g., anti-Id antibodies to antibodies for use according to the invention), and epitope-binding fragments of any of the above. The antibodies can be of any isotype (e.g., IgG, IgE, IgM, IgD, IgA and IgY), class (e.g., IgGI, IgG2, IgG3, IgG4, IgAI and IgA2) or subclass. Both the light and heavy chains are divided into regions of structural and functional homology. The terms "constant" and "variable" are used functionally. In this regard, it will be appreciated that the variable domains of both the light (VL) and heavy (VH) chain portions determine antigen recognition and specificity. Conversely, the constant domains of the light chain (CL) and the heavy chain (CHI, CH2 or CH3) confer important biological properties such as secretion, transplacental mobility, Fc receptor binding, complement binding, and the like. By convention the numbering of the constant region domains increases as they become more distal from the antigen binding site or amino-terminus of the antibody. The N-terminus is a variable region and at the C-terminus is a constant region; the CH3 and CL domains actually comprise the carboxy-terminus of the heavy and light chain, respectively.

The phrase "antibody fragment", as used herein, refers to one or more portions of an antibody that retain the ability to specifically interact with (e.g., by binding, steric hindrance, stabilizing/destabilizing, spatial distribution) an HER3 epitope and inhibit signal transduction. Examples of binding fragments include, but are not limited to, a Fab fragment, a monovalent fragment consisting of the VL, VH, CL and CHI domains; a F(ab)₂ fragment, a bivalent fragment comprising two Fab fragments linked by a disulfide bridge at the hinge region; a Fd fragment consisting of the VH and CHI domains; a Fv fragment consisting of the VL and VH domains of a single arm of an antibody; a dAb fragment (Ward et al, (1989) Nature 341 :544- 546), which consists of a VH domain; and an isolated complementarity determining region (CDR).

Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al, (1988) Science 242:423-426; and Huston et al, (1988) Proc. Natl. Acad. Sci. 85:5879-5883). Such single chain antibodies are also intended to be encompassed within the term "antibody fragment". These antibody fragments are obtained using conventional techniques known to those of skill in the art, and the fragments are screened for utility in the same manner as are intact antibodies. Antibody fragments can also be incorporated into single domain antibodies, maxibodies, minibodies, intrabodies, diabodies, triabodies, tetrabodies, v-NAR and bis-scFv (see, e.g., Hollinger and Hudson, (2005) Nature Biotechnology 23: 1126-1136). Antibody fragments can be grafted into scaffolds based on polypeptides such as Fibronectin type III (Fn3) (see U.S. Pat. No. 6,703,199, which describes fibronectin polypeptide monobodies).

Antibody fragments can be incorporated into single chain molecules comprising a pair of tandem Fv segments (VH-CH1-VH-CH1) which, together with complementary light chain polypeptides, form a pair of antigen binding regions (Zapata et al., (1995) Protein Eng. 8: 1057-1062; and U.S. Pat. No. 5,641,870).

The term "epitope" includes any protein determinant capable of specific binding to an immunoglobulin or otherwise interacting with a molecule. Epitopic determinants generally consist of chemically active surface groupings of molecules such as amino acids or carbohydrate or sugar side chains and can have specific three-dimensional structural characteristics, as well as specific charge characteristics. An epitope may be "linear" or "conformational."

The term "linear epitope" refers to an epitope with all of the points of interaction between the protein and the interacting molecule (such as an antibody) occur linearly along the primary amino acid sequence of the protein (continuous). Once a desired epitope on an antigen is determined, it is possible to generate antibodies to that epitope, e.g., using the techniques described herein. Alternatively, during the discovery process, the generation and characterization of antibodies may elucidate information about desirable epitopes. From this information, it is then possible to competitively screen antibodies for binding to the same epitope. An approach to achieve this is to conduct cross-competition studies to find antibodies that competitively bind with one another, e.g., the antibodies compete for binding to the antigen. A high throughput process for "binning" antibodies based upon their cross- competition is described in WO 2003/48731. As will be appreciated by one of skill in the art, practically anything to which an antibody can specifically bind could be an epitope. An epitope can comprises those residues to which the antibody binds.

The term "conformational epitope" refers to an epitope in which discontinuous amino acids that come together in three dimensional conformation. In a conformational epitope, the points of interaction occur across amino acid residues on the protein that are separated from one another. In one embodiment, the conformational epitope is defined by (i) HER3 amino acid residues 265-277 and 315 (of domain 2) and (ii) HER3 amino acid residues 571, 582-584, 596-597, 600-602, 609-615 (of domain 4) of SEQ ID NO: 1, or a subset thereof. As will be appreciated by one of skill in the art, the space that is occupied by a residue or side chain that creates the shape of a molecule helps to determine what an epitope is.

Generally, antibodies specific for a particular target antigen will preferentially recognize an epitope on the target antigen in a complex mixture of proteins and/or macromolecules.

Regions of a given polypeptide that include an epitope can be identified using any number of epitope mapping techniques, well known in the art. See, e.g., Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871 ; Geysen et al, (1984) Proc. Natl. Acad. Sci. USA 8:3998-4002; Geysen et al, (1985) Proc. Natl. Acad. Sci. USA 82:78-182; Geysen et al, (1986) Mol. Immunol. 23:709-715. Similarly, conformational epitopes are readily identified by determining spatial conformation of amino acids such as by, e.g., hydrogen/deuterium exchange, x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al, (1981) Proc. Natl. Acad. Sci USA 78:3824-3828; for determining antigenicity profiles, and the Kyte-Doolittle technique, Kyte et al, (1982) J.Mol. Biol. 157: 105-132; for hydropathy plots.

The term "paratope" as used herein refers to the general structure of a binding region that determines binding to an epitope. This structure influences whether or not and in what manner the binding region might bind to an epitope. Paratope can refer to an antigenic site of an antibody that is responsible for an antibody or fragment thereof, to bind to an antigenic determinant. Paratope also refers to the idiotope of the antibody, and the complementary determining region (CDR) region that binds to the epitope. In one embodiment, the paratope is the region of the antibody that binds to the conformational epitope comprising (i) HER3 amino acid residues 265-277 and 315 (of domain 2), and (ii) HER3amino acid residues 571, 582-584, 596-597, 600-602, 609-615 (of domain 4) of SEQ ID NO: 1, or a subset thereof. In one embodiment, the paratope is the region of the antibody that comprises the CDR sequences. In one embodiment, the paratope comprises at least one amino acid residue that binds with HER3 residues: Asn266, Lys267, Leu268, Thr269, Gln271, Glu273, Pro274, Asn275, Pro276, His277, Asn315, Asp571, Pro583, His584, Ala596, Lys597. In one embodiment, the paratope comprises at least one amino acid residue that binds with HER3 residues: Tyr265, Lys267, Leu268, Phe270, Gly582, Pro583, Lys597, Ile600, Lys602, Glu609, Arg611, Pro612, Cys613, His614, Glu615. As will be appreciated by one of skill in the art, the paratope of any antibody, or variant thereof, can be determined in the manner set forth by the present application.

The phrases "monoclonal antibody" or "monoclonal antibody composition" as used herein refers to polypeptides, including antibodies, antibody fragments, bispecific antibodies, etc. that have substantially identical to amino acid sequence or are derived from the same genetic source. This term also includes preparations of antibody molecules of single molecular composition. A monoclonal antibody composition displays a single binding specificity and affinity for a particular epitope.

The phrase "human antibody", as used herein, includes antibodies having variable regions in which both the framework and CDR regions are derived from sequences of human origin. Furthermore, if the antibody contains a constant region, the constant region also is derived from such human sequences, e.g., human germline sequences, or mutated versions of human germline sequences or antibody containing consensus framework sequences derived from human framework sequences analysis, for example, as described in Knappik et al., (2000) J Mol Biol 296:57-86). The structures and locations of immunoglobulin variable domains, e.g., CDRs, may be defined using well known numbering schemes, e.g., the Kabat numbering scheme, the Chothia numbering scheme, or a combination of Kabat and Chothia (see, e.g., Sequences of Proteins of Immunological Interest, U.S. Department of Health and Human Services (1991), eds. Kabat et al.; Lazikani et al., (1997) J. Mol. Bio. 273:927-948); Kabat et al., (1991) Sequences of Proteins of Immunological Interest, 5th edit., NIH Publication no. 91-3242 U.S. Department of Health and Human Services; Chothia et al., (1987) J. Mol. Biol. 196:901-917; Chothia et al., (1989) Nature 342:877-883; and Al-Lazikani et al., (1997) J. Mol. Biol. 273:927-948. The human antibodies for use according to the invention may include amino acid residues not encoded by human sequences (e.g., mutations introduced by random or sitespecific mutagenesis in vitro or by somatic mutation in vivo, or a conservative substitution to promote stability or manufacturing). The phrase "human monoclonal antibody" as used herein refers to antibodies displaying a single binding specificity which have variable regions in which both the framework and CDR regions are derived from human sequences. In one embodiment, the human monoclonal antibodies are produced by a hybridoma which includes a B cell obtained from a transgenic nonhuman animal, e.g., a transgenic mouse, having a genome comprising a human heavy chain transgene and a light chain transgene fused to an immortalized cell.

The phrase "recombinant human antibody", as used herein, includes all human antibodies that are prepared, expressed, created or isolated by recombinant means, such as antibodies isolated from an animal (e.g., a mouse) that is transgenic or transchromosomal for human immunoglobulin genes or a hybridoma prepared therefrom, antibodies isolated from a host cell transformed to express the human antibody, e.g., from a transfectoma, antibodies isolated from a recombinant, combinatorial human antibody library, and antibodies prepared, expressed, created or isolated by any other means that involve splicing of all or a portion of a human immunoglobulin gene, sequences to other DNA sequences. Such recombinant human antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire in vivo.

Specific binding between two entities means a binding with an equilibrium constant (KA) (kₒₙ/k_{off}) of at least 10²M-¹, at least 5xI0²M⁻¹, at least 10³M¹, at least 5xI0³M⁻¹ , at least 10⁴M" at least 5xI0⁴M⁻¹, at least 10⁵M⁻¹, at least 5xI0⁵M⁻¹, at least 10⁶M⁻¹, at least 5xI0⁶M⁻¹, at least 10⁷M⁻¹, at least 5xI0⁷M⁻¹, at least 10⁸M⁻¹, at least 5xI0⁸M⁻¹, at least 10⁹M⁻¹, at least 5xI0⁹M⁻¹, at least 10¹⁰M⁻¹, at least 5xI0¹⁰M⁻¹, at least 10ⁿM⁻¹, at least 5xI0ⁿM⁻¹, at least 10¹²M⁻¹, at least 5xI0¹²M⁻¹, at least 10¹³M⁻¹, at least 5xI0¹³ M⁻¹, at least 10¹⁴M⁻¹, at least 5xI0¹⁴M⁻¹, at least 10¹⁵M⁻¹, or at least 5xI0¹⁵M⁻¹. The phrase "specifically (or selectively) binds" to an antibody (e.g., a HER3 binding antibody) refers to a binding reaction that is determinative of the presence of a cognate antigen (e.g., a human HER3) in a heterogeneous population of proteins and other biologics. In addition to the equilibrium constant (KA) noted above, an HER3 binding antibody for use according to the invention typically also has a dissociation rate constant (KD) (k_{off}/kₒₙ) of less than 5x10⁻²M, less than 10⁻²M, less than 5x10⁻³M, less than 10⁻³M, less than 5x10⁻⁴M, less than 10⁻⁴M, less than 5x10⁻⁵M, less than 10⁻⁵M, less than 5x10⁻⁶M, less than 10⁻⁶M, less than 5x10⁻⁷M, less than 10⁻⁷M, less than 5x10⁻⁸M, less than 10⁻⁸M, less than 5x10⁻⁹M, less than 10⁻⁹M, less than 5x10⁻¹⁰M, less than 10⁻¹⁰M, less than 5x10⁻¹¹M, less than 10⁻¹¹M, less than 5x10⁻¹²M, less than 10⁻¹²M, less than 5x10⁻¹³M, less than 10⁻¹³M, less than 5x10⁻¹⁴M, less than 10⁻¹⁴M, less than 5x10⁻¹⁵M, or less than 10⁻¹⁵M or lower, and binds to HER3 with an affinity that is at least twofold greater than its affinity for binding to a non-specific antigen (e.g., HSA).

In one embodiment, the antibody or fragment thereof has dissociation constant (KD) of less than 3000 pM, less than 2500 pM, less than 2000 pM, less than 1500 pM, less than 1000 pM, less than 750 pM, less than 500 pM, less than 250 pM, less than 200 pM, less than 150 pM, less than 100 pM, less than 75 pM, less than 10 pM, less than 1 pM as assessed using a method described herein or known to one of skill in the art (e.g., a BIAcore assay, ELISA, FACS, SET) (Biacore International AB, Uppsala, Sweden). The term "K_{aSS0C}" or "Kₐ", as used herein, refers to the association rate of a particular antibody-antigen interaction, whereas the term "K_{dis}" or "K_{d}," as used herein, refers to the dissociation rate of a particular antibody- antigen interaction. The term "KD", as used herein, refers to the dissociation constant, which is obtained from the ratio of K_{d} to Kₐ (i.e. K_{d}/Kₐ) and is expressed as a molar concentration (M). KD values for antibodies can be determined using methods well established in the art. A method for determining the KD of an antibody is by using surface plasmon resonance, or using a biosensor system such as a Biacore® system.

The term "affinity" as used herein refers to the strength of interaction between antibody and antigen at single antigenic sites. Within each antigenic site, the variable region of the antibody "arm" interacts through weak noncovalent forces with antigen at numerous sites; the more interactions, the stronger the affinity. The term "avidity" as used herein refers to an informative measure of the overall stability or strength of the antibody-antigen complex. It is controlled by three major factors: antibody epitope affinity; the valence of both the antigen and antibody; and the structural arrangement of the interacting parts. Ultimately these factors define the specificity of the antibody, that is, the likelihood that the particular antibody is binding to a precise antigen epitope.

The term "valency" as used herein refers to the number of potential target binding sites in a polypeptide. Each target binding site specifically binds one target molecule or specific site (i.e, epitope) on a target molecule. When a polypeptide comprises more than one target binding site, each target binding site may specifically bind the same or different molecules (e.g., may bind to different molecules, e.g., different antigens, or different epitopes on the same molecule).

The phrase "antagonist antibody" as used herein refers to an antibody that binds with HER3 and neutralizes the biological activity of HER3 signaling, e.g., reduces, decreases and/or inhibits HER3 induced signaling activity, e.g., in a phospho- HER3 or phospho-Akt assay. Accordingly, an antibody that "inhibits" one or more of these HER3 functional properties (e.g., biochemical, immunochemical, cellular, physiological or other biological activities, or the like) as determined according to methodologies known to the art and described herein, will be understood to relate to a statistically significant decrease in the particular activity relative to that seen in the absence of the antibody (e.g., or when a control antibody of irrelevant specificity is present). An antibody that inhibits HER3 activity effects such a statistically significant decrease by at least 10% of the measured parameter, by at least 50%>, 80%> or 90%>, and in certain embodiments an antibody for use according to the invention may inhibit greater than 95%, 98% or 99% of HER3 functional activity as evidenced by a reduction in the level of cellular HER3 phosphorylation.

The phrase "isolated antibody" refers to an antibody that is substantially free of other antibodies having different antigenic specificities (e.g. , an isolated antibody that specifically binds HER3 is substantially free of antibodies that specifically bind antigens other than HER3). An isolated antibody that specifically binds HER3 may, however, have cross- reactivity to other antigens. Moreover, an isolated antibody may be substantially free of other cellular material and/or chemicals.

The phrase "conservatively modified variant" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" include individual substitutions, deletions or additions to a polypeptide sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). In some embodiments, the term "conservative sequence modifications" are used to refer to amino acid modifications that do not significantly affect or alter the binding characteristics of the antibody containing the amino acid sequence.

The terms "cross-compete" and "cross-competing" are used interchangeably herein to mean the ability of an antibody or other binding agent to interfere with the binding of other antibodies or binding agents to HER3 in a standard competitive binding assay.

The ability or extent to which an antibody or other binding agent is able to interfere with the binding of another antibody or binding molecule to HER3 , and therefore whether it can be said to cross-compete according to the invention, can be determined using standard competition binding assays. One suitable assay involves the use of the Biacore technology {e.g. by using the BIAcore 3000 instrument (Biacore, Uppsala, Sweden)), which can measure the extent of interactions using surface plasmon resonance technology. Another assay for measuring cross-competing uses an ELISA-based approach.

The term "optimized" as used herein refers to a nucleotide sequence has been altered to encode an amino acid sequence using codons that are preferred in the production cell or organism, generally a eukaryotic cell, for example, a cell of Pichia, a cell of Trichoderma, a Chinese Hamster Ovary cell (CHO) or a human cell. The optimized nucleotide sequence is engineered to retain completely or as much as possible the amino acid sequence originally encoded by the starting nucleotide sequence, which is also known as the "parental" sequence.

Standard assays to evaluate the binding ability of the antibodies toward HER3 of various species are known in the art, including for example, ELISAs, western blots and RIAs. Suitable assays are described in detail in the Examples. The binding kinetics (e.g., binding affinity) of the antibodies also can be assessed by standard assays known in the art, such as by Biacore analysis, or FACS relative affinity (Scatchard). Assays to evaluate the effects of the antibodies on functional properties of HER3 (e.g., receptor binding assays, modulating the Her pathway) known in the art may be used.

The phrases "percent identical" or "percent identity," in the context of two or more nucleic acids or polypeptide sequences, refers to two or more sequences or subsequences that are the same. Two sequences are "substantially identical" if two sequences have a specified percentage of amino acid residues or nucleotides that are the same {i.e., 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positivevalued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative- scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11 , an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, (1989) Proc. Natl. Acad. Sci. USA 89: 10915) alignments (B) of 50, expectation (E) of 10, M=5, N=-4, and a comparison of both strands.

The BLAST algorithm also performs a statistical analysis of the similarity between two sequences (see, e.g., Karlin and Altschul, (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller, (1988) Comput. Appl. Biosci. 4: 11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a

PAM250 matrix, and a gap weight of 16, 14, 12, 10, 8, 6, or 4 and a length weight of 1, 2, 3, 4, 5, or 6. Other than percentage of sequence identity noted above, another indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

The phrase "nucleic acid" is used herein interchangeably with the term "polynucleotide" and refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (e.g. , degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., (1991) Nucleic Acid Res. 19:5081; Ohtsuka et al., (1985) J. Biol. Chem. 260:2605-2608; and Rossolini et al., (1994) Mol. Cell. Probes 8:91-98).

The phrase "operably linked" refers to a functional relationship between two or more polynucleotide (e.g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis- acting. However, some transcriptional regulatory sequences, such as enhancers, need not be physically contiguous or located in close proximity to the coding sequences whose transcription they enhance.

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

The phrase "signal transduction" or "signaling activity" as used herein refers to a biochemical causal relationship generally initiated by a protein-protein interaction such as binding of a growth factor to a receptor, resulting in transmission of a signal from one portion of a cell to another portion of a cell. For HER3, the transmission involves specific phosphorylation of one or more tyrosine, serine, or threonine residues on one or more proteins in the series of reactions causing signal transduction. Penultimate processes typically include nuclear events, resulting in a change in gene expression.

The term "subject" includes human and non-human animals. Nonhuman animals include all vertebrates, e.g., mammals and non-mammals, such as non-human primates, sheep, dog, cow, chickens, amphibians, and reptiles. Except when noted, the terms "patient" or "subject" are used herein interchangeably.

The term "anti-cancer agent" means any agent that can be used to treat a cell proliferative disorder such as cancer, including cytotoxic agents, chemotherapeutic agents, radiotherapy and radiotherapeutic agents, targeted anti-cancer agents, and immunotherapeutic agents. "Tumor" refers to neoplastic cell growth and proliferation, whether malignant or benign, and all pre-cancerous and cancerous cells and tissues.

The term "anti-tumor activity" means a reduction in the rate of tumor cell proliferation, viability, or metastatic activity. A possible way of showing anti-tumor activity is show a decline in growth rate of abnormal cells that arises during therapy or tumor size stability or reduction. Such activity can be assessed using accepted in vitro or in vivo tumor models, including but not limited to xenograft models, allograft models, MMTV models, and other known models known in the art to investigate anti-tumor activity.

The term "malignancy" refers to a non-benign tumor or a cancer. As used herein, the term "cancer" includes a malignancy characterized by deregulated or uncontrolled cell growth. The term "cancer" includes primary malignant tumors (e.g., those whose cells have not migrated to sites in the subject's body other than the site of the original tumor) and secondary malignant tumors (e.g., those arising from metastasis, the migration of tumor cells to secondary sites that are different from the site of the original tumor).

"Treating", "treat", or "treatment" within the context of the instant invention, means an alleviation of symptoms associated with a disorder or disease, or halt of further progression or worsening of those symptoms, or prevention or prophylaxis of the disease or disorder. For example, within the context of this invention, successful treatment may include an alleviation of symptoms related to low grade serous ovarian cancer or a halting in the progression of a disease such as low grade serous ovarian cancer.

"Low grade serous ovarian tumors" within the context of the present invention are characterized based in a number of criteria, for example low grade serous tumors have low to no chromosomal instability, typically have mutated KRAS, BRAF and HER2 genes, demonstrate slow tumor development, typically have cell nuclei that are uniform, small and round and generally have low mitotic index.

### HER3 Antibodies

In some embodiments, the methods of the disclosure are directed to administering an antibody or fragment thereof that specifically binds to HER3 for the treatment of low-grade serous ovarian cancer. In some embodiments, the antibody or fragment thereof blocks both ligand-dependent and ligand-independent signal transduction. In another embodiment, the antibodies bind to HER3 and do not block ErbB ligand binding to the ligand binding site (i.e. both ligand and antibody can bind HER3 concurrently). The antibody or fragment thereof in accordance with the present invention binds to a conformational epitope comprising amino acid residues within domain 2 and domain 4 of HER3. In other embodiments, the antibody or fragment thereof binds to an epitope comprising amino acid residues 208-328 within domain 2 of HER3. In yet other embodiments, the antibody or fragment thereof recognizes a non-linear epitope of HER3 comprising amino acid residues within domain 3 of HER3 and binds to at least one amino acid residue selected from binding surface B.

Specific examples of HER3 antibodies are known in the art. In one embodiment, the VH of the antibody or fragment thereof binds to at least one of the following HER3 residues: Asn266, Lys267, Leu268, Thr269, Gln271, Glu273, Pro274, Asn275, Pro276, His277, Asn315, Asp571, Pro583, His584, Ala596, Lys597. In another embodiment, the VL of the antibody or fragment thereof binds to at least one of the following HER3 residues: Tyr265, Lys267, Leu268, Phe270, Gly582, Pro583, Lys597, Ile600, Lys602, Glu609, Arg611, Pro612, Cys613, His614, Glu615.

In some embodiments, the antibody or fragment thereof binds to human HER3 protein having a conformational epitope comprising (i) HER3 amino acid residues 265-277 and 315 (of domain 2) and (ii) HER3 amino acid residues 571, 582-584, 596-597, 600-602, 609-615 (of domain 4) of SEQ ID NO: 1, or a subset thereof. In some embodiments, the antibody or fragment thereof binds to amino acids within or overlapping amino acid residues 265-277 and 315 (of domain 2) and (ii) HER3 amino acid residues 571, 582-584, 596-597, 600-602, 609-615 (of domain 4) of SEQ ID NO: 1. In some embodiments, the antibody or fragment thereof binds to amino acids within (and/or amino acid sequences consisting of) amino acids 265-277 and 315 (of domain 2) and (ii) HER3 amino acid residues 571, 582-584, 596-597, 600-602, 609-615 (of domain 4) of SEQ ID NO: 1, or a subset thereof. In some embodiments, the antibody or fragment thereof binds to the conformational epitope such that it restricts the mobility of domain 2 and domain 4, stabilizing it in an inactive or closed conformation. The failure to form the active conformation results in failure to activate signal transduction. In some embodiments, the antibody or fragment thereof binds to the conformational epitope such that it occludes the dimerization loop within domain 2, thereby rendering it unavailable for receptor-receptor interaction. The failure to form homo- or heterodimers results in failure to activate signal transduction. The present invention can utilize HER3 antibodies that recognize a conformational epitope of HER3 such that they block both ligand-dependent and ligand-independent HER3 signal transduction pathways. Such a class of antibodies are disclosed in Table 1.

The isolated antibody or fragment thereof can be a monoclonal antibody, a polyclonal antibody, a chimeric antibody, a humanized antibody, and a synthetic antibody.

In another example, the isolated antibody or fragment thereof can bind to the same conformational epitope as an antibody described in Table 1.

**Table 1: Examples of HER3 Antibodies useful in the methods of the present disclosure.**

| SEQ ID NUMBER | Ab region | |
|---|---|---|
| **MOR09823** | | |
| SEQ ID NO: 2 (Kabat) | HCDR1 | SYAMS |
| SEQ ID NO: 3 (Kabat) | HCDR2 | VTGAVGRTYYPDSVKG |
| SEQ ID NO: 4 (Kabat) | HCDR3 | WGDEGFDI |
| SEQ ID NO: 5 (Kabat) | LCDR1 | RASQGISNWLA |
| SEQ ID NO: 6 (Kabat) | LCDR2 | GASSLQS |
| SEQ ID NO: 7 (Kabat) | LCDR3 | QQYSSFPTT |
| SEQ ID NO: 8 (Chothia) | HCDR1 | GFTFSSY |
| SEQ ID NO: 9 (Chothia) | HCDR2 | GAVGR |
| SEQ ID NO: 10 (Chothia) | HCDR3 | WGDEGFDI |
| SEQ ID NO: 11 (Chothia) | LCDR1 | SQGISNW |
| SEQ ID NO: 12 (Chothia) | LCDR2 | GAS |
| SEQ ID NO: (Chothia) 13 | LCDR3 | YSSFPT |
| SEQ ID NO: 14 | VL | DIQMTQSPSSLSASVGDRVTITCRASQGISNWLAWYQQKP GKAPKLLIYG ASSLQSGVPSRFSGSGSGTDFTLTISSLQPEDFAVYYCQQ YSSFPTTFGQ GTKVEIK |
| SEQ ID NO: 15 | VH | QVQLVESGGGLVQPGGSLRLSCAASGFTFSSYAMSWVRQA PGKGLEWVSV TGAVGRTYYPDSVKGRFTISRDNSKNTLYLQMNSLRAEDT AVYYCARWGD EGFDIWGQGTLVTVSS |
| SEQ ID NO: 16 | DNA VL | GATATCCAGATGACCCAGAGCCCGTCTAGCCTGAGCGCGA GCGTGGGTGATCGTGTGACCATTACCTGCAGAGCGAGCCA GGGTATTTCTAATTGGCTGGCTTGGTACCAGCAGAAACCA GGTAAAGCACCGAAACTATTAATTTATGGTGCTTCTTCTT TGCAAAGCGGGGTCCCGTCCCGTTTTAGCGGCTCTGGATC CGGCACTGATTTTACCCTGACCATTAGCAGCCTGCAACCT GAAGACTTTGCGGTTTATTATTGCCAGCAGTATTCTTCTT TTCCTACTACCTTTGGCCAGGGTACGAAAGTTGAAATTAA A |
| SEQ ID NO: 17 | DNA VH | CAGGTGCAATTGGTGGAAAGCGGCGGCGGCCTGGTGCAAC CGGGCGGCAGCCTGCGTCTGAGCTGCGCGGCCTCCGGATT TACCTTTAGCAGCTATGCGATGAGCTGGGTGCGCCAAGCC CCTGGGAAGGGTCTCGAGTGGGTGAGCGTTACTGGTGCTG TTGGTCGTACTTATTATCCTGATTCTGTTAAGGGTCGTTT TACCATTTCACGTGATAATTCGAAAAACACCCTGTATCTG CAAATGAACAGCCTGCGTGCGGAAGATACGGCCGTGTATT ATTGCGCGCGTTGGGGTGATGAGGGTTTTGATATTTGGGG CCAAGGCACCCTGGTGACGGTTAGCTCA |
| SEQ ID NO: 18 | Light Kappa | DIQMTQSPSSLSASVGDRVTITCRASQGISNWLAWYQQKP GKAPKLLIYG ASSLQSGVPSRFSGSGSGTDFTLTISSLQPEDFAVYYCQQ YSSFPTTFGQ GTKVEIKRTVAAPSVFIFPPSDEQLKSGTASVVCLLNNFY |
| | | PREAKVQWKV DNALQSGNSQESVTEQDSKDSTYSLSSTLTLSKADYEKHK VYACEVTHQG LSSPVTKSFNRGEC |
| SEQ ID NO: 19 | Heavy IgG1 | QVQLVESGGGLVQPGGSLRLSCAASGFTFSSYAMSWVRQA PGKGLEWVSV TGAVGRTYYPDSVKGRFTISRDNSKNTLYLQMNSLRAEDT AVYYCARWGD EGFDIWGQGTLVTVSSASTKGPSVFPLAPSSKSTSGGTAA LGCLVKDYFP EPVTVSWNSGALTSGVHTFPAVLQSSGLYSLSSVVTVPSS SLGTQTYICN VNHKPSNTKVDKRVEPKSCDKTHTCPPCPAPELLGGPSVF LFPPKPKDTL MISRTPEVTCVVVDVSHEDPEVKFNWYVDGVEVHNAKTKP REEQYNSTYR VVSVLTVLHQDWLNGKEYKCKVSNKALPAPIEKTISKAKG QPREPQVYTL PPSREEMTKNQVSLTCLVKGFYPSDIAVEWESNGQPENNY KTTPPVLDSD GSFFLYSKLTVDKSRWQQGNVFSCSVMHEALHNHYTQKSL SLSPGK |

| **MOR09824** | | |
|---|---|---|
| SEQ ID NO: 20 (Kabat) | HCDR1 | SYAMS |
| SEQ ID NO: 21 (Kabat) | HCDR2 | VISAWGHVKYYADSVKG |
| SEQ ID NO: 22 (Kabat) | HCDR3 | WGDEGFDI |
| SEQ ID NO: 23 (Kabat) | LCDR1 | RASQGISNWLA |
| SEQ ID NO: 24 (Kabat) | LCDR2 | GASSLQS |
| SEQ ID NO: 25 (Kabat) | LCDR3 | QQYSSFPTT |
| SEQ ID NO: 26 (Chothia) | HCDR1 | GFTFSSY |
| SEQ ID NO: 27 (Chothia) | HCDR2 | SAWGHV |
| SEQ ID NO: 28 (Chothia) | HCDR3 | WGDEGFDI |
| SEQ ID NO: 29 (Chothia) | LCDR1 | SQGISNW |
| SEQ ID NO: 30 (Chothia) | LCDR2 | GAS |
| SEQ ID NO: 31 (Chothia) | LCDR3 | YSSFPT |
| SEQ ID NO: 32 | VL | DIQMTQSPSSLSASVGDRVTITCRASQGISNWLAWYQQKP GKAPKLLIYG ASSLQSGVPSRFSGSGSGTDFTLTISSLQPEDFAVYYCQQ YSSFPTTFGQ GTKVEIK |
| SEQ ID NO: 33 | VH | QVQLVESGGGLVQPGGSLRLSCAASGFTFSSYAMSWVRQA PGKGLEWVSV ISAWGHVKYYADSVKGRFTISRDNSKNTLYLQMNSLRAED TAVYYCARWG DEGFDIWGQGTLVTVSS |
| SEQ ID NO: 34 | DNA VL | GATATCCAGATGACCCAGAGCCCGTCTAGCCTGAGCGCGA GCGTGGGTGATCGTGTGACCATTACCTGCAGAGCGAGCCA GGGTATTTCTAATTGGCTGGCTTGGTACCAGCAGAAACCA GGTAAAGCACCGAAACTATTAATTTATGGTGCTTCTTCTT TGCAAAGCGGGGTCCCGTCCCGTTTTAGCGGCTCTGGATC CGGCACTGATTTTACCCTGACCATTAGCAGCCTGCAACCT GAAGACTTTGCGGTTTATTATTGCCAGCAGTATTCTTCTT TTCCTACTACCTTTGGCCAGGGTACGAAAGTTGAAATTAA A |
| SEQ ID NO: 35 | DNA VH | CAGGTGCAATTGGTGGAAAGCGGCGGCGGCCTGGTGCAAC CGGGCGGCAGCCTGCGTCTGAGCTGCGCGGCCTCCGGATT TACCTTTAGCAGCTATGCGATGAGCTGGGTGCGCCAAGCC CCTGGGAAGGGTCTCGAGTGGGTGAGCGTTATTTCTGCTT GGGGTCATGTTAAGTATTATGCTGATTCTGTTAAGGGTCG TTTTACCATTTCACGTGATAATTCGAAAAACACCCTGTAT CTGCAAATGAACAGCCTGCGTGCGGAAGATACGGCCGTGT ATTATTGCGCGCGTTGGGGTGATGAGGGTTTTGATATTTG GGGCCAAGGCACCCTGGTGACGGTTAGCTCA |
| SEQ ID NO: 36 | Light Kappa | DIQMTQSPSSLSASVGDRVTITCRASQGISNWLAWYQQKP GKAPKLLIYG ASSLQSGVPSRFSGSGSGTDFTLTISSLQPEDFAVYYCQQ YSSFPTTFGQ GTKVEIKRTVAAPSVFIFPPSDEQLKSGTASVVCLLNNFY PREAKVQWKV DNALQSGNSQESVTEQDSKDSTYSLSSTLTLSKADYEKHK VYACEVTHQG LSSPVTKSFNRGEC |
| SEQ ID NO: 37 | Heavy IgG1 | QVQLVESGGGLVQPGGSLRLSCAASGFTFSSYAMSWVRQA PGKGLEWVSV ISAWGHVKYYADSVKGRFTISRDNSKNTLYLQMNSLRAED TAVYYCARWG DEGFDIWGQGTLVTVSSASTKGPSVFPLAPSSKSTSGGTA ALGCLVKDYF PEPVTVSWNSGALTSGVHTFPAVLQSSGLYSLSSVVTVPS SSLGTQTYIC NVNHKPSNTKVDKRVEPKSCDKTHTCPPCPAPELLGGPSV FLFPPKPKDT LMISRTPEVTCVVVDVSHEDPEVKFNWYVDGVEVHNAKTK PREEQYNSTY RVVSVLTVLHQDWLNGKEYKCKVSNKALPAPIEKTISKAK GQPREPQVYT LPPSREEMTKNQVSLTCLVKGFYPSDIAVEWESNGQPENN YKTTPPVLDS DGSFFLYSKLTVDKSRWQQGNVFSCSVMHEALHNHYTQKS LSLSPGK |

| **MOR09825** | | |
|---|---|---|
| SEQ ID NO: 38 (Kabat) | HCDR1 | SYAMS |
| SEQ ID NO: 39 (Kabat) | HCDR2 | AINSQGKSTYYADSVKG |
| SEQ ID NO: 40 (Kabat) | HCDR3 | WGDEGFDI |
| SEQ ID NO: 41 (Kabat) | LCDR1 | RASQGISNWLA |
| SEQ ID NO: 42 (Kabat) | LCDR2 | GASSLQS |
| SEQ ID NO: 43 (Kabat) | LCDR3 | QQYSSFPTT |
| SEQ ID NO: 44 (Chothia) | HCDR1 | GFTFSSY |
| SEQ ID NO: 45 (Chothia) | HCDR2 | NSQGKS |
| SEQ ID NO: 46 (Chothia) | HCDR3 | WGDEGFDI |
| SEQ ID NO: 47 (Chothia) | LCDR1 | SQGISNW |
| SEQ ID NO: 48 (Chothia) | LCDR2 | GAS |
| SEQ ID NO: 49 (Chothia) | LCDR3 | YSSFPT |
| SEQ ID NO: 50 | VL | DIQMTQSPSSLSASVGDRVTITCRASQGISNWLAWYQQKP GKAPKLLIYG ASSLQSGVPSRFSGSGSGTDFTLTISSLQPEDFAVYYCQQ YSSFPTTFGQ GTKVEIK |
| SEQ ID NO: 51 | VH | QVQLVESGGGLVQPGGSLRLSCAASGFTFSSYAMSWVRQA PGKGLEWVSA INSQGKSTYYADSVKGRFTISRDNSKNTLYLQMNSLRAED TAVYYCARWG DEGFDIWGQGTLVTVSS |
| SEQ ID NO: 52 | DNA VL | GATATCCAGATGACCCAGAGCCCGTCTAGCCTGAGCGCGA GCGTGGGTGATCGTGTGACCATTACCTGCAGAGCGAGCCA GGGTATTTCTAATTGGCTGGCTTGGTACCAGCAGAAACCA GGTAAAGCACCGAAACTATTAATTTATGGTGCTTCTTCTT TGCAAAGCGGGGTCCCGTCCCGTTTTAGCGGCTCTGGATC CGGCACTGATTTTACCCTGACCATTAGCAGCCTGCAACCT GAAGACTTTGCGGTTTATTATTGCCAGCAGTATTCTTCTT TTCCTACTACCTTTGGCCAGGGTACGAAAGTTGAAATTAA A |
| SEQ ID NO: 53 | DNA VH | CAGGTGCAATTGGTGGAAAGCGGCGGCGGCCTGGTGCAAC CGGGCGGCAGCCTGCGTCTGAGCTGCGCGGCCTCCGGATT TACCTTTAGCAGCTATGCGATGAGCTGGGTGCGCCAAGCC CCTGGGAAGGGTCTCGAGTGGGTGAGCGCTATTAATTCTC AGGGTAAGTCTACTTATTATGCTGATTCTGTTAAGGGTCG TTTTACCATTTCACGTGATAATTCGAAAAACACCCTGTAT CTGCAAATGAACAGCCTGCGTGCGGAAGATACGGCCGTGT ATTATTGCGCGCGTTGGGGTGATGAGGGTTTTGATATTTG GGGCCAAGGCACCCTGGTGACGGTTAGCTCA |
| SEQ ID NO: 54 | Light Kappa | DIQMTQSPSSLSASVGDRVTITCRASQGISNWLAWYQQKP GKAPKLLIYG |
| | | ASSLQSGVPSRFSGSGSGTDFTLTISSLQPEDFAVYYCQQ YSSFPTTFGQ GTKVEIKRTVAAPSVFIFPPSDEQLKSGTASVVCLLNNFY PREAKVQWKV DNALQSGNSQESVTEQDSKDSTYSLSSTLTLSKADYEKHK VYACEVTHQG LSSPVTKSFNRGEC |
| SEQ ID NO: 55 | Heavy IgG1 | QVQLVESGGGLVQPGGSLRLSCAASGFTFSSYAMSWVRQA PGKGLEWVSA INSQGKSTYYADSVKGRFTISRDNSKNTLYLQMNSLRAED TAVYYCARWG DEGFDIWGQGTLVTVSSASTKGPSVFPLAPSSKSTSGGTA ALGCLVKDYF PEPVTVSWNSGALTSGVHTFPAVLQSSGLYSLSSVVTVPS SSLGTQTYIC NVNHKPSNTKVDKRVEPKSCDKTHTCPPCPAPELLGGPSV FLFPPKPKDT LMISRTPEVTCVVVDVSHEDPEVKFNWYVDGVEVHNAKTK PREEQYNSTY RVVSVLTVLHQDWLNGKEYKCKVSNKALPAPIEKTISKAK GQPREPQVYT LPPSREEMTKNQVSLTCLVKGFYPSDIAVEWESNGQPENN YKTTPPVLDS DGSFFLYSKLTVDKSRWQQGNVFSCSVMHEALHNHYTQKS LSLSPGK |

| **MOR09974** | | |
|---|---|---|
| SEQ ID NO: 56 (Kabat) | HCDR1 | SYAMS |
| SEQ ID NO: 57 (Kabat) | HCDR2 | VINPSGNFTNYADSVKG |
| SEQ ID NO: 58 (Kabat) | HCDR3 | WGDEGFDI |
| SEQ ID NO: 59 (Kabat) | LCDR1 | RASQGISNWLA |
| SEQ ID NO: 60 (Kabat) | LCDR2 | GASSLQS |
| SEQ ID NO: 61 (Kabat) | LCDR3 | QQYSSFPTT |
| SEQ ID NO: 62 (Chothia) | HCDR1 | GFTFSSY |
| SEQ ID NO: 63 (Chothia) | HCDR2 | NPSGNF |
| SEQ ID NO: 64 (Chothia) | HCDR3 | WGDEGFDI |
| SEQ ID NO: 65 (Chothia) | LCDR1 | SQGISNW |
| SEQ ID NO: 66 (Chothia) | LCDR2 | GAS |
| SEQ ID NO: 67 (Chothia) | LCDR3 | YSSFPT |
| SEQ ID NO: 68 | VL | DIQMTQSPSSLSASVGDRVTITCRASQGISNWLAWYQQKP GKAPKLLIYG |
| | | ASSLQSGVPSRFSGSGSGTDFTLTISSLQPEDFAVYYCQQ YSSFPTTFGQ GTKVEIK |
| SEQ ID NO: 69 | VH | QVQLVESGGGLVQPGGSLRLSCAASGFTFSSYAMSWVRQA PGKGLEWVSV INPSGNFTNYADSVKGRFTISRDNSKNTLYLQMNSLRAED TAVYYCARWG DEGFDIWGQGTLVTVSS |
| SEQ ID NO: 70 | DNA VL | GATATCCAGATGACCCAGAGCCCGTCTAGCCTGAGCGCGA GCGTGGGTGATCGTGTGACCATTACCTGCAGAGCGAGCCA GGGTATTTCTAATTGGCTGGCTTGGTACCAGCAGAAACCA GGTAAAGCACCGAAACTATTAATTTATGGTGCTTCTTCTT TGCAAAGCGGGGTCCCGTCCCGTTTTAGCGGCTCTGGATC CGGCACTGATTTTACCCTGACCATTAGCAGCCTGCAACCT GAAGACTTTGCGGTTTATTATTGCCAGCAGTATTCTTCTT TTCCTACTACCTTTGGCCAGGGTACGAAAGTTGAAATTAA A |
| SEQ ID NO: 71 | DNA VH | CAGGTGCAATTGGTGGAAAGCGGCGGCGGCCTGGTGCAAC CGGGCGGCAGCCTGCGTCTGAGCTGCGCGGCCTCCGGATT TACCTTTAGCAGCTATGCGATGAGCTGGGTGCGCCAAGCC CCTGGGAAGGGTCTCGAGTGGGTGAGCGTTATTAATCCTT CTGGTAATTTTACTAATTATGCTGATTCTGTTAAGGGTCG TTTTACCATTTCACGTGATAATTCGAAAAACACCCTGTAT CTGCAAATGAACAGCCTGCGTGCGGAAGATACGGCCGTGT ATTATTGCGCGCGTTGGGGTGATGAGGGTTTTGATATTTG GGGCCAAGGCACCCTGGTGACGGTTAGCTCA |
| SEQ ID NO: 72 | Light Kappa | DIQMTQSPSSLSASVGDRVTITCRASQGISNWLAWYQQKP GKAPKLLIYG ASSLQSGVPSRFSGSGSGTDFTLTISSLQPEDFAVYYCQQ YSSFPTTFGQ GTKVEIKRTVAAPSVFIFPPSDEQLKSGTASVVCLLNNFY PREAKVQWKV DNALQSGNSQESVTEQDSKDSTYSLSSTLTLSKADYEKHK VYACEVTHQG LSSPVTKSFNRGEC |
| SEQ ID NO: 73 | Heavy IgG1 | QVQLVESGGGLVQPGGSLRLSCAASGFTFSSYAMSWVRQA PGKGLEWVSV INPSGNFTNYADSVKGRFTISRDNSKNTLYLQMNSLRAED TAVYYCARWG DEGFDIWGQGTLVTVSSASTKGPSVFPLAPSSKSTSGGTA ALGCLVKDYF PEPVTVSWNSGALTSGVHTFPAVLQSSGLYSLSSVVTVPS SSLGTQTYIC NVNHKPSNTKVDKRVEPKSCDKTHTCPPCPAPELLGGPSV FLFPPKPKDT LMISRTPEVTCVVVDVSHEDPEVKFNWYVDGVEVHNAKTK PREEQYNSTY RVVSVLTVLHQDWLNGKEYKCKVSNKALPAPIEKTISKAK GQPREPQVYT LPPSREEMTKNQVSLTCLVKGFYPSDIAVEWESNGQPENN YKTTPPVLDS DGSFFLYSKLTVDKSRWQQGNVFSCSVMHEALHNHYTQKS LSLSPGK |

| **MOR10452** | | |
|---|---|---|
| SEQ ID NO: 74 (Kabat) | HCDR1 | SYAMS |
| SEQ ID NO: 75 (Kabat) | HCDR2 | NTSPIGYTYYAGSVKG |
| SEQ ID NO: 76 (Kabat) | HCDR3 | WGDEGFDI |
| SEQ ID NO: 77 (Kabat) | LCDR1 | RASQGISNWLA |
| SEQ ID NO: 78 (Kabat) | LCDR2 | GASSLQS |
| SEQ ID NO: 79 (Kabat) | LCDR3 | QQYSSFPTT |
| SEQ ID NO: 80 (Chothia) | HCDR1 | GFTFSSY |
| SEQ ID NO: 81 (Chothia) | HCDR2 | SPIGY |
| SEQ ID NO: 82 (Chothia) | HCDR3 | WGDEGFDI |
| SEQ ID NO: 83 (Chothia) | LCDR1 | SQGISNW |
| SEQ ID NO: 84 (Chothia) | LCDR2 | GAS |
| SEQ ID NO: 85 (Chothia) | LCDR3 | YSSFPT |
| SEQ ID NO: 86 | VL | DIQMTQSPSSLSASVGDRVTITCRASQGISNWLAWYQQKP GKAPKLLIYG ASSLQSGVPSRFSGSGSGTDFTLTISSLQPEDFAVYYCQQ YSSFPTTFGQ GTKVEIK |
| SEQ ID NO: 87 | VH | QVQLVESGGGLVQPGGSLRLSCAASGFTFSSYAMSWVRQA PGKGLEWVSN TSPIGYTYYAGSVKGRFTISRDNSKNTLYLQMNSLRAEDT AVYYCARWGD EGFDIWGQGTLVTVSS |
| SEQ ID NO: 88 | DNA VL | GATATCCAGATGACCCAGAGCCCGTCTAGCCTGAGCGCGA GCGTGGGTGATCGTGTGACCATTACCTGCAGAGCGAGCCA GGGTATTTCTAATTGGCTGGCTTGGTACCAGCAGAAACCA GGTAAAGCACCGAAACTATTAATTTATGGTGCTTCTTCTT TGCAAAGCGGGGTCCCGTCCCGTTTTAGCGGCTCTGGATC CGGCACTGATTTTACCCTGACCATTAGCAGCCTGCAACCT GAAGACTTTGCGGTTTATTATTGCCAGCAGTATTCTTCTT TTCCTACTACCTTTGGCCAGGGTACGAAAGTTGAAATTAA A |
| SEQ ID NO: 89 | DNA VH | CAGGTGCAATTGGTGGAAAGCGGCGGCGGCCTGGTGCAAC CGGGCGGCAGCCTGCGTCTGAGCTGCGCGGCCTCCGGATT TACCTTTAGCAGCTATGCGATGAGCTGGGTGCGCCAAGCC CCTGGGAAGGGTCTCGAGTGGGTGAGCAATACTTCTCCTA TTGGTTATACTTATTATGCTGGTTCTGTTAAGGGTCGTTT TACCATTTCACGTGATAATTCGAAAAACACCCTGTATCTG CAAATGAACAGCCTGCGTGCGGAAGATACGGCCGTGTATT ATTGCGCGCGTTGGGGTGATGAGGGTTTTGATATTTGGGG |
| | | CCAAGGCACCCTGGTGACGGTTAGCTCA |
| SEQ ID NO: 90 | Light Kappa | DIQMTQSPSSLSASVGDRVTITCRASQGISNWLAWYQQKP GKAPKLLIYG ASSLQSGVPSRFSGSGSGTDFTLTISSLQPEDFAVYYCQQ YSSFPTTFGQ GTKVEIKRTVAAPSVFIFPPSDEQLKSGTASVVCLLNNFY PREAKVQWKV DNALQSGNSQESVTEQDSKDSTYSLSSTLTLSKADYEKHK VYACEVTHQG LSSPVTKSFNRGEA |
| SEQ ID NO: 91 | Heavy Chain (only VH and CH1 domains) | QVQLVESGGGLVQPGGSLRLSCAASGFTFSSYAMSWVRQA PGKGLEWVSN TSPIGYTYYAGSVKGRFTISRDNSKNTLYLQMNSLRAEDT AVYYCARWGD EGFDIWGQGTLVTVSSASTKGPSVFPLAPSSKSTSGGTAA LGCLVKDYFP EPVTVSWNSGALTSGVHTFPAVLQSSGLYSLSSVVTVPSS SLGTQTYICN VNHKPSNTKVDKKVEPKS |

| **MOR10701** | | |
|---|---|---|
| SEQ ID NO: 92 (Kabat) | HCDR1 | SYAMS |
| SEQ ID NO: 93 (Kabat) | HCDR2 | VTGAVGRSTYYPDSVKG |
| SEQ ID NO: 94 (Kabat) | HCDR3 | WGDEGFDI |
| SEQ ID NO: 95 (Kabat) | LCDR1 | RASQGISNWLA |
| SEQ ID NO: 96 (Kabat) | LCDR2 | GASSLQS |
| SEQ ID NO: 97 (Kabat) | LCDR3 | QQYSSFPTT |
| SEQ ID NO: 98 (Chothia) | HCDR1 | GFTFSSY |
| SEQ ID NO: 99 (Chothia) | HCDR2 | GAVGRS |
| SEQ ID NO: 100 (Chothia) | HCDR3 | WGDEGFDI |
| SEQ ID NO: 101 (Chothia) | LCDR1 | SQGISNW |
| SEQ ID NO: 102 (Chothia) | LCDR2 | GAS |
| SEQ ID NO: 103 (Chothia) | LCDR3 | YSSFPT |
| SEQ ID NO: 104 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 105 | VH | EVQLLESGGGLVQPGGSLRLSCAASGFTFSSYAMSWVRQA PGKGLEWVSVTGAVGRSTYYPDSVKGRFTISRDNSKNTLY LQMNSLRAEDTAVYYCARWGDEGFDIWGQGTLVTVSS |
| SEQ ID NO: 106 | DNA VL | GATATCCAGATGACCCAGAGCCCCAGCAGCCTGAGCGCCA GCGTGGGCGACAGAGTGACCATCACCTGTCGGGCCAGCCA |
| | | GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 107 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGTGACAGGCGCCG TGGGCAGAAGCACCTACTACCCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 108 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 109 | Heavy IgG1 | EVQLLESGGGLVQPGGSLRLSCAASGFTFSSYAMSWVRQA PGKGLEWVSVTGAVGRSTYYPDSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10702** | | |
|---|---|---|
| SEQ ID NO: 110 (Kabat) | HCDR1 | SYAMS |
| SEQ ID NO: 111 (Kabat) | HCDR2 | VISAWGHVKYYADSVKG |
| SEQ ID NO: 112 (Kabat) | HCDR3 | WGDEGFDI |
| SEQ ID NO: 113 (Kabat) | LCDR1 | RASQGISNWLA |
| SEQ ID NO: 114 (Kabat) | LCDR2 | GASSLQS |
| SEQ ID NO: 115 (Kabat) | LCDR3 | QQYSSFPTT |
| SEQ ID NO: 116 (Chothia) | HCDR1 | GFTFSSY |
| SEQ ID NO: 117 (Chothia) | HCDR2 | SAWGHV |
| SEQ ID NO: 118 (Chothia) | HCDR3 | WGDEGFDI |
| SEQ ID NO: 119 (Chothia) | LCDR1 | SQGISNW |
| SEQ ID NO: 120 (Chothia) | LCDR2 | GAS |
| SEQ ID NO: 121 (Chothia) | LCDR3 | YSSFPT |
| SEQ ID NO: 122 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 123 | VH | EVQLLESGGGLVQPGGSLRLSCAASGFTFSSYAMSWVRQA PGKGLEWVSVISAWGHVKYYADSVKGRFTISRDNSKNTLY LQMNSLRAEDTAVYYCARWGDEGFDIWGQGTLVTVSS |
| SEQ ID NO: 124 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 125 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGTGATCAGCGCCT GGGGCCACGTGAAGTACTACGCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 126 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 127 | Heavy IgG1 | EVQLLESGGGLVQPGGSLRLSCAASGFTFSSYAMSWVRQP GKGLEWVSVISAWGHVKYYADSVKGRFTISRDNSKNTLYL QMNSLRAEDTAVYYCARWGDEGFDIWGQGTLVTVSSASTK GPSVFPLAPSSKSTSGGTAALGCLVKDYFPEPVTVSWNSG ALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10703** | | |
|---|---|---|
| SEQ ID NO: 128 (Kabat) | HCDR1 | SYAMS |
| SEQ ID NO: 129 (Kabat) | HCDR2 | AINSQGKSTYYADSVKG |
| SEQ ID NO: 130 (Kabat) | HCDR3 | WGDEGFDI |
| SEQ ID NO: 131 (Kabat) | LCDR1 | RASQGISNWLA |
| SEQ ID NO: 132 (Kabat) | LCDR2 | GASSLQS |
| SEQ ID NO: 133 (Kabat) | LCDR3 | QQYSSFPTT |
| SEQ ID NO: 134 (Chothia) | HCDR1 | GFTFSSY |
| SEQ ID NO: 135 (Chothia) | HCDR2 | NSQGKS |
| SEQ ID NO: 136 (Chothia) | HCDR3 | WGDEGFDI |
| SEQ ID NO: 137 (Chothia) | LCDR1 | SQGISNW |
| SEQ ID NO: 138 (Chothia) | LCDR2 | GAS |
| SEQ ID NO: 139 (Chothia) | LCDR3 | YSSFPT |
| SEQ ID NO: 140 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 141 | VH | EVQLLESGGGLVQPGGSLRLSCAASGFTFSSYAMSWVRQA PGKGLEWVSAINSQGKSTYYADSVKGRFTISRDNSKNTLY LQMNSLRAEDTAVYYCARWGDEGFDIWGQGTLVTVSS |
| SEQ ID NO: 142 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 143 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGCCATCAACAGCC AGGGCAAGAGCACCTACTACGCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 144 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG |
| | | LSSPVTKSFNRGEC |
| SEQ ID NO: 145 | Heavy IgG1 | EVQLLESGGGLVQPGGSLRLSCAASGFTFSSYAMSWVRQA PGKGLEWVSAINSQGKSTYYADSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10703 N52S** | | |
|---|---|---|
| SEQ ID NO: 146 (Kabat) | HCDR1 | SYAMS |
| SEQ ID NO: 147 (Kabat) | HCDR2 | AI**S**SQGKSTYYADSVKG |
| SEQ ID NO: 148 (Kabat) | HCDR3 | WGDEGFDI |
| SEQ ID NO: 149 (Kabat) | LCDR1 | RASQGISNWLA |
| SEQ ID NO: 150 (Kabat) | LCDR2 | GASSLQS |
| SEQ ID NO: 151 (Kabat) | LCDR3 | QQYSSFPTT |
| SEQ ID NO: 152 (Chothia) | HCDR1 | GFTFSSY |
| SEQ ID NO: 153 (Chothia) | HCDR2 | SSQGKS |
| SEQ ID NO: 154 (Chothia) | HCDR3 | WGDEGFDI |
| SEQ ID NO: 155 (Chothia) | LCDR1 | SQGISNW |
| SEQ ID NO: 156 (Chothia) | LCDR2 | GAS |
| SEQ ID NO: 157 (Chothia) | LCDR3 | YSSFPT |
| SEQ ID NO: 158 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 159 | VH | EVQLLESGGGLVQPGGSLRLSCAASGFTFSSYAMSWVRQA PGKGLEWVSAISSQGKSTYYADSVKGRFTISRDNSKNTLY LQMNSLRAEDTAVYYCARWGDEGFDIWGQGTLVTVSS |
| SEQ ID NO: 160 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT |
| | | TCCCCACCACCTTCGGCCAGGGCACCAAGGTGGAAATCAA G |
| SEQ ID NO: 161 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGCCATCAGCAGCC AGGGCAAGAGCACCTACTACGCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 162 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 163 | Heavy IgG1 | EVQLLESGGGLVQPGGSLRLSCAASGFTFSSYAMSWVRQA PGKGLEWVSAISSQGKSTYYADSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10703 N52G** | | |
|---|---|---|
| SEQ ID NO: 164 (Kabat) | HCDR1 | SYAMS |
| SEQ ID NO: 165 (Kabat) | HCDR2 | AI**G**SQGKSTYYADSVKG |
| SEQ ID NO: 166 (Kabat) | HCDR3 | WGDEGFDI |
| SEQ ID NO: 167 (Kabat) | LCDR1 | RASQGISNWLA |
| SEQ ID NO: 168 (Kabat) | LCDR2 | GASSLQS |
| SEQ ID NO: 169 (Kabat) | LCDR3 | QQYSSFPTT |
| SEQ ID NO: 170 (Chothia) | HCDR1 | GFTFSSY |
| SEQ ID NO: 171 (Chothia) | HCDR2 | **G**SQGKS |
| SEQ ID NO: 172 (Chothia) | HCDR3 | WGDEGFDI |
| SEQ ID NO: 173 (Chothia) | LCDR1 | SQGISNW |
| SEQ ID NO: 174 (Chothia) | LCDR2 | GAS |
| SEQ ID NO: 175 (Chothia) | LCDR3 | YSSFPT |
| SEQ ID NO: 176 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 177 | VH | EVQLLESGGGLVQPGGSLRLSCAASGFTFSSYAMSWVRQA APGKGLEWVSAIGSQGKSTYYADSVKGRFTISRDNSKNTLY LQMNSLRAEDTAVYYCARWGDEGFDIWGQGTLVTVSS |
| SEQ ID NO: 178 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 179 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGCCATCGGCAGCC AGGGCAAGAGCACCTACTACGCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 180 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 181 | Heavy IgG1 | EVQLLESGGGLVQPGGSLRLSCAASGFTFSSYAMSWVRQA PGKGLEWVSAIGSQGKSTYYADSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10703 N52S_S52aN** | | |
|---|---|---|
| SEQ ID NO: 182 (Kabat) | HCDR1 | SYAMS |
| SEQ ID NO: 183 (Kabat) | HCDR2 | AI**SN**QGKSTYYADSVKG |
| SEQ ID NO: 184 (Kabat) | HCDR3 | WGDEGFDI |
| SEQ ID NO: 185 (Kabat) | LCDR1 | RASQGISNWLA |
| SEQ ID NO: 186 (Kabat) | LCDR2 | GASSLQS |
| SEQ ID NO: 187 (Kabat) | LCDR3 | QQYSSFPTT |
| SEQ ID NO: 188 (Chothia) | HCDR1 | GFTFSSY |
| SEQ ID NO: 189 (Chothia) | HCDR2 | **SN**QGKS |
| SEQ ID NO: 190 (Chothia) | HCDR3 | WGDEGFDI |
| SEQ ID NO: 191 (Chothia) | LCDR1 | SQGISNW |
| SEQ ID NO: 192 (Chothia) | LCDR2 | GAS |
| SEQ ID NO: 193 (Chothia) | LCDR3 | YSSFPT |
| SEQ ID NO: 194 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 195 | VH | EVQLLESGGGLVQPGGSLRLSCAASGFTFSSYAMSWVRQA PGKGLEWVSAISNQGKSTYYADSVKGRFTISRDNSKNTLY LQMNSLRAEDTAVYYCARWGDEGFDIWGQGTLVTVSS |
| SEQ ID NO: 196 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 197 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGCCATCAGCAACC AGGGCAAGAGCACCTACTACGCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 198 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 199 | Heavy IgG1 | EVQLLESGGGLVQPGGSLRLSCAASGFTFSSYAMSWVRQA PGKGLEWVSAISNQGKSTYYADSVKGRFTISRDNSKNTLY LQMNSLRAEDTAVYYCARWGDEGFDIWGQGTLVTVSSAST |
| | | KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10703 A50V_N52S** | | |
|---|---|---|
| SEQ ID NO: 200 (Kabat) | HCDR1 | SYAMS |
| SEQ ID NO: 201 (Kabat) | HCDR2 | **V**I**S**SQGKSTYYADSVKG |
| SEQ ID NO: 202 (Kabat) | HCDR3 | WGDEGFDI |
| SEQ ID NO: 203 (Kabat) | LCDR1 | RASQGISNWLA |
| SEQ ID NO: 204 (Kabat) | LCDR2 | GASSLQS |
| SEQ ID NO: 205 (Kabat) | LCDR3 | QQYSSFPTT |
| SEQ ID NO: 206 (Chothia) | HCDR1 | GFTFSSY |
| SEQ ID NO: 207 (Chothia) | HCDR2 | **S**SQGKS |
| SEQ ID NO: 208 (Chothia) | HCDR3 | WGDEGFDI |
| SEQ ID NO: 209 (Chothia) | LCDR1 | SQGISNW |
| SEQ ID NO: 210 (Chothia) | LCDR2 | GAS |
| SEQ ID NO: 211 (Chothia) | LCDR3 | YSSFPT |
| SEQ ID NO: 212 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 213 | VH | EVQLLESGGGLVQPGGSLRLSCAASGFTFSSYAMSWVRQA PGKGLEWVSVISSQGKSTYYADSVKGRFTISRDNSKNTLY LQMNSLRAEDTAVYYCARWGDEGFDIWGQGTLVTVSS |
| SEQ ID NO: 214 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 215 | DNA VH | GAGGTGCAATTGCTGGAAAGCGGCGGAGGCCTGGTGCAGC CTGGCGGCAGCCTGAGACTGTCTTGCGCCGCCAGCGGCTT |
| | | CACCTTCAGCAGCTACGCCATGAGCTGGGTCCGCCAGGCC CCTGGCAAGGGACTGGAATGGGTGTCCGTCATCAGCAGCC AGGGCAAGAGCACCTACTACGCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 216 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 217 | Heavy IgG1 | EVQLLESGGGLVQPGGSLRLSCAASGFTFSSYAMSWVRQA PGKGLEWVSVISSQGKSTYYADSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10703 A50V_N52G** | | |
|---|---|---|
| SEQ ID NO: 218 (Kabat) | HCDR1 | SYAMS |
| SEQ ID NO: 219 (Kabat) | HCDR2 | **V**I**G**SQGKSTYYADSVKG |
| SEQ ID NO: 220 (Kabat) | HCDR3 | WGDEGFDI |
| SEQ ID NO: 221 (Kabat) | LCDR1 | RASQGISNWLA |
| SEQ ID NO: 222 (Kabat) | LCDR2 | GASSLQS |
| SEQ ID NO: 223 (Kabat) | LCDR3 | QQYSSFPTT |
| SEQ ID NO: 224 (Chothia) | HCDR1 | GFTFSSY |
| SEQ ID NO: 225 (Chothia) | HCDR2 | **G**SQGKS |
| SEQ ID NO: 226 (Chothia) | HCDR3 | WGDEGFDI |
| SEQ ID NO: 227 (Chothia) | LCDR1 | SQGISNW |
| SEQ ID NO: 228 (Chothia) | LCDR2 | GAS |
| SEQ ID NO: 229 (Chothia) | LCDR3 | YSSFPT |
| SEQ ID NO: 230 | VL | DIQMTQSPSSLSASVGDRVTITCRASQGISNWLAWYQQKP |
| | | GKAPKLLIYGASSLQSGVPSRFSGSGSGTDFTLTISSLQP EDFATYYCQQYSSFPTTFGQGTKVEIK |
| SEQ ID NO: 231 | VH | EVQLLESGGGLVQPGGSLRLSCAASGFTFSSYAMSWVRQA PGKGLEWVSVIGSQGKSTYYADSVKGRFTISRDNSKNTLY LQMNSLRAEDTAVYYCARWGDEGFDIWGQGTLVTVSS |
| SEQ ID NO: 232 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 233 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGTCATCGGCAGCC AGGGCAAGAGCACCTACTACGCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 234 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 235 | Heavy IgG1 | EVQLLESGGGLVQPGGSLRLSCAASGFTFSSYAMSWVRQA PGKGLEWVSVIGSQGKSTYYADSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10703 S52aA** | | |
|---|---|---|
| SEQ ID NO: 236 (Kabat) | HCDR1 | SYAMS |
| SEQ ID NO: 237 (Kabat) | HCDR2 | AIN**A**QGKSTYYADSVKG |
| SEQ ID NO: 238 (Kabat) | HCDR3 | WGDEGFDI |
| SEQ ID NO: 239 (Kabat) | LCDR1 | RASQGISNWLA |
| SEQ ID NO: 240 (Kabat) | LCDR2 | GASSLQS |
| SEQ ID NO: 241 (Kabat) | LCDR3 | QQYSSFPTT |
| SEQ ID NO: 242 (Chothia) | HCDR1 | GFTFSSY |
| SEQ ID NO: 243 (Chothia) | HCDR2 | N**A**QGKS |
| SEQ ID NO: 244 (Chothia) | HCDR3 | WGDEGFDI |
| SEQ ID NO: 245 (Chothia) | LCDR1 | SQGISNW |
| SEQ ID NO: 246 (Chothia) | LCDR2 | GAS |
| SEQ ID NO: 247 (Chothia) | LCDR3 | YSSFPT |
| SEQ ID NO: 248 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 249 | VH | EVQLLESGGGLVQPGGSLRLSCAASGFTFSSYAMSWVRQA PGKGLEWVSAINAQGKSTYYADSVKGRFTISRDNSKNTLY LQMNSLRAEDTAVYYCARWGDEGFDIWGQGTLVTVSS |
| SEQ ID NO: 250 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 251 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGCCATCAACGCCC AGGGCAAGAGCACCTACTACGCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 252 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 253 | Heavy IgG1 | EVQLLESGGGLVQPGGSLRLSCAASGFTFSSYAMSWVRQA PGKGLEWVSAINAQGKSTYYADSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD |
| | | GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10703 S52aT** | | |
|---|---|---|
| SEQ ID NO: 254 (Kabat) | HCDR1 | SYAMS |
| SEQ ID NO: 255 (Kabat) | HCDR2 | AIN**T**QGKSTYYADSVKG |
| SEQ ID NO: 256 (Kabat) | HCDR3 | WGDEGFDI |
| SEQ ID NO: 257 (Kabat) | LCDR1 | RASQGISNWLA |
| SEQ ID NO: 258 (Kabat) | LCDR2 | GASSLQS |
| SEQ ID NO: 259 (Kabat) | LCDR3 | QQYSSFPTT |
| SEQ ID NO: 260 (Chothia) | HCDR1 | GFTFSSY |
| SEQ ID NO: 261 (Chothia) | HCDR2 | N**T**QGKS |
| SEQ ID NO: 262 (Chothia) | HCDR3 | WGDEGFDI |
| SEQ ID NO: 263 (Chothia) | LCDR1 | SQGISNW |
| SEQ ID NO: 264 (Chothia) | LCDR2 | GAS |
| SEQ ID NO: 265 (Chothia) | LCDR3 | YSSFPT |
| SEQ ID NO: 266 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 267 | VH | EVQLLESGGGLVQPGGSLRLSCAASGFTFSSYAMSWVRQA PGKGLEWVSAINTQGKSTYYADSVKGRFTISRDNSKNTLY LQMNSLRAEDTAVYYCARWGDEGFDIWGQGTLVTVSS |
| SEQ ID NO: 268 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 269 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGCCATCAACACCC AGGGCAAGAGCACCTACTACGCCGACAGCGTGAAGGGCCG GTTCACCATCAGCCGGGACAACAGCAAGAACACCCTGTAC |
| | | CTGCAGATGAACAGCCTGCGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 270 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 271 | Heavy IgG1 | EVQLLESGGGLVQPGGSLRLSCAASGFTFSSYAMSWVRQA PGKGLEWVSAINTQGKSTYYADSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10701 R55S** | | |
|---|---|---|
| SEQ ID NO: 272 (Kabat) | HCDR1 | SYAMS |
| SEQ ID NO: 273 (Kabat) | HCDR2 | VTGAVGSS**T**YYPDSVKG |
| SEQ ID NO: 274 (Kabat) | HCDR3 | WGDEGFDI |
| SEQ ID NO: 275 (Kabat) | LCDR1 | RASQGISNWLA |
| SEQ ID NO: 276 (Kabat) | LCDR2 | GASSLQS |
| SEQ ID NO: 277 (Kabat) | LCDR3 | QQYSSFPTT |
| SEQ ID NO: 278 (Chothia) | HCDR1 | GFTFSSY |
| SEQ ID NO: 279 (Chothia) | HCDR2 | GAVG**S**S |
| SEQ ID NO: 280 (Chothia) | HCDR3 | WGDEGFDI |
| SEQ ID NO: 281 (Chothia) | LCDR1 | SQGISNW |
| SEQ ID NO: 282 (Chothia) | LCDR2 | GAS |
| SEQ ID NO: 283 (Chothia) | LCDR3 | YSSFPT |
| SEQ ID NO: 284 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 285 | VH | EVQLLESGGGLVQPGGSLRLSCAASGFTFSSYAMSWVRQA PGKGLEWVSVTGAVGSSTYYPDSVKGRFTISRDNSKNTLY LQMNSLRAEDTAVYYCARWGDEGFDIWGQGTLVTVSS |
| SEQ ID NO: 286 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 287 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGTGACAGGCGCCG TGGGCAGCAGCACCTACTACCCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 288 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 289 | Heavy IgG1 | EVQLLESGGGLVQPGGSLRLSCAASGFTFSSYAMSWVRQA PGKGLEWVSVTGAVGSSTYYPDSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10701 R55G** | | |
|---|---|---|
| SEQ ID NO: 290 (Kabat) | HCDR1 | SYAMS |
| SEQ ID NO: 291 (Kabat) | HCDR2 | VTGAV**G**GSTYYPDSVKG |
| SEQ ID NO: 292 (Kabat) | HCDR3 | WGDEGFDI |
| SEQ ID NO: 293 (Kabat) | LCDR1 | RASQGISNWLA |
| SEQ ID NO: 294 (Kabat) | LCDR2 | GASSLQS |
| SEQ ID NO: 295 (Kabat) | LCDR3 | QQYSSFPTT |
| SEQ ID NO: 296 (Chothia) | HCDR1 | GFTFSSY |
| SEQ ID NO: 297 (Chothia) | HCDR2 | GAVG**G**S |
| SEQ ID NO: 298 (Chothia) | HCDR3 | WGDEGFDI |
| SEQ ID NO: 299 (Chothia) | LCDR1 | SQGISNW |
| SEQ ID NO: 300 (Chothia) | LCDR2 | GAS |
| SEQ ID NO: 301 (Chothia) | LCDR3 | YSSFPT |
| SEQ ID NO: 302 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 303 | VH | EVQLLESGGGLVQPGGSLRLSCAASGFTFSSYAMSWVRQA PGKGLEWVSVTGAVGGSTYYPDSVKGRFTISRDNSKNTLY LQMNSLRAEDTAVYYCARWGDEGFDIWGQGTLVTVSS |
| SEQ ID NO: 304 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 305 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGTGACAGGCGCCG TGGGCGGAAGCACCTACTACCCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 306 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 307 | Heavy IgG1 | EVQLLESGGGLVQPGGSLRLSCAASGFTFSSYAMSWVRQA PGKGLEWVSVTGAVGGSTYYPDSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10701 R55K** | | |
|---|---|---|
| SEQ ID NO: 308 (Kabat) | HCDR1 | SYAMS |
| SEQ ID NO: 309 (Kabat) | HCDR2 | VTGAVG**K**STYYPDSVKG |
| SEQ ID NO: 310 (Kabat) | HCDR3 | WGDEGFDI |
| SEQ ID NO: 311 (Kabat) | LCDR1 | RASQGISNWLA |
| SEQ ID NO: 312 (Kabat) | LCDR2 | GASSLQS |
| SEQ ID NO: 313 (Kabat) | LCDR3 | QQYSSFPTT |
| SEQ ID NO: 314 (Chothia) | HCDR1 | GFTFSSY |
| SEQ ID NO: 315 (Chothia) | HCDR2 | GAVG**K**S |
| SEQ ID NO: 316 (Chothia) | HCDR3 | WGDEGFDI |
| SEQ ID NO: 317 (Chothia) | LCDR1 | SQGISNW |
| SEQ ID NO: 318 (Chothia) | LCDR2 | GAS |
| SEQ ID NO: 319 (Chothia) | LCDR3 | YSSFPT |
| SEQ ID NO: 320 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 321 | VH | EVQLLESGGGLVQPGGSLRLSCAASGFTFSSYAMSWVRQA PGKGLEWVSVTGAVGKSTYYPDSVKGRFTISRDNSKNTLY LQMNSLRAEDTAVYYCARWGDEGFDIWGQGTLVTVSS |
| SEQ ID NO: 322 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 323 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGTGACAGGCGCCG TGGGCAAAAGCACCTACTACCCCGACAGCGTGAAGGGCCG GTTCACCATCAGCCGGGACAACAGCAAGAACACCCTGTAC CTGCAGATGAACAGCCTGCGGGCCGAGGACACCGCCGTGT ACTACTGTGCCAGATGGGGCGACGAGGGCTTCGACATCTG GGGCCAGGGCACCCTGGTCACCGTCAGCTCA |
| SEQ ID NO: 324 | Light Kappa | DIQMTQSPSSLSASVGDRVTITCRASQGISNWLAWYQQKP GKAPKLLIYGASSLQSGVPSRFSGSGSGTDFTLTISSLQP |
| | | EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 325 | Heavy IgG1 | EVQLLESGGGLVQPGGSLRLSCAASGFTFSSYAMSWVRQA PGKGLEWVSVTGAVGKSTYYPDSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR10701 deletion S56** | | |
|---|---|---|
| SEQ ID NO: 326 (Kabat) | HCDR1 | SYAMS |
| SEQ ID NO: 327 (Kabat) | HCDR2 | VTGAVGRTYYPDSVKG |
| SEQ ID NO: 328 (Kabat) | HCDR3 | WGDEGFDI |
| SEQ ID NO: 329 (Kabat) | LCDR1 | RASQGISNWLA |
| SEQ ID NO: 330 (Kabat) | LCDR2 | GASSLQS |
| SEQ ID NO: 331 (Kabat) | LCDR3 | QQYSSFPTT |
| SEQ ID NO: 332 (Chothia) | HCDR1 | GFTFSSY |
| SEQ ID NO: 333 (Chothia) | HCDR2 | GAVGRT |
| SEQ ID NO: 334 (Chothia) | HCDR3 | WGDEGFDI |
| SEQ ID NO: 335 (Chothia) | LCDR1 | SQGISNW |
| SEQ ID NO: 336 (Chothia) | LCDR2 | GAS |
| SEQ ID NO: 337 (Chothia) | LCDR3 | YSSFPT |
| SEQ ID NO: 338 | VL | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIK |
| SEQ ID NO: 339 | VH | EVQLLESGGGLVQPGGSLRLSCAASGFTFSSYAMSWVRQA PGKGLEWVSVTGAVGRTYYPDSVKGRFTISRDNSKNTLYL QMNSLRAEDTAVYYCARWGDEGFDIWGQGTLVTVSS |
| SEQ ID NO: 340 | DNA VL | GATATCCAGATGACCCAGAGCCCCAGCAGCCTGAGCGCCA GCGTGGGCGACAGAGTGACCATCACCTGTCGGGCCAGCCA GGGCATCAGCAACTGGCTGGCCTGGTATCAGCAGAAGCCC GGCAAGGCCCCCAAGCTGCTGATCTACGGCGCCAGCTCCC |
| | | TGCAGAGCGGCGTGCCAAGCAGATTCAGCGGCAGCGGCTC CGGCACCGACTTCACCCTGACCATCAGCAGCCTGCAGCCC GAGGACTTCGCCACCTACTACTGCCAGCAGTACAGCAGCT TCCCCACCACCTTCGGCCAGGGCACCAAGGTGGAAATCAA G |
| SEQ ID NO: 341 | DNA VH | GAGGTGCAATTGCTGGAAAGCGGCGGAGGCCTGGTGCAGC CTGGCGGCAGCCTGAGACTGTCTTGCGCCGCCAGCGGCTT CACCTTCAGCAGCTACGCCATGAGCTGGGTCCGCCAGGCC CCTGGCAAGGGACTGGAATGGGTGTCCGTGACAGGCGCCG TGGGCAGAACCTACTACCCCGACAGCGTGAAGGGCCGGTT CACCATCAGCCGGGACAACAGCAAGAACACCCTGTACCTG CAGATGAACAGCCTGCGGGCCGAGGACACCGCCGTGTACT ACTGTGCCAGATGGGGCGACGAGGGCTTCGACATCTGGGG CCAGGGCACCCTGGTCACCGTCAGCTCA |
| SEQ ID NO: 342 | Light Kappa | DIQMTQSPSSLSASVGDRVTITCRASQGISNWLAWYQQKP GKAPKLLIYGASSLQSGVPSRFSGSGSGTDFTLTISSLQP EDFATYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 343 | Heavy IgG1 | EVQLLESGGGLVQPGGSLRLSCAASGFTFSSYAMSWVRQA PGKGLEWVSVTGAVGRTYYPDSVKGRFTISRDNSKNTLYL QMNSLRAEDTAVYYCARWGDEGFDIWGQGTLVTVSSASTK GPSVFPLAPSSKSTSGGTAALGCLVKDYFPEPVTVSWNSG ALTSGVHTFPAVLQSSGLYSLSSVVTVPSSSLGTQTYICN VNHKPSNTKVDKRVEPKSCDKTHTCPPCPAPELLGGPSVF LFPPKPKDTLMISRTPEVTCVVVDVSHEDPEVKFNWYVDG VEVHNAKTKPREEQYNSTYRVVSVLTVLHQDWLNGKEYKC KVSNKALPAPIEKTISKAKGQPREPQVYTLPPSREEMTKN QVSLTCLVKGFYPSDIAVEWESNGQPENNYKTTPPVLDSD GSFFLYSKLTVDKSRWQQGNVFSCSVMHEALHNHYTQKSL SLSPGK |

| **MOR12609** | | |
|---|---|---|
| SEQ ID NO: 344 (Kabat) | HCDR1 | SYAMS |
| SEQ ID NO: 345 (Kabat) | HCDR2 | VINGLGYTTFYADSVKG |
| SEQ ID NO: 346 (Kabat) | HCDR3 | WGDEGFDI |
| SEQ ID NO: 347 (Kabat) | LCDR1 | RASQGISNWLA |
| SEQ ID NO: 348 (Kabat) | LCDR2 | GASSLQS |
| SEQ ID NO: 349 (Kabat) | LCDR3 | QQYSSFPTT |
| SEQ ID NO: 350 (Chothia) | HCDR1 | GFTFSSY |
| SEQ ID NO: 351 (Chothia) | HCDR2 | NGLGYT |
| SEQ ID NO: 352 (Chothia) | HCDR3 | WGDEGFDI |
| SEQ ID NO: 353 (Chothia) | LCDR1 | SQGISNW |
| SEQ ID NO: 354 (Chothia) | LCDR2 | GAS |
| SEQ ID NO: 355 (Chothia) | LCDR3 | YSSFPT |
| SEQ ID NO: 356 | VL | DIQMTQSPSSLSASVGDRVTITCRASQGISNWLAWYQQKP GKAPKLLIYGASSLQSGVPSRFSGSGSGTDFTLTISSLQP EDFAVYYCQQYSSFPTTFGQGTKVEIK |
| SEQ ID NO: 357 | VH | QVQLVESGGGLVQPGGSLRLSCAASGFTFSSYAMSWVRQA PGKGLEWVSVINGLGYTTFYADSVKGRFTISRDNSKNTLY LQMNSLRAEDTAVYYCARWGDEGFDIWGQGTLVTVSS |
| SEQ ID NO: 358 | DNA VL | GATATCCAGATGACCCAGAGCCCGTCTAGCCTGAGCGCGA GCGTGGGTGATCGTGTGACCATTACCTGCAGAGCGAGCCA GGGTATTTCTAATTGGCTGGCTTGGTACCAGCAGAAACCA GGTAAAGCACCGAAACTATTAATTTATGGTGCTTCTTCTT TGCAAAGCGGGGTCCCGTCCCGTTTTAGCGGCTCTGGATC CGGCACTGATTTTACCCTGACCATTAGCAGCCTGCAACCT GAAGACTTTGCGGTTTATTATTGCCAGCAGTATTCTTCTT TTCCTACTACCTTTGGCCAGGGTACGAAAGTTGAAATTAA A |
| SEQ ID NO: 359 | DNA VH | CAGGTGCAATTGGTGGAAAGCGGCGGCGGCCTGGTGCAAC CGGGCGGCAGCCTGCGTCTGAGCTGCGCGGCCTCCGGATT TACCTTTAGCAGCTATGCGATGAGCTGGGTGCGCCAAGCC CCTGGGAAGGGTCTCGAGTGGGTGAGCGTTATTAATGGTC TTGGTTATACTACTTTTTATGCTGATTCTGTTAAGGGTCG TTTTACCATTTCACGTGATAATTCGAAAAACACCCTGTAT CTGCAAATGAACAGCCTGCGTGCGGAAGATACGGCCGTGT ATTATTGCGCGCGTTGGGGTGATGAGGGTTTTGATATTTG GGGCCAAGGCACCCTGGTGACGGTTAGCTCA |
| SEQ ID NO: 360 | Light Kappa | DIQMTQSPSSLSASVGDRVTITCRASQGISNWLAWYQQKP GKAPKLLIYGASSLQSGVPSRFSGSGSGTDFTLTISSLQP EDFAVYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 361 | Heavy IgG1 | QVQLVESGGGLVQPGGSLRLSCAASGFTFSSYAMSWVRQA PGKGLEWVSVINGLGYTTFYADSVKGRFTISRDNSKNTLY LQMNSLRAEDTAVYYCARWGDEGFDIWGQGTLVTVSSAST KGPSVFPLAPSSKSTSGGTAALGCLVKDYFPEPVTVSWNS GALTSGVHTFPAVLQSSGLYSLSSVVTVPSSSLGTQTYIC NVNHKPSNTKVDKRVEPKSCDKTHTCPPCPAPELLGGPSV FLFPPKPKDTLMISRTPEVTCVVVDVSHEDPEVKFNWYVD GVEVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQKS LSLSPGK |

| **MOR12610** | | |
|---|---|---|
| SEQ ID NO: 362 (Kabat) | HCDR1 | SYAMS |
| SEQ ID NO: 363 (Kabat) | HCDR2 | GTGPYGGTYYPDSVKG |
| SEQ ID NO: 364 (Kabat) | HCDR3 | WGDEGFDI |
| SEQ ID NO: 365 (Kabat) | LCDR1 | RASQGISNWLA |
| SEQ ID NO: 366 (Kabat) | LCDR2 | GASSLQS |
| SEQ ID NO: 367 (Kabat) | LCDR3 | QQYSSFPTT |
| SEQ ID NO: 368 (Chothia) | HCDR1 | GFTFSSY |
| SEQ ID NO: 369 (Chothia) | HCDR2 | GPYGG |
| SEQ ID NO: 370 (Chothia) | HCDR3 | WGDEGFDI |
| SEQ ID NO: 371 (Chothia) | LCDR1 | SQGISNW |
| SEQ ID NO: 372 (Chothia) | LCDR2 | GAS |
| SEQ ID NO: 373 (Chothia) | LCDR3 | YSSFPT |
| SEQ ID NO: 374 | VL | DIQMTQSPSSLSASVGDRVTITCRASQGISNWLAWYQQKP GKAPKLLIYGASSLQSGVPSRFSGSGSGTDFTLTISSLQP EDFAVYYCQQYSSFPTTFGQGTKVEIK |
| SEQ ID NO: 375 | VH | QVQLVESGGGLVQPGGSLRLSCAASGFTFSSYAMSWVRQA PGKGLEWVSGTGPYGGTYYPDSVKGRFTISRDNSKNTLYL QMNSLRAEDTAVYYCARWGDEGFDIWGQGTLVTVSS |
| SEQ ID NO: 376 | DNA VL | GATATCCAGATGACCCAGAGCCCGTCTAGCCTGAGCGCGA GCGTGGGTGATCGTGTGACCATTACCTGCAGAGCGAGCCA GGGTATTTCTAATTGGCTGGCTTGGTACCAGCAGAAACCA GGTAAAGCACCGAAACTATTAATTTATGGTGCTTCTTCTT TGCAAAGCGGGGTCCCGTCCCGTTTTAGCGGCTCTGGATC CGGCACTGATTTTACCCTGACCATTAGCAGCCTGCAACCT GAAGACTTTGCGGTTTATTATTGCCAGCAGTATTCTTCTT TTCCTACTACCTTTGGCCAGGGTACGAAAGTTGAAATTAA A |
| SEQ ID NO: 377 | DNA VH | CAGGTGCAATTGGTGGAAAGCGGCGGCGGCCTGGTGCAAC CGGGCGGCAGCCTGCGTCTGAGCTGCGCGGCCTCCGGATT TACCTTTAGCAGCTATGCGATGAGCTGGGTGCGCCAAGCC CCTGGGAAGGGTCTCGAGTGGGTGAGCGGTACTGGTCCTT ATGGTGGTACTTATTATCCTGATTCTGTTAAGGGTCGTTT TACCATTTCACGTGATAATTCGAAAAACACCCTGTATCTG CAAATGAACAGCCTGCGTGCGGAAGATACGGCCGTGTATT ATTGCGCGCGTTGGGGTGATGAGGGTTTTGATATTTGGGG CCAAGGCACCCTGGTGACGGTTAGCTCA |
| SEQ ID NO: 378 | Light Kappa | DIQMTQSPSSLSASVGDRVTITCRASQGISNWLAWYQQKP GKAPKLLIYGASSLQSGVPSRFSGSGSGTDFTLTISSLQP EDFAVYYCQQYSSFPTTFGQGTKVEIKRTVAAPSVFIFPP SDEQLKSGTASVVCLLNNFYPREAKVQWKVDNALQSGNSQ ESVTEQDSKDSTYSLSSTLTLSKADYEKHKVYACEVTHQG LSSPVTKSFNRGEC |
| SEQ ID NO: 379 | Heavy IgG1 | QVQLVESGGGLVQPGGSLRLSCAASGFTFSSYAMSWVRQA PGKGLEWVSGTGPYGGTYYPDSVKGRFTISRDNSKNTLYL |
| | | QMNSLRAEDTAVYYCARWGDEGFDIWGQGTLVTVSSASTK GPSVFPLAPSSKSTSGGTAALGCLVKDYFPEPVTVSWNSG ALTSGVHTFPAVLQSSGLYSLSSVVTVPSSSLGTQTYICN VNHKPSNTKVDKRVEPKSCDKTHTCPPCPAPELLGGPSVF LFPPKPKDTLMISRTPEVTCVVVDVSHEDPEVKFNWYVDG VEVHNAKTKPREEQYNSTYRVVSVLTVLHQDWLNGKEYKC KVSNKALPAPIEKTISKAKGQPREPQVYTLPPSREEMTKN QVSLTCLVKGFYPSDIAVEWESNGQPENNYKTTPPVLDSD GSFFLYSKLTVDKSRWQQGNVFSCSVMHEALHNHYTQKSL SLSPGK |

The present disclosure provides antibodies that specifically bind a HER3 protein (*e.g*., human and/or cynomologus HER3), said antibodies comprising a VH domain having an amino acid sequence of SEQ ID NO: 15, 33, 51, 69, 87, 105, 123, 141, 159, 177, 195, 213, 231, 249, 267, 285, 303, 321, 339, 357, and 375. The present disclosure provides antibodies that specifically bind a HER3 protein (*e.g*., human and/or cynomologus HER3), said antibodies comprising a VL domain having an amino acid sequence of SEQ ID NO: 14, 32, 50, 68, 86, 104, 122, 140, 158, 176, 194, 212, 230, 248, 266, 284, 302, 320, 338, 356, and 374. The present disclosure also provides antibodies that specifically bind to a HER3 protein (*e.g*., human and/or cynomologus HER3), said antibodies comprising a VH CDR having an amino acid sequence of any one of the VH CDRs listed in Table 1, *infra.* In particular, the disclosure provides antibodies that specifically bind to a HER3 protein (*e.g*., human and/or cynomologus HER3), said antibodies comprising (or alternatively, consisting of) one, two, three, four, five or more VH CDRs having an amino acid sequence of any of the VH CDRs listed in Table 1, *infra.*

Other antibodies of the disclosure include amino acids that have been mutated, yet have at least 60, 70, 80, 90, 95, or 98 percent identity in the CDR regions with the CDR regions depicted in the sequences described in Table 1. In some embodiments, it includes mutant amino acid sequences wherein no more than 1, 2, 3, 4 or 5 amino acids have been mutated in the CDR regions when compared with the CDR regions depicted in the sequence described Table 1, while still maintaining their specificity for the original antibody's epitope

Other antibodies of the disclosure include amino acids that have been mutated, yet have at least 60, 70, 80, 90, 95, or 98 percent identity in the framework regions with the framework regions depicted in the sequences described in Table 1. In some embodiments, it includes mutant amino acid sequences wherein no more than 1, 2, 3, 4, 5, 6, or 7 amino acids have been mutated in the framework regions when compared with the framework regions depicted in the sequence described Table 1, while still maintaining their specificity for the original antibody's epitope. The present disclosure also provides nucleic acid sequences that encode VH, VL, the full length heavy chain, and the full length light chain of the antibodies that specifically bind to a HER3 protein (*e.g*., human and/or cynomologus HER3).

The HER3 antibodies for use according to the invention can bind to the conformational epitope of HER3 comprising amino acid residues from domain 2 and domain 4 of HER3.

In another aspect, the present disclosure provides HER3-binding antibodies that comprise the heavy chain and light chain CDR1s, CDR2s and CDR3s as described in Table 1, or combinations thereof. The amino acid sequences of the VH CDR1s of the antibodies are shown in SEQ ID NOs: 2, 8, 20, 26, 38, 44, 56, 62, 74, 80, 92, 98, 110, 116, 128, 134, 146, 152, 164, 170, 182, 188, 200, 206, 218, 224, 236, 242, 254, 260, 272, 278, 290, 296, 308, 314, 326, 332, 344, 350, 362, and 368. The amino acid sequences of the VH CDR2s of the antibodies and are shown in SEQ ID NOs: 3, 9, 21, 27, 39, 45, 57, 63, 75, 81, 93, 99, 111, 117, 129, 135, 147, 153, 165, 171, 183, 189, 201, 207, 219, 225, 237, 243, 255, 261, 273, 279, 291, 297, 309, 315, 327, 333, 345, 351, 363, and 369. The amino acid sequences of the VH CDR3s of the antibodies are shown in SEQ ID NOs: 4, 10, 22, 28, 40, 46, 58, 64, 76, 82, 94, 100, 112, 118, 130, 136, 148, 154, 166, 172, 184, 190, 202, 208, 220, 226, 238, 244, 256, 262, 274, 280, 292, 298, 310, 316, 328, 334, 346, 352, 364, and 370. The amino acid sequences of the VL CDR1s of the antibodies are shown in SEQ ID NOs: 5, 11, 23, 29, 41, 47, 59, 65, 77, 83, 95, 101, 113, 119, 131, 137, 149, 155, 167, 173, 185, 191, 203, 209, 221, 227, 239, 245, 257, 263, 275, 281, 293, 299, 311, 317, 329, 335, 347, 353, 365, and 371. The amino acid sequences of the VL CDR2s of the antibodies are shown in SEQ ID NOs: 6, 12, 24, 30, 42, 48, 60, 66, 78, 84, 96, 102, 114, 120, 132, 138, 150, 156, 168, 174, 186, 192, 204, 210, 222, 228, 240, 246, 258, 264, 276, 282, 294, 300, 312, 318, 330, 336, 348, 354, 366, and 372. The amino acid sequences of the VL CDR3s of the antibodies are shown in SEQ ID NOs: 7, 13, 25, 31, 43, 49, 61, 67, 79, 85, 97, 103, 115, 121, 133, 139, 151, 157, 169, 175, 187, 193, 205, 211, 223, 229, 241, 247, 259, 265, 277, 283, 295, 301, 313, 319, 331, 337, 349, 355, 367, and 373. The CDR regions are delineated using the Kabat system (Kabat et al., (1991) Sequences of Proteins of Immunological Interest, Fifth Edition, U.S. Department of Health and Human Services, NIH Publication No. 91-3242; Chothia et al., (1987) J. Mol. Biol. 196:901-917; Chothia et al., (1989) Nature 342: 877-883; and Al-Lazikani et al., (1997) J. Mol. Biol. 273, 927-948).

In a specific embodiment, an antibody for use according to the invention that binds to HER3 comprises a heavy chain variable region CDR1 of SEQ ID NO: 2; a CDR2 of SEQ ID NO: 3; a CDR3 of SEQ ID NO: 4; a light chain variable region CDR1 of SEQ ID NO: 5; a CDR2 of SEQ ID NO: 6; and a CDR3 of SEQ ID NO: 7.

In a specific embodiment, an antibody for use according to the invention that binds to HER3 comprises a heavy chain variable region CDR1 of SEQ ID NO: 20; a CDR2 of SEQ ID NO: 21; a CDR3 of SEQ ID NO: 22; a light chain variable region CDR1 of SEQ ID NO: 23; a CDR2 of SEQ ID NO: 24; and a CDR3 of SEQ ID NO: 25.

In a specific embodiment, an antibody for use according to the invention that binds to HER3 comprises a heavy chain variable region CDR1 of SEQ ID NO: 38; a CDR2 of SEQ ID NO: 39; a CDR3 of SEQ ID NO: 40; a light chain variable region CDR1 of SEQ ID NO: 41; a CDR2 of SEQ ID NO: 42; and a CDR3 of SEQ ID NO: 43.

In a specific embodiment, an antibody for use according to the invention that binds to HER3 comprises a heavy chain variable region CDR1 of SEQ ID NO: 56; a CDR2 of SEQ ID NO: 57; a CDR3 of SEQ ID NO: 58; a light chain variable region CDR1 of SEQ ID NO: 59; a CDR2 of SEQ ID NO: 60; and a CDR3 of SEQ ID NO: 61.

In a specific embodiment, an antibody for use according to the invention that binds to HER3 comprises a heavy chain variable region CDR1 of SEQ ID NO: 74; a CDR2 of SEQ ID NO: 75; a CDR3 of SEQ ID NO: 76; a light chain variable region CDR1 of SEQ ID NO: 77; a CDR2 of SEQ ID NO: 78; and a CDR3 of SEQ ID NO: 79.

In a specific embodiment, an antibody for use according to the invention that binds to HER3 comprises a heavy chain variable region CDR1 of SEQ ID NO: 92; a CDR2 of SEQ ID NO: 93; a CDR3 of SEQ ID NO: 94; a light chain variable region CDR1 of SEQ ID NO: 95; a CDR2 of SEQ ID NO: 96; and a CDR3 of SEQ ID NO: 97.

In a specific embodiment, an antibody for use according to the invention that binds to HER3 comprises a heavy chain variable region CDR1 of SEQ ID NO: 110; a CDR2 of SEQ ID NO: 111; a CDR3 of SEQ ID NO: 112; a light chain variable region CDR1 of SEQ ID NO: 113; a CDR2 of SEQ ID NO: 114; and a CDR3 of SEQ ID NO: 115.

In a specific embodiment, an antibody for use according to the invention that binds to HER3 comprises a heavy chain variable region CDR1 of SEQ ID NO: 128; a CDR2 of SEQ ID NO: 129; a CDR3 of SEQ ID NO: 130; a light chain variable region CDR1 of SEQ ID NO: 131; a CDR2 of SEQ ID NO: 132; and a CDR3 of SEQ ID NO: 133.

In a specific embodiment, an antibody for use according to the invention that binds to HER3 comprises a heavy chain variable region CDR1 of SEQ ID NO: 146; a CDR2 of SEQ ID NO: 147; a CDR3 of SEQ ID NO: 148; a light chain variable region CDR1 of SEQ ID NO: 149; a CDR2 of SEQ ID NO: 150; and a CDR3 of SEQ ID NO: 151.

In a specific embodiment, an antibody for use according to the invention that binds to HER3 comprises a heavy chain variable region CDR1 of SEQ ID NO: 164; a CDR2 of SEQ ID NO: 165; a CDR3 of SEQ ID NO: 166; a light chain variable region CDR1 of SEQ ID NO: 167; a CDR2 of SEQ ID NO: 168; and a CDR3 of SEQ ID NO: 169.

In a specific embodiment, an antibody for use according to the invention that binds to HER3 comprises a heavy chain variable region CDR1 of SEQ ID NO: 182; a CDR2 of SEQ ID NO: 183; a CDR3 of SEQ ID NO: 184; a light chain variable region CDR1 of SEQ ID NO: 185; a CDR2 of SEQ ID NO: 186; and a CDR3 of SEQ ID NO: 187.

In a specific embodiment, an antibody for use according to the invention that binds to HER3 comprises a heavy chain variable region CDR1 of SEQ ID NO: 200; a CDR2 of SEQ ID NO: 201; a CDR3 of SEQ ID NO: 202; a light chain variable region CDR1 of SEQ ID NO: 203; a CDR2 of SEQ ID NO: 204; and a CDR3 of SEQ ID NO: 205.

In a specific embodiment, an antibody for use according to the invention that binds to HER3 comprises a heavy chain variable region CDR1 of SEQ ID NO: 218; a CDR2 of SEQ ID NO: 219; a CDR3 of SEQ ID NO: 220; a light chain variable region CDR1 of SEQ ID NO: 221; a CDR2 of SEQ ID NO: 222; and a CDR3 of SEQ ID NO: 223.

In a specific embodiment, an antibody for use according to the invention that binds to HER3 comprises a heavy chain variable region CDR1 of SEQ ID NO: 236; a CDR2 of SEQ ID NO: 237; a CDR3 of SEQ ID NO: 238; a light chain variable region CDR1 of SEQ ID NO: 239; a CDR2 of SEQ ID NO: 240; and a CDR3 of SEQ ID NO: 241.

In a specific embodiment, an antibody for use according to the invention that binds to HER3 comprises a heavy chain variable region CDR1 of SEQ ID NO: 254; a CDR2 of SEQ ID NO: 255; a CDR3 of SEQ ID NO: 256; a light chain variable region CDR1 of SEQ ID NO: 257; a CDR2 of SEQ ID NO: 258; and a CDR3 of SEQ ID NO: 259.

In a specific embodiment, an antibody for use according to the invention that binds to HER3 comprises a heavy chain variable region CDR1 of SEQ ID NO: 272; a CDR2 of SEQ ID NO: 273; a CDR3 of SEQ ID NO: 274; a light chain variable region CDR1 of SEQ ID NO: 275; a CDR2 of SEQ ID NO: 276; and a CDR3 of SEQ ID NO: 277.

In a specific embodiment, an antibody for use according to the invention that binds to HER3 comprises a heavy chain variable region CDR1 of SEQ ID NO: 290; a CDR2 of SEQ ID NO: 291; a CDR3 of SEQ ID NO: 292; a light chain variable region CDR1 of SEQ ID NO: 293; a CDR2 of SEQ ID NO: 294; and a CDR3 of SEQ ID NO: 295.

In a specific embodiment, an antibody for use according to the invention that binds to HER3 comprises a heavy chain variable region CDR1 of SEQ ID NO: 308; a CDR2 of SEQ ID NO: 309; a CDR3 of SEQ ID NO: 310; a light chain variable region CDR1 of SEQ ID NO: 311; a CDR2 of SEQ ID NO: 312; and a CDR3 of SEQ ID NO: 313.

In a specific embodiment, an antibody for use according to the invention that binds to HER3 comprises a heavy chain variable region CDR1 of SEQ ID NO: 326; a CDR2 of SEQ ID NO: 327; a CDR3 of SEQ ID NO: 328; a light chain variable region CDR1 of SEQ ID NO: 329; a CDR2 of SEQ ID NO: 330; and a CDR3 of SEQ ID NO: 331.

In a specific embodiment, an antibody for use according to the invention that binds to HER3 comprises a heavy chain variable region CDR1 of SEQ ID NO: 344; a CDR2 of SEQ ID NO: 345; a CDR3 of SEQ ID NO: 346; a light chain variable region CDR1 of SEQ ID NO: 347; a CDR2 of SEQ ID NO: 348; and a CDR3 of SEQ ID NO: 349.

In a specific embodiment, an antibody for use according to the invention that binds to HER3 comprises a heavy chain variable region CDR1 of SEQ ID NO: 362; a CDR2 of SEQ ID NO: 363; a CDR3 of SEQ ID NO: 364; a light chain variable region CDR1 of SEQ ID NO: 365; a CDR2 of SEQ ID NO: 366; and a CDR3 of SEQ ID NO: 367.

In a specific embodiment, an antibody for use according to the invention that binds to HER3 comprises a VH of SEQ ID NO. 15 and VL of SEQ ID NO: 14. In a specific embodiment, an antibody for use according to the invention that binds to HER3 comprises a VH of SEQ ID NO: 33 and VL of SEQ ID NO: 32. In a specific embodiment, an antibody for use according to the invention that binds to HER3 comprises a VH of SEQ ID NO: 51 and VL of SEQ ID NO: 50. In a specific embodiment, an antibody for use according to the invention that binds to HER3 comprises a SEQ ID NO: 69 and VL of SEQ ID NO: 68. In a specific embodiment, an antibody for use according to the invention that binds to HER3 comprises a VH of SEQ ID NO: 87 and VL of SEQ ID NO: 86. In a specific embodiment, an antibody for use according to the invention that binds to HER3 comprises a VH of SEQ ID NO: 105 and VL of SEQ ID NO: 104. In a specific embodiment, an antibody for use according to the invention that binds to HER3 comprises a VH of SEQ ID NO: 123 and VL of SEQ ID NO: 122. In a specific embodiment, an antibody for use according to the invention that binds to HER3 comprises a VH of SEQ ID NO: 141 and VL of SEQ ID NO: 140. In a specific embodiment, an antibody for use according to the invention that binds to HER3 comprises a VH of SEQ ID NO: 159 and VL of SEQ ID NO: 158. In a specific embodiment, an antibody for use according to the invention that binds to HER3 comprises a VH of SEQ ID NO: 177 and VL of SEQ ID NO: 176. In a specific embodiment, an antibody for use according to the invention that binds to HER3 comprises a VH of SEQ ID NO: 195 and VL of SEQ ID NO: 194. In a specific embodiment, an antibody for use according to the invention that binds to HER3 comprises a VH of SEQ ID NO: 213 and VL of SEQ ID NO: 212. In a specific embodiment, an antibody for use according to the invention that binds to HER3 comprises a VH of SEQ ID NO: 231 and VL of SEQ ID NO: 230. In a specific embodiment, an antibody for use according to the invention that binds to HER3 comprises a VH of SEQ ID NO: 249 and VL of SEQ ID NO: 248. In a specific embodiment, an antibody for use according to the invention that binds to HER3 comprises a VH of SEQ ID NO: 267 and VL of SEQ ID NO: 266. In a specific embodiment, an antibody for use according to the invention that binds to HER3 comprises a VH of SEQ ID NO: 285 and VL of SEQ ID NO: 284. In a specific embodiment, an antibody for use according to the invention that binds to HER3 comprises a VH of SEQ ID NO: 303 and VL of SEQ ID NO: 302. In a specific embodiment, an antibody for use according to the invention that binds to HER3 comprises a VH of SEQ ID NO: 321 and VL of SEQ ID NO: 320. In a specific embodiment, an antibody for use according to the invention that binds to HER3 comprises a VH of SEQ ID NO: 339 and VL of SEQ ID NO: 338. In a specific embodiment, an antibody for use according to the invention that binds to HER3 comprises a VH of SEQ ID NO: 357 and VL of SEQ ID NO: 356. In a specific embodiment, an antibody for use according to the invention that binds to HER3 comprises a VH of SEQ ID NO: 375 and VL of SEQ ID NO: 374.

The antibodies disclosed herein can be derivatives of single chain antibodies, diabodies, domain antibodies, nanobodies, and unibodies. In yet another embodiment, the present disclosure provides an antibody or fragment thereof comprising amino acid sequences that are homologous to the sequences described in Table 1, and said antibody binds to a HER3 protein (*e.g*., human and/or cynomologus HER3), and retains the desired functional properties of those antibodies described in Table 1.

For example, the disclosure provides an isolated monoclonal antibody (or a functional fragment thereof) comprising a heavy chain variable region and a light chain variable region, wherein the heavy chain variable region comprises an amino acid sequence that is at least 80%, at least 90%, or at least 95% identical to an amino acid sequence selected from the group consisting of SEQ ID NOs: 15, 33, 51, 69, 87, 105, 123, 141, 159, 177, 195, 213, 231, 249, 267, 285, 303, 321, 339, 357, and 375; the light chain variable region comprises an amino acid sequence that is at least 80%, at least 90%, or at least 95% identical to an amino acid sequence selected from the group consisting of SEQ ID NOs: 14, 32, 50, 68, 86, 104, 122, 140, 158, 176, 194, 212, 230, 248, 266, 284, 302, 320, 338, 356, and 374; the antibody binds to HER3 (*e.g.*, human and/or cynomologus HER3) and neutralizes the signaling activity of HER3, which can be measured in a phosphorylation assay or other measure of HER signaling (e.g., phospo-HER3 assays, phospho-Akt assays, cell proliferation, and ligand blocking assays as described in WO2012022814). Also disclosed are variable heavy and light chain parental nucleotide sequences; and full length heavy and light chain sequences optimized for expression in a mammalian cell. Other antibodies of the disclosure include amino acids or nucleic acids that have been mutated, yet have at least 60, 70, 80, 90, 95, or 98% percent identity to the sequences described above. In some embodiments, it include mutant amino acid sequences wherein no more than 1, 2, 3, 4 or 5 amino acids have been mutated by amino acid deletion, insertion or substitution in the variable regions when compared with the variable regions depicted in the sequence described above.

In other embodiments, the VH and/or VL amino acid sequences may be 50%, 60%, 70%, 80%, 90%, 95%, 96%, 97%, 98% or 99% identical to the sequences set forth in Table 1. In other embodiments, the VH and/or VL amino acid sequences may be identical except an amino acid substitution in no more than 1,2,3,4 or 5 amino acid position. An antibody having VH and VL regions having high (*i. e.*, 80% or greater) identity to the VH and VL regions of the antibodies described in Table 1 can be obtained by mutagenesis (*e.g.*, site-directed or PCR-mediated mutagenesis), followed by testing of the encoded altered antibody for retained function using the functional assays described herein.

In other embodiments, the variable regions of heavy chain and/or light chain nucleotide sequences may be 60%, 70%, 80%, 90%, 95%, 96%, 97%, 98% or 99% identical to the sequences set forth above.

In certain embodiments, an antibody of the disclosure has a heavy chain variable region comprising CDR1, CDR2, and CDR3 sequences and a light chain variable region comprising CDR1, CDR2, and CDR3 sequences, wherein one or more of these CDR sequences have specified amino acid sequences based on the antibodies described herein or conservative modifications thereof, and wherein the antibodies retain the desired functional properties of the HER3-binding antibodies for use according to the invention.

Accordingly, the disclosure provides an isolated HER3 monoclonal antibody, or a fragment thereof, consisting of a heavy chain variable region comprising CDR1, CDR2, and CDR3 sequences and a light chain variable region comprising CDR1, CDR2, and CDR3 sequences, wherein: the heavy chain variable region CDR1 amino acid sequences are selected from the group consisting of SEQ ID NOs: 2, 8, 20, 26, 38, 44, 56, 62, 74, 80, 92, 98, 110, 116, 128, 134, 146, 152, 164, 170, 182, 188, 200, 206, 218, 224, 236, 242, 254, 260, 272, 278, 290, 296, 308, 314, 326, 332, 344, 350, 362, and 368, and conservative modifications thereof; the heavy chain variable region CDR2 amino acid sequences are selected from the group consisting of SEQ ID NOs: 3, 9, 21, 27, 39, 45, 57, 63, 75, 81, 93, 99, 111, 117, 129, 135, 147, 153, 165, 171, 183, 189, 201, 207, 219, 225, 237, 243, 255, 261, 273, 279, 291, 297, 309, 315, 327, 333, 345, 351, 363, and 369 and conservative modifications thereof; the heavy chain variable region CDR3 amino acid sequences are selected from the group consisting of SEQ ID NOs: 4, 10, 22, 28, 40, 46, 58, 64, 76, 82, 94, 100, 112, 118, 130, 136, 148, 154, 166, 172, 184, 190, 202, 208, 220, 226, 238, 244, 256, 262, 274, 280, 292, 298, 310, 316, 328, 334, 346, 352, 364, and 370 and conservative modifications thereof; the light chain variable regions CDR1 amino acid sequences are selected from the group consisting of SEQ ID NOs: 5, 11, 23, 29, 41, 47, 59, 65, 77, 83, 95, 101, 113, 119, 131, 137, 149, 155, 167, 173, 185, 191, 203, 209, 221, 227, 239, 245, 257, 263, 275, 281, 293, 299, 311, 317, 329, 335, 347, 353, 365, and 371 and conservative modifications thereof; the light chain variable regions CDR2 amino acid sequences are selected from the group consisting of SEQ ID NOs: 6, 12, 24, 30, 42, 48, 60, 66, 78, 84, 96, 102, 114, 120, 132, 138, 150, 156, 168, 174, 186, 192, 204, 210, 222, 228, 240, 246, 258, 264, 276, 282, 294, 300, 312, 318, 330, 336, 348, 354, 366, and 372, and conservative modifications thereof; the light chain variable regions of CDR3 amino acid sequences are selected from the group consisting of SEQ ID NOs: 7, 13, 25, 31, 43, 49, 61, 67, 79, 85, 97, 103, 115, 121, 133, 139, 151, 157, 169, 175, 187, 193, 205, 211, 223, 229, 241, 247, 259, 265, 277, 283, 295, 301, 313, 319, 331, 337, 349, 355, 367, and 373, and conservative modifications thereof; the antibody or fragment thereof specifically binds to HER3, and neutralizes HER3 activity by inhibiting a HER signaling pathway, which can be measured in a phosphorylation assay or other measure of HER signaling (e.g., phospo-HER3 assays, phospho-Akt assays, cell proliferation, and ligand blocking assays as described in WO2012022814).

In another example, the isolated antibody or fragment thereof that cross-competes with an antibody described in Table 1. The antibodies can comprises a VH selected from the group consisting of SEQ ID NO: 15, SEQ ID NO: 33, SEQ ID NO: 51, SEQ ID NO: 69, SEQ ID NO: 87, SEQ ID NO: 105, SEQ ID NO: 123, SEQ ID NO: 141, SEQ ID NO: 159, SEQ ID NO: 177, SEQ ID NO: 195, SEQ ID NO: 213, SEQ ID NO: 231, SEQ ID NO: 249, SEQ ID NO: 267, SEQ ID NO: 285, SEQ ID NO: 303, SEQ ID NO: 321, SEQ ID NO: 339, SEQ ID NO: 357, and SEQ ID NO: 375; and a VL selected from the group consisting of SEQ ID NO: 14, SEQ ID NO: 32, SEQ ID NO: 50, SEQ ID NO: 68, SEQ ID NO: 86, SEQ ID NO: 104, SEQ ID NO: 122, SEQ ID NO: 140, SEQ ID NO: 158, SEQ ID NO: 176, SEQ ID NO: 194, SEQ ID NO: 212, SEQ ID NO: 230, SEQ ID NO: 248, SEQ ID NO: 266, SEQ ID NO: 284, SEQ ID NO: 302, SEQ ID NO: 320, SEQ ID NO: 338, SEQ ID NO: 356, and SEQ ID NO: 374 or an amino acid sequence with 97-99 percent identity thereof.

In another example, the isolated antibody or fragment thereof comprises a heavy chain variable region CDR1 selected from the group consisting of SEQ ID NO: 2, 20, 38, 56, 74, 92, 110, 128, 146, 164, 182, 200, 218, 236, 254, 272, 290, 308, 326, 344, and 362; CDR2 selected from the group consisting of SEQ ID NO: 3, 21, 39, 57, 75, 93, 111, 129, 147, 165, 183, 201, 219, 237, 255, 273, 291, 309, 327, 345, and 363; CDR3 selected from the group consisting of SEQ ID NO: 4, 22, 40, 58, 76, 94, 112, 130, 148, 166, 184, 202, 220, 238, 256, 274, 292, 310, 328, 346, and 364; a light chain variable region CDR1 selected from the group consisting of SEQ ID NO: 5, 23, 41, 59, 77, 95, 113, 131, 149, 167, 185, 203, 221, 239, 257, 275, 293, 311, 329, 347, and 365; CDR2 selected from the group consisting of SEQ ID NO: 6, 24, 42, 60, 78, 96, 114, 132, 150, 166, 186, 204, 222, 240, 258, 276, 294, 312, 330, 348, and 366; and CDR3 selected from the group consisting of SEQ ID NO: 7, 25, 43, 61, 79, 97, 115, 133, 151, 169, 187, 205, 223, 241, 259, 277, 295, 313, 331, 349, and 367.

In a specific example, the isolated antibody or fragment thereof for use according to the invention, comprises a heavy chain variable region CDR1 of SEQ ID NO: 128; CDR2 of SEQ ID NO: 129; CDR3 of SEQ ID NO: 130; a light chain variable region CDR1 of SEQ ID NO: 131; CDR2 of SEQ ID NO: 132; and CDR3 of SEQ ID NO: 133.

The antibodies used in the invention can be fragment of an antibody that binds to HER3 selected from the group consisting of; Fab, F(ab₂)', F(ab)₂', scFv.

The present disclosure also includes antibodies that interacts with (e.g., by binding, steric hindrance, stabilizing/destabilizing, spatial distribution) the same epitope as do the HER3-binding antibodies described in Table 1.

The present disclosure provides fully human antibodies that specifically bind to a HER3 protein (e.g., human and/or cynomologus/ mouse/rat HER3). Compared to the chimeric or humanized antibodies, the human HER3 -binding antibodies for use according to the invention have further reduced antigenicity when administered to human subjects.

The human HER3-binding antibodies can be generated using methods that are known in the art. For example, the humaneering technology used to converting non-human antibodies into engineered human antibodies. U.S. Patent Publication No. 20050008625 describes an *in vivo* method for replacing a nonhuman antibody variable region with a human variable region in an antibody while maintaining the same or providing better binding characteristics compared to that of the nonhuman antibody.

In another aspect, the present invention features biparatopic, bispecific or multispecific molecules comprising a HER3-binding antibody, or a fragment thereof, for use according to the invention. An antibody for use according to the invention, or fragments thereof, can be derivatized or linked to another functional molecule, *e.g.*, another peptide or protein (*e.g.*, another antibody or ligand for a receptor) to generate a bispecific molecule that binds to at least two different binding sites or target molecules. The antibody for use according to the invention may in fact be derivatized or linked to more than one other functional molecule to generate biparatopic or multi-specific molecules that bind to more than two different binding sites and/or target molecules; such biparatopic or multi-specific molecules. To create a bispecific molecule for use according to the invention, an antibody for use according to the invention can be functionally linked (*e.g.*, by chemical coupling, genetic fusion, non-covalent association or otherwise) to one or more other binding molecules, such as another antibody, antibody fragment, peptide or binding mimetic, such that a bispecific molecule results.

In some embodiments, the antibodies or fragments thereof that can be used for treatment of low-grade serous ovarian cancer may be any of the antibodies and fragments thereof that are described in WO 2012/022814; WO 2013/084147; WO 2013/084148; and WO 2013/084151.

### Antibody Combinations

In another aspect, the invention pertains to HER3 antibodies, or fragments thereof for use according to the invention used with other therapeutic agents such as another antibodies, small molecule inhibitors, mTOR inhibitors or PI3Kinase inhibitors. In yet other aspects, HER2 and/or EGFR antibodies may be used in combination with HER3 antibodies or other therapeutic agents. Examples include, but are not limited to, the following:

HER1 inhibitors: The HER3 antibodies or fragments thereof can be used with HER1 inhibitors which include, but are not limited to, Matuzumab (EMD72000),

Erbitux®/Cetuximab (Imclone), Vectibix® /Panitumumab (Amgen), mAb 806, and Nimotuzumab (TheraCIM), Iressa®/Gefitinib (Astrazeneca); CI-1033 (PD183805) (Pfizer), Lapatinib (GW-572016) (Glaxo SmithKline), Tykerb® /Lapatinib Ditosylate (SmithKlineBeecham), Tarceva® / Erlotinib HCL (OSI-774) (OSI Pharma), and PKI-166 (Novartis), and N-[4-[(3-Chloro-4-fluorophenyl)amino]-7-[[(3"S")-tetrahydro-3-furanyl]oxy]- 6-quinazolinyl]-4(dimethylamino)-2-butenamide, sold under the tradename Tovok® by Boehringer Ingelheim).

HER2 inhibitors: The HER3 antibodies or fragments thereof can be used with HER2 inhibitors which include, but are not limited to, Pertuzumab (sold under the trademark Omnitarg®, by Genentech), Trastuzumab (sold under the trademark Herceptin® by Genentech/Pvoche), MM-111, neratinib (also known as HKI-272, (2E)-N-[4-[[3-chloro-4- [(pyridin-2-yl)methoxy]phenyl] amino] -3 -cyano-7-ethoxyquinolin-6-yl] -4-(dimethylamino)but-2-enamide, and described PCT Publication No. WO 05/028443), lapatinib or lapatinib ditosylate (sold under the trademark Tykerb® by Glaxo SmithKline.

HER3 inhibitors: The HER3 antibodies or fragments thereof can be used with HER3 inhibitors which include, but are not limited to, MM- 121, MM-111, IB4C3, 2DID12 (U3 Pharma AG), AMG888 (Amgen), AV-203(Aveo), MEHD7945A (Genentech), and small molecules that inhibit HER3.

HER4 inhibitors: The HER3 antibodies or fragments thereof can be used with HER4 inhibitors.

PI3K inhibitors: The HER3 antibodies or fragments thereof can be used with PI3 kinase inhibitors which include, but are not limited to, 4-[2-(IH-lndazol-4-yl)-6-[[4-(methylsulfonyl)piperazin- 1 -yl]methyl]thieno [3 ,2-d]pyrimidin-4-yl]morpholine (also known as GDC 0941 and described in PCT Publication Nos. WO 09/036082 and WO 09/055730), 2- Methyl-2-[4-[3-methyl-2-oxo-8-(quinolin-3-yl)-2,3-dihydroimidazo[4,5-c]quinolin-l-yl]phenyl]propionitrile (also known as BEZ 235 or NVP-BEZ 235, and described in PCT Publication No. WO 06/122806), BMK120 and BYL719. In one example, the PI3K inhibitor is (S)-Pyrrolidine-I,2-dicarboxylic acid 2-amide I-({4-methyl-5-[2-(2,2,2-trifluoro-I,I-dimethyl-ethyl)-pyridin-4-yl]-thiazol-2-yl}-amide).

mTOR inhibitors: The HER3 antibodies or fragments thereof can be used with mTOR inhibitors which include, but are not limited to, Temsirolimus (sold under the tradename Torisel® by Pfizer), ridaforolimus (formally known as deferolimus, (IR,2R,4S)-4-[(2R)-2 [(1R,9S,12S,15R,16E,18R,19R,21R, 23S,24E,26E,28Z,30S,32S,35R)-I,18-dihydroxy-19,30-dimethoxy- 15, 17,21,23, 29,35-hexamethyl-2,3,10,14,20-pentaoxo-II,36-dioxa-4-azatricyclo[30.3.1.04,9] hexatriaconta-16,24,26,28-tetraen-12-yl]propyl]-2- methoxycyclohexyl dimethylphosphinate, also known as Deforolimus, AP23573 and MK8669 (Ariad Pharm.), and described in PCT Publication No. WO 03/064383), everolimus (RADOOI) (sold under the tradename Afinitor® by Novartis), One or more therapeutic agents may be administered either simultaneously or before or after administration of a HER3 antibody or fragment thereof for use according to the present invention.

### Prophylactic and Therapeutic Uses

The present disclosure provides methods of treating a disease or disorder by administering a therapeutically effective amount (e.g., a dose of an antibody which inhibits low-grade serous ovarian cancer growth) of an antibody of fragment thereof that specifically binds to HER3. In a specific embodiment, the present disclosure provides a method of treating low-grade serous ovarian cancer. In some embodiments, a therapeutically effective amount of an antibody of fragment thereof that specifically binds to HER2 may be administered. In some embodiments, a therapeutically effective amount of an antibody of fragment thereof that specifically binds to EGFR may be administered. In yet other embodiments, an additional therapeutic agent may be administered. In some embodiments the additional therapeutic agent is selected from one or more HER1 inhibitors and/or one or more HER2 inhibitors and/or one or more HER3 inhibitors and/ one or more HER4 inhibitors, and/or one or more mTOR inhibitors and/or one or more PI3 Kinase inhibitors. In some embodiments, the addition therapeutic agent is selected from: gemcitabine, paclitaxel, imatinib mesylate, sunitinib malate, adriamycin, daunomycin, cisplatin, etoposide, vinblastine, vincristine, and methotrexate.

### Pharmaceutical Compositions

To prepare pharmaceutical or sterile compositions including HER3 - binding antibodies (intact or binding fragments), the HER3-binding antibodies (intact or binding fragments) is mixed with a pharmaceutically acceptable carrier or excipient. The compositions can additionally contain one or more other therapeutic agents that are suitable for treating or preventing low-grade serous ovarian cancer.

Formulations of therapeutic and diagnostic agents can be prepared by mixing with physiologically acceptable carriers, excipients, or stabilizers in the form of, e.g., lyophilized powders, slurries, aqueous solutions, lotions, or suspensions (see, e.g., Hardman et al., (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules are available (see, e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, N.Y.; Bach (ed.) (1993) Monoclonal Antibodies and Peptide Therapy in Autoimmune Diseases, Marcel Dekker, New York, N.Y.; Baert et al, (2003) New Engl. J. Med. 348:601-608; Milgrom et al, (1999) New Engl. J. Med. 341 : 1966-1973; Slamon et al, (2001) New Engl. J. Med. 344:783-792; Beniaminovitz et al, (2000) New Engl. J. Med. 342:613-619; Ghosh et al, (2003) New Engl. J. Med. 348:24-32; Lipsky et al, (2000) New Engl. J. Med. 343: 1594-1602).

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced.

Actual dosage levels of the active ingredients in the pharmaceutical compositions of the present disclosure may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present disclosure employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors known in the medical arts. Compositions comprising antibodies or fragments thereof for use according to the invention can be provided by continuous infusion, or by doses at intervals of, e.g., one day, one week, or 1-7 times per week. Doses may be provided intravenously, subcutaneously, topically, orally, nasally, rectally, intramuscular, intracerebrally, or by inhalation. A specific dose protocol is one involving the maximal dose or dose frequency that avoids significant undesirable side effects. A total weekly dose may be at least 0.05 µ /kg body weight, at least 0.2 µg/kg, at least 0.5 µg/kg, at least 1 µg/kg, at least 10 µg/kg, at least 100 µg/kg, at least 0.2 mg/kg, at least 1.0 mg/kg, at least 2.0 mg/kg, at least 10 mg/kg, at least 25 mg/kg, at least 30 mg/kg, at least 40 mg/kg or at least 50 mg/kg (see, e.g., Yang et al, (2003) New Engl. J. Med. 349:427-434; Herold et al, (2002) New Engl. J. Med. 346: 1692-1698; Liu et al, (1999) J. Neurol. Neurosurg. Psych. 67:451-456; Portielji et al, (2003) Cancer Immunol. Immunother. 52: 133-144). The desired dose of antibodies or fragments thereof is about the same as for an antibody or polypeptide, on a moles/kg body weight basis. The desired plasma concentration of the antibodies or fragments thereof is about, on a moles/kg body weight basis. The dose may be at least 15 µg at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, or at least 100 µg. The doses administered to a subject may number at least 1, 2, 3, 4, 5, 6, 7, 8, 9, 10, 11, or 12, or more. For antibodies or fragments thereof for use according to the invention, the dosage administered to a patient may be 0.0001 mg/kg to 100 mg/kg of the patient's body weight. The dosage may be between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight.

The dosage of the antibodies or fragments thereof for use according to the invention may be calculated using the patient's weight in kilograms (kg) multiplied by the dose to be administered in mg/kg. The dosage of the antibodies or fragments thereof for use accorging to the invention may be 150 µg/kg or less, 125 µg/kg or less, 100 µg/kg or less, 95 µg/kg or less, 90 µg/kg or less, 85 µg/kg or less, 80 µg/kg or less, 75 µg/kg or less, 70 µg/kg or less, 65 µg/kg or less, 60 µg/kg or less, 55 µg/kg or less, 50 µg/kg or less, 45 µg/kg or less, 40 µg/kg or less, 35 µg/kg or less, 30 µg/kg or less, 25 µg/kg or less, 20 µg/kg or less, 15 µg/kg or less, 10 µg/kg or less, 5 µg/kg or less, 2.5 µg/kg or less, 2 µg/kg or less, 1.5 µg/kg or less, 1 µg/kg or less, 0.5 µg/kg or less, or 0.5 µg/kg or less of a patient's body weight.

Unit dose of the antibodies or fragments thereof for use according to the invention may be 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 60 mg, , 0.25 mg to 40 mg, 0.25 mg to 20 mg, 0.25 to 15 mg, 0.25 to 12 mg, 0.25 to 10 mg, 0.25 to 8 mg, 0.25 mg to 7 mg, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

The dosage of the antibodies or fragments thereof for use according to the invention may achieve a serum titer of at least 0.1 µg/ml, at least 0.5 µg/ml, at least 1 µg/ml, at least 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in a subject. Alternatively, the dosage of the antibodies or fragments thereof for use according to the invention may achieve a serum titer of at least 0.1 µg/ml, at least 0.5 µg/ml, at least 1 µg/ml, at least, 2 µg/ml, at least 5 µg/ml, at least 6 µg/ml, at least 10 µg/ml, at least 15 µg/ml, at least 20 .mu.g/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in the subject.

Doses of antibodies or fragments thereof for use according to the invention may be repeated and the administrations may be separated by at least 1 day, 2 days, 3 days, 5 days, 7 days, 10 days, 15 days, 30 days, 45 days, 2 months, 75 days, 3 months, or at least 6 months.

An effective amount for a particular patient may vary depending on factors such as the condition being treated, the overall health of the patient, the method route and dose of administration and the severity of side effects (see, e.g., Maynard et al., (1996) A Handbook of SOPs for Good Clinical Practice, Interpharm Press, Boca Raton, Fla.; Dent (2001) Good Laboratory and Good Clinical Practice, Urch PubL, London, UK).

The route of administration may be by, e.g., topical or cutaneous application, injection or infusion by intravenous, intraperitoneal, intracerebral, intramuscular, intraocular, intraarterial, intracerebrospinal, intralesional, or by sustained release systems or an implant (see, e.g.,Sidman et al., (1983) Biopolymers 22:547-556; Langer et al., (1981) J. Biomed. Mater. Res. 15: 167-277; Langer (1982) Chem. Tech. 12:98-105; Epstein et al, (1985) Proc. Natl. Acad. Sci. USA 82:3688-3692; Hwang et al., (1980) Proc. Natl. Acad. Sci. USA 77:4030-4034; U.S. Pat. Nos. 6,350,466 and 6,316,024). Where necessary, the composition may also include a solubilizing agent and a local anesthetic such as lidocaine to ease pain at the site of the injection. In addition, pulmonary administration can also be employed, e.g., by use of an inhaler or nebulizer, and formulation with an aerosolizing agent. See, e.g., U.S. Pat. Nos. 6,019,968, 5,985,320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903.

A composition of the present disclosure may also be administered via one or more routes of administration using one or more of a variety of methods known in the art. As will be appreciated by the skilled artisan, the route and/or mode of administration will vary depending upon the desired results. Selected routes of administration for antibodies or fragments thereof for use according to the invention include intravenous, intramuscular, intradermal, intraperitoneal, subcutaneous, spinal or other parenteral routes of administration, for example by injection or infusion. Parenteral administration may represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the disclosure can be administered via a non-parenteral route, such as a topical, epidermal or mucosal route of administration, for example, intranasally, orally, vaginally, rectally, sublingually or topically. In one embodiment, the antibodies or fragments thereof for use according to the invention is administered by infusion. In another embodiment, the multispecific epitope binding protein for use according to the invention is administered subcutaneously. If the antibodies or fragments thereof for use according to the invention are administered in a controlled release or sustained release system, a pump may be used to achieve controlled or sustained release (see Langer, supra; Sefton, (1987) CRC Crit. Ref Biomed. Eng. 14:20; Buchwald et al., (1980), Surgery 88:507; Saudek et al, (1989) N. Engl. J. Med. 321 :574). Polymeric materials can be used to achieve controlled or sustained release of the therapies of the disclosure (see e.g., Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, (1983) J. Macromol. Sci. Rev. Macromol. Chem. 23:61; see also Levy et al., (1985) Science 228: 190; During et al, (1989) Ann. Neurol. 25:351; Howard et al, (1989) J. Neurosurg. 7 1:105); U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, e.g., Goodson, in Medical Applications of Controlled Release, supra, vol. 2, pp. 115-138 (1984)).

Controlled release systems are discussed in the review by Langer, (1990), Science 249: 1527- 1533). Any technique known to one of skill in the art can be used to produce sustained release formulations comprising one or more antibodies or fragments thereof for use according to the invention. See, e.g., U.S. Pat. No. 4,526,938, PCT publication WO 91/05548, PCT publication WO 96/20698, Ning et al, (1996), Radiotherapy & Oncology 39: 179-189, Song et al, (1995) PDA Journal of Pharmaceutical Science & Technology 50:372-397, Cleek et al., (1997) Pro. Int'l. Symp. Control. Rel. Bioact. Mater. 24:853-854, and Lam et al, (1997) Proc. Int'l. Symp. Control Rel. Bioact. Mater. 24:759-760.

If the antibodies or fragments thereof for use according to the invention are administered topically, they can be formulated in the form of an ointment, cream, transdermal patch, lotion, gel, shampoo, spray, aerosol, solution, emulsion, or other form well-known to one of skill in the art. See, e.g., Remington's Pharmaceutical Sciences and Introduction to Pharmaceutical Dosage Forms, 19th ed., Mack Pub. Co., Easton, Pa. (1995). For non-sprayable topical dosage forms, viscous to semi-solid or solid forms comprising a carrier or one or more excipients compatible with topical application and having a dynamic viscosity, in some instances, greater than water are typically employed. Suitable formulations include, without limitation, solutions, suspensions, emulsions, creams, ointments, powders, liniments, salves, and the like, which are, if desired, sterilized or mixed with auxiliary agents (e.g., preservatives, stabilizers, wetting agents, buffers, or salts) for influencing various properties, such as, for example, osmotic pressure. Other suitable topical dosage forms include sprayable aerosol preparations wherein the active ingredient, in some instances, in combination with a solid or liquid inert carrier, is packaged in a mixture with a pressurized volatile (e.g., a gaseous propellant, such as freon) or in a squeeze bottle. Moisturizers or humectants can also be added to pharmaceutical compositions and dosage forms if desired. Examples of such additional ingredients are well-known in the art.

If the compositions comprising antibodies or fragments thereof are administered intranasally, it can be formulated in an aerosol form, spray, mist or in the form of drops. In particular, prophylactic or therapeutic agents for use according to the present invention can be conveniently delivered in the form of an aerosol spray presentation from pressurized packs or a nebuliser, with the use of a suitable propellant (e.g., dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, e.g., gelatin) for use in an inhaler or insufflator may be formulated containing a powder mix of the compound and a suitable powder base such as lactose or starch.

Methods for co-administration or treatment with a second therapeutic agent, e.g., a cytokine, steroid, chemotherapeutic agent, antibiotic, or radiation, are known in the art (see, e.g., Hardman et al., (eds.) (2001) Goodman and Gilman's The Pharmacological Basis of Therapeutics, IO.sup.th ed., McGraw-Hill, New York, N.Y.; Poole and Peterson (eds.) (2001) Pharmacotherapeutics for Advanced Practice: A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%>; at least 40%>, or at least 50%.

Additional therapies (e.g., prophylactic or therapeutic agents), which can be administered in combination with the antibodies or fragments thereof for use according to the the invention may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the antibodies or fragments thereof for use according to the invention. The two or more therapies may be administered within one same patient visit.

The antibodies or fragments thereof for use according to the invention and the other therapies may be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, i.e., the cycle in order to reduce the development of resistance to one of the therapies, to avoid or reduce the side effects of one of the therapies, and/or to improve the efficacy of the therapies.

In certain embodiments, the antibodies or fragments thereof for use according to the invention can be formulated to ensure proper distribution in vivo. For example, the blood-brain barrier (BBB) excludes many highly hydrophilic compounds. To ensure that the therapeutic compounds of the disclosure cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., Ranade, (1989) J. Clin. Pharmacol. 29:685). Exemplary targeting moieties include folate or biotin (see, e.g., U.S. Pat. No. 5,416,016 to Low et al); mannosides (Umezawa et al, (1988) Biochem. Biophys. Res. Commun. 153: 1038); antibodies (Bloeman et al, (1995) FEBS Lett. 357: 140; Owais et al., (1995) Antimicrob. Agents Chemother. 39: 180); surfactant protein A receptor (Briscoe et al, (1995) Am. J. Physiol. 1233: 134); p 120 (Schreier et al, (1994) J. Biol. Chem. 269:9090); see also K. Keinanen; M. L. Laukkanen (1994) FEBS Lett. 346: 123; J. J. Killion; I. J. Fidler (1994) Immunomethods 4:273.

The disclosure provides protocols for the administration of pharmaceutical composition comprising antibodies or fragments thereof for use according to the invention alone or in combination with other therapies to a subject in need thereof. The therapies (e.g., prophylactic or therapeutic agents) of the combination therapies of the present disclosure can be administered concomitantly or sequentially to a subject. The therapy (e.g., prophylactic or therapeutic agents) of the combination therapies of the present disclosure can also be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time and repeating this sequential administration, i.e., the cycle, in order to reduce the development of resistance to one of the therapies (e.g., agents) to avoid or reduce the side effects of one of the therapies (e.g., agents), and/or to improve, the efficacy of the therapies.

The therapies (e.g., prophylactic or therapeutic agents) of the combination therapies of the disclosure can be administered to a subject concurrently. The term "concurrently" is not limited to the administration of therapies (e.g., prophylactic or therapeutic agents) at exactly the same time, but rather it is meant that a pharmaceutical composition comprising antibodies or fragments thereof for use according to the invention are administered to a subject in a sequence and within a time interval such that the antibodies for use according to the invention can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (e.g., prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (e.g., prophylactic or therapeutic agents) are administered to a within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration. The invention having been fully described, it is further illustrated by the following examples and claims, which are illustrative and are not meant to be further limiting.

In one example, the disclosure includes a method of treating low-grade serous ovarian cancer in a subject, the method comprising administering to the subject a therapeutically effective amount of an antibody or fragment thereof that specifically binds to HER3, e.g., as shown in Table 1. In one example, the method includes administering an antibody that comprises HCDR1, HCDR2, and HCDR3 of MOR10703 to a patient having low-grade serous ovarian cancer. In yet another example, the method includes administering an antibody that comprises HCDR1, HCDR2, and HCDR3 of MOR10703 at a dose between 10-50 mg/kg to a patient having low-grade serous ovarian cancer. In still yet another example, the method includes administering an antibody that comprises HCDR1, HCDR2, and HCDR3 of MOR10703 at a dose (e.g., once per week, once per two weeks, once per three weeks or once a month) between 10-50 mg/kg to a patient having low-grade serous ovarian cancer. In still yet another example, the method includes administering an antibody that comprises HCDR1, HCDR2, and HCDR3 of MOR10703 at a weekly dose of 40 mg/kg to a patient having low-grade serous ovarian cancer.

### EXAMPLES

HER3 blockade with MOR10703 or RNAi can inhibit proliferation in a subset of primary ovarian cancer cells.

MOR10703, a monoclonal anti-ectodomain HER3 antibody, was added to a panel of primary ovarian cancer cell strains, each obtained from ascites derived from a single ovarian cancer- bearing patient. Subsequent cell proliferation and viability were assessed in an ATP-based CellTiter-Glo assay performed after 6 days of continued antibody exposure (Figure 1). Although the majority of the 21 primary ovarian cancer cell packs tested were found to contain HER3 phosphorylated at the Y1289 position, as determined by Western blotting, only four revealed evidence of sensitivity to MOR10703 exposure.

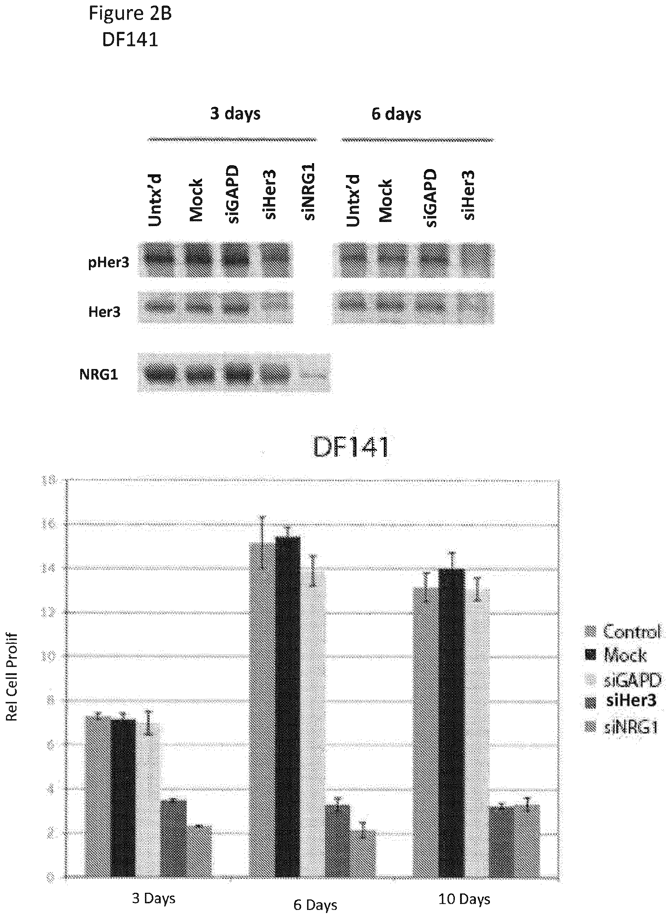

To determine whether the effect observed with MOR10703 was mediated by interference with the NRG1/HER3 signaling circuit, we performed RNA-interference (RNAi) on two of the sensitive cell packs, DF76 and DF141, with well-validated (Sheng et al.) small interfering RNAs (siRNAs) that target NRG1 and HER3 (Figure 2). Both siNRG1 and siHER3, tested independently, affected cell proliferation/viability as compared to mock transfection or control siRNA. These results suggest that the observed effects of MOR10703 in these cells are a result of interference with activated HER3 function.

Sensitivity to HER3 blockade is present in primary low-grade serous ovarian cancer cells, but not in low-grade serous ovarian cancer cell lines.

It was investigated whether the primary cell packs with observed sensitivity to HER3 antibody share any common characteristics that might help to further define a subset of ovarian cancer where HER3-directed therapy is most effective. Although DF76 and DF141 both contained activated HER3, this characteristic was also present in cell packs which were not sensitive to MOR10703 (Table 1). However, histopathological characterization of both of these sensitive cell packs, as well as of the two other sensitive cell packs, DF192 and DF225, was that of Grade 1 or Grade 2 serous ovarian cancer, consistent with a diagnosis of low-grade serous ovarian carcinoma. No MOR10703-sensitivity was observed among any of the other cell packs, the majority of which were characterized as high-grade serous ovarian carcinomas.

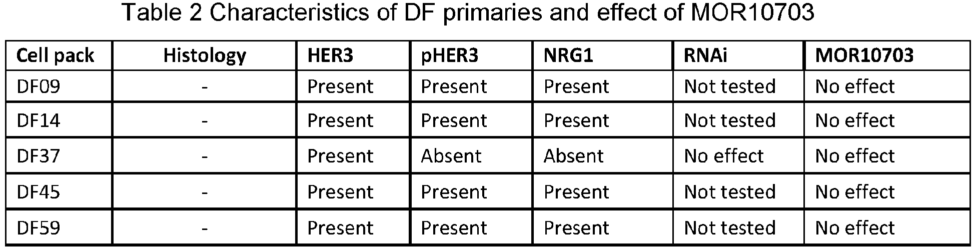

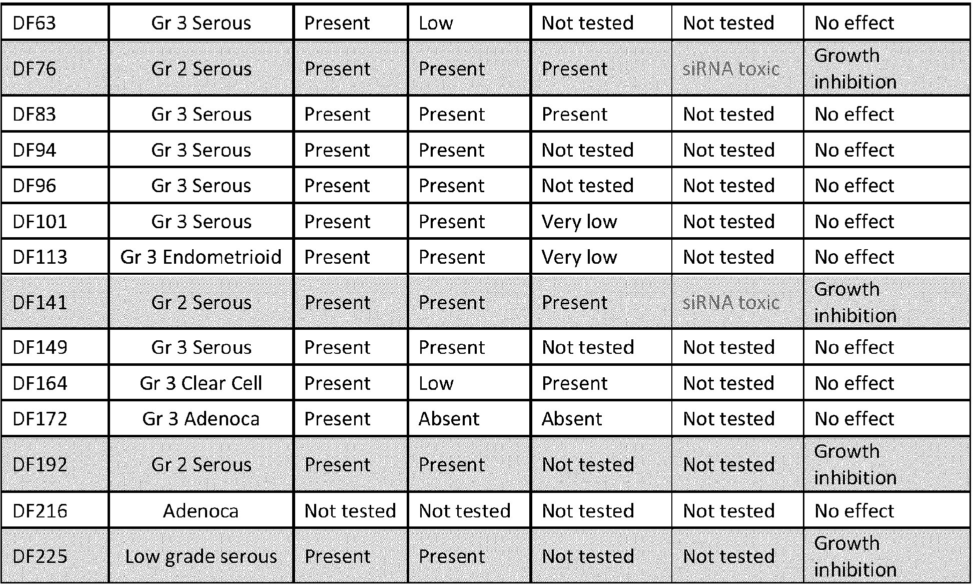

Given these findings, it was investigated whether low-grade serous ovarian carcinoma cell lines are also sensitive to HER3-pathway interference. Three low-grade serous ovarian cancer cell lines were identified from the literature. One of them, HEY, revealed low or no expression of HER3, lacked detectable, activated HER3, and was insensitive to MOR10703 (Figure 3). The other two cell lines, MPSC1 and HOC-7, expressed HER3 that was activated (at lower levels in HOC-7 than in MPSC1), but they, too, were insensitive to MOR10703 (Figure 3).

Differences in tumor genomic sequences were investigated to determine if sequence differences could explain the differences in MOR10703 sensitivity between the primary low-grade serous ovarian cancer cells and the low-grade serous ovarian cancer cell lines that were tested. Next-generation sequencing of approximately 700 genes in which tumor -associated mutations have been detected was performed. While HEY, MPSC1, and HOC-7 all revealed mutations in BRAF or KRAS (HEY: BRAF G464E, KRAS G12D; MPSC1: V600L; HOC-7: KRAS G12A), no mutations in either of these genes was detected in the primary low grade samples. No additional mutations clearly differentiated the primary low-grade serous ovarian cancer responder strains from the non-responder low-grade serous cell lines.

Effects of combined EGFR family pathway blockade on primary low-grade serous ovarian cancer cells.

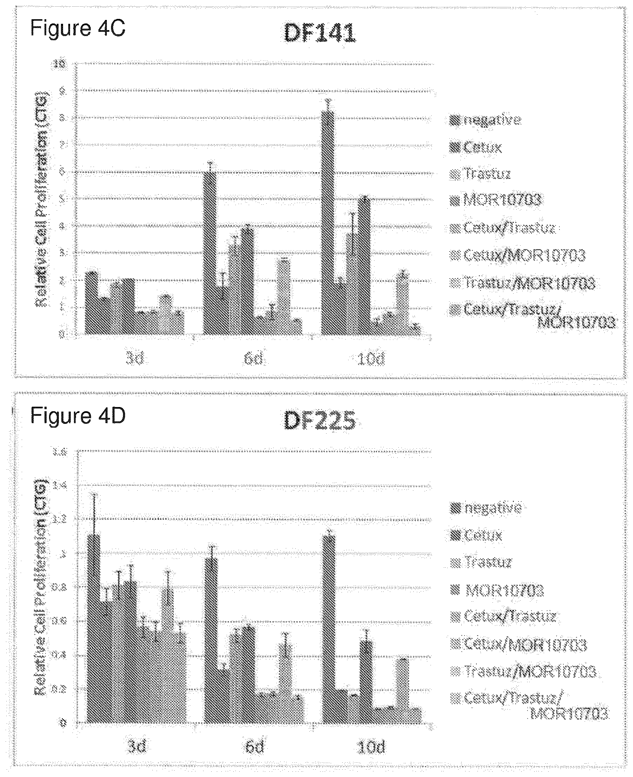

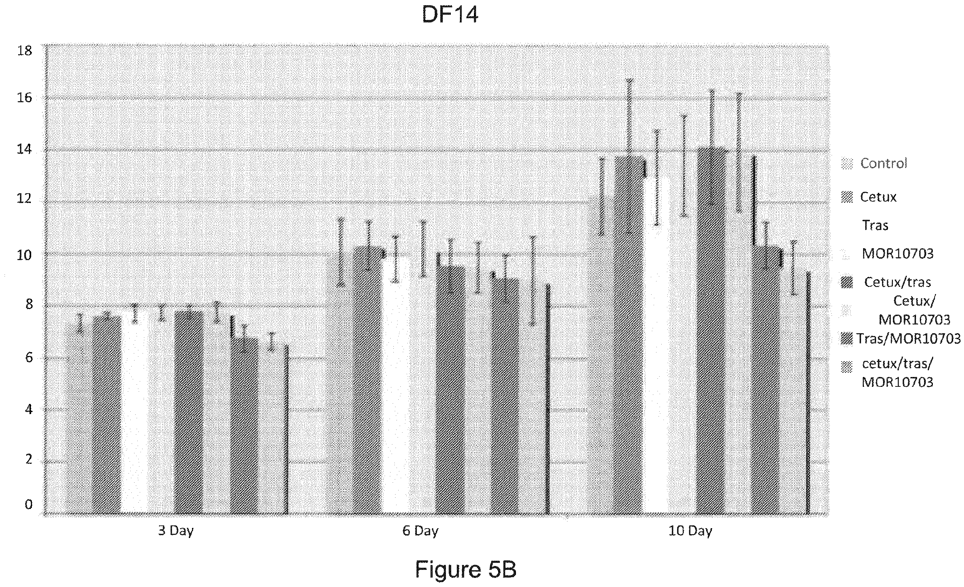

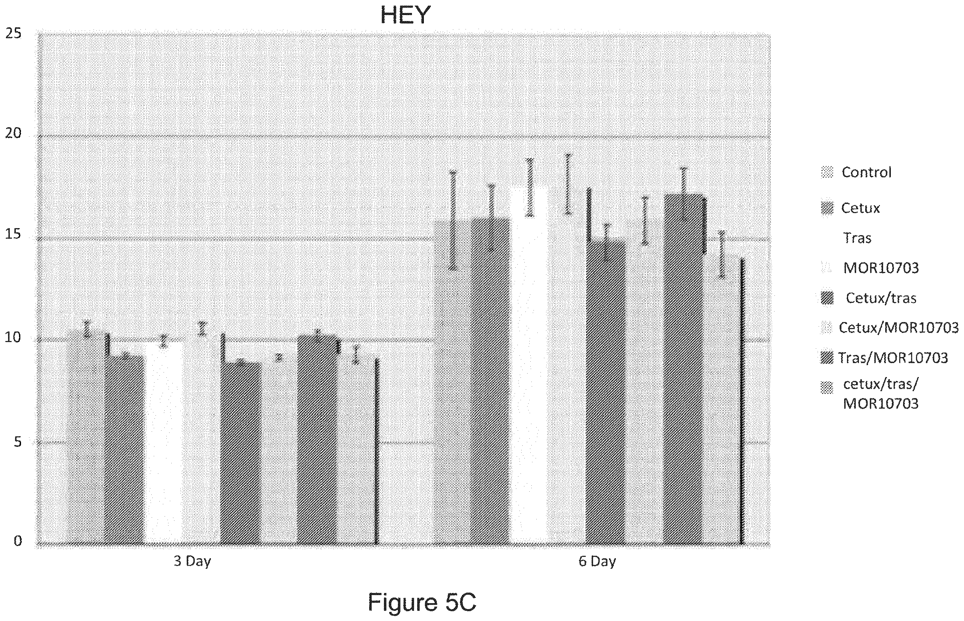

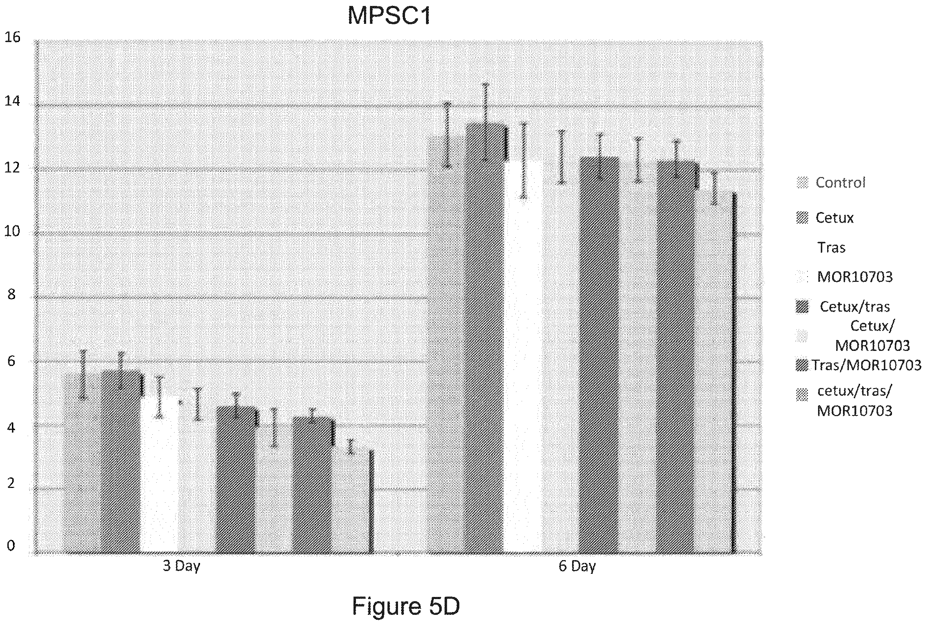

Given the reports that increased signaling of certain EGFR family members can compensate for loss of signaling by other family members (Sergina et al., 2007; Engelman et al., 2007), inhibition of function of EGFR family members other than HER3 were investigate to determine whether inhibition might compromise cell proliferation in MOR10703-sensitive cells. The primary low-grade serous ovarian cancer responder strains were, therefore, exposed to the anti-EGFR monoclonal antibody cetuximab and the anti-Her2 monoclonal antibody trastuzumab, either alone, in combination with one another, or in combination with MOR10703 (Figure 4). All four primary low-grade serous cell strains (DF76, DF141, DF192, and DF225) demonstrated significant sensitivity to anti-EGFR family inhibition either with an anti-EGFR antibody alone, with an anti-HER2 antibody alone or with an anti-HER3 antibody alone, and also with increased effect seen when the antibodies were combined. By contrast, sensitivity to EGFR family antibodies was absent in two high grade primary ovarian cancer cells (DF09, DF14) and in the above-noted low-grade serous ovarian cancer cell lines (Figure 5).

The results show that a specific subtype of human ovarian cancer, the low-grade serous subtype, is more sensitive to HER3 pathway interference by anti-HER3 monoclonal antibodies. In a collection of 21 primary ovarian cancer cell strains, four showed sensitivity to the anti-HER3 monoclonal antibody MOR10703. All four of these primary cell strains were derived from Grade 1 or Grade 2 ovarian cancers consistent with a low-grade serous ovarian cancer cell type. Transduction of siRNA directed at NRG1 and HER3 in two of these primary cell strains also resulted in inhibition of cell proliferation, supporting the hypothesis that the effects observed with MOR10703 in these cells are mediated by interference with the NRG1/HER3 signaling circuit. Low-grade serous ovarian cancer cells also have been shown to have a heightened sensitivity to monoclonal antibodies directed at other members of the EGFR family, and that the effects of the various anti-EGFR family monoclonal antibodies can be additive.

Low-grade serous ovarian cancer is a challenging form of ovarian cancer. Although less common than the more standard high-grade serous ovarian cancers, low-grade serous tumors can, nonetheless, be lethal and have been demonstrated to be less responsive to standard ovarian cancer chemotherapies (Gershenson, Sun, Lu, Obstet Gynecol, 2006, 361). Targeted therapy has therefore been an area of active interest in these tumors.

Prior work has suggested that a significant percentage of low-grade serous cancers harbor KRAS or BRAF mutations (Singer, Oldt, et al., JNCI, 2003, 484), and a Phase 2 trial of the MEK 1/2 inhibitor selumetinib in women with recurrent low-grade serous ovarian cancer demonstrated low level activity, with a response rate of 15% and a median progression-free survival (PFS) of 11 months (Farley, Brady et al., Lancet Oncol 2013, p134). In addition, 63% of patients experienced a PFS of greater than 6 months in duration. Additional trials of such targeted therapies are currently underway, including a trial comparing the MEK-inhibitor, MEK162, to standard chemotherapy (NCT01849874, clinicaltrials.gov) and a trial comparing the combination of the MEK inhibitor, pimasertib, with the PI3K inhibitor SAR245409 to pimasertib alone (NCT01936363, clinicaltrials.gov).

The HER3 pathway may be another potential target of interest in low-grade serous ovarian cancers. One discrepancy in our results was that none of the three low-grade serous ovarian cancer cell lines we could obtain showed any sensitivity to MOR10703 or other antibodies targeting the EGFR family while all four of the primary ovarian cancer cell strains did demonstrate sensitivity.

Interestingly, upon molecular profiling, we noted that each of the low grade cell lines contained a mutation in KRAS, BRAF, or both, consistent with prior published literature (Estep, PLOSOne, 2007, e1279; Pohlle-Ming Shih, Cancer Research, 2005, 1994), while in our primary cell packs, no BRAF or KRAS mutations were detected. BRAF and KRAS mutations have been documented in up to 30 to 50% of low-grade serous tumors (Farley, Lancet Oncol 2013, p134). It is possible that the survival and growth of BRAF/KRAS-mutated low-grade tumors is driven by alternative pathways that operate downstream and thereby nullify any independent HER3 signaling effects, rendering BRAF/KRAS-mutated tumors less likely to respond to HER3 pathway interference.

The EGFR family consists of four closely related family members: EGFR, Her2, HER3, and ErbB4, and signaling through these kinases has canonically been described through ligand-activated homo- and hetero-dimers between the family members (Yarden, 2001). Furthermore, when signaling through a specific family member is blocked (e.g., through small molecule inhibitors or blockading antibodies), upregulation of the activity of other family members has been described as a potential resistance mechanism (Sergina; Engelman; Garrett, PNAS 2011, 5021). This potential for signaling through the different EGFR family members to compensate for the blockade of any given family member may underlie the observation that the primary low-grade serous cell strains are more sensitive to combined blockade of the EGFR family members. From a clinical perspective, this might suggest that selected multi-kinase inhibition that encompasses all of the EGFR family members might have greater effect on these tumors than HER3-directed therapy alone. A Phase 2 trial of a pan-ErbB family inhibitor, CI-1033, demonstrated minimal activity in platinum-refractory patients (Campos et al., 2005); however, this trial encompassed all ovarian cancer subtypes and the activity of such a molecule on low-grade serous ovarian cancers remains unknown.

Reagents and antibodies. Antibodies for Western blotting directed at pHer3 (Y1289), pAKT (T308), and total AKT were obtained from Cell Signaling Technology. Anti-HER3 C-terminal region Ab (clone 2F12) for Western blotting was obtained from Lab Vision. Anti-NRG1 Ab for Western blotting was obtained from Santa Cruz (sc-348). The anti-HER3 monoclonal antibody MOR10703 was obtained from Novartis, Inc. Clinical grade cetuximab (ImClone LLC, New York, NY) and trastuzumab (Genentech, Inc., South San Francisco, CA) were purchased for in vitro experiments.

Cell lines. Low-grade serous ovarian cancer cell lines were identified by literature search. HEY cells were reported to have initially been derived from a moderately-differentiated papillary cystadenocarcinoma of the ovary (Buick, Pullano, Trent, Cancer Res, 1985, 3668). MPSC1 cells were established from a low-grade serous carcinoma (Pohl) and generously provided by Dr. le-Ming Shih at Johns Hopkins University. HOC-7 cells were derived from a patient with well-differentiated serous adenocarcinoma of the ovary (Buick) and were generously provided by Dr. Kwong-Kwok Wong at MD Anderson Cancer Center and Dr. Louis Dubeau at University of Southern California. HEY cells were cultured in RPMI 1640 (Gibco) supplemented with 10% fetal bovine serum (FBS, Invitrogen) and 1x L-glutamine (Gibco). MPSC1 cells were cultured in RPMI 1640 with 5% FBS and 1x L-glutamine. HOC7 cells were cultured in DMEM (Gibco) with 10% FBS and 1x L-glutamine. Cells were incubated at 37°C in a 5% CO2-containing atmosphere.

Primary cells. Primary cells were obtained from patients with advanced ovarian cancer at Dana-Farber Cancer Institute (DFCI) who underwent paracentesis for malignant ascites or debulking surgery under protocols approved by the Dana-Farber / Harvard Cancer Center Institutional Review Board (DFHCC IRB) and the Partners Human Research Committee. Consent from patients was obtained as per IRB guidelines. Ascites fluid was processed and tumor cells were purified according to a previously described protocol (Clauss et al., 2009). Primary cell strains used for in vitro experiments were grown in RPMI 1640 with 10% FBS and 1x anti-anti (Invitrogen). Cells were incubated at 37°C in a 5% CO2-containing atmosphere.

Assessment of cell proliferation. Primary cells were plated at a density of approximately 30% and cell lines were plated at 3000 to 5000 cells per well in 96-well plates. MOR10703, cetuximab, and trastuzumab were added at a concentration of 1:1000 from their starting concentrations (10 mg/ml, 2 mg/ml, and 21 mg/ml, respectively). Media and antibodies were exchanged every 3 days after plating. Cell proliferation and growth was assessed using CellTiter-Glo (Promega, Madison, WI), and relative growth was compared to a baseline measurement taken on the day of plating.

RNA interference. siRNA against NRG1 was performed with NRG1-9, purchased from Qiagen. siRNA against HER3 was performed with the siRNA sequence AAGAGGATGTCAACGGTTA (SEQ ID NO: 2). Control siRNAs were obtained from Dharmacon. RNAi was performed with serial siRNAs using Lipofectamine ™ RNAi Max (Invitrogen). Cells lines were plated at 1000 to 3000 cells per well in 96-well and 6-well plates. Cells were transfected serially on days zero (day of plating), two, and four with 5ul Lipofectamine™ RNAiMax and 100pmol of siRNA for 6-well plates. Transfection volumes were scaled down for 96-well plates at 1:10 on the day of plating, and 1:25 on days two and four. Media was changed after each transfection.

Cell extracts and Western blotting. SDS-free RIPA buffer (Boston Bioproducts, Ashland, MA) containing freshly added 50mM NaF, 0.4 mM sodium orthovanadate, and complete protease inhibitor cocktail tablets (Roche) was used for extract preparation. For Western blotting, immunoprecipitates were washed three times with lysis buffer, resolved by SDS gel electrophoresis, and the separated proteins blotted onto nitrocellulose membranes that were developed with appropriate antibodies.

Next-generation sequencing. Next-generation sequencing was performed by the Center for Cancer Genome Discovery using OncoPanel version 2 (OPv2). DNA was isolated from ovarian cancer cell lines and DF cell strains using the Qiagen DNeasy kit (Qiagen).

## Claims

1. An agent for use in treating low-grade serous ovarian cancer, the agent comprising an antibody or fragment thereof that specifically binds to an Epidermal Growth Factor Receptor (EGFR) family member, the antibody or fragment thereof comprising:
a heavy chain variable region CDR1 of SEQ ID NO: 2; CDR2 of SEQ ID NO: 3; CDR3 of SEQ ID NO: 4; a light chain variable region CDR1 of SEQ ID NO: 5; CDR2 of SEQ ID NO: 6; and CDR3 of SEQ ID NO: 7; or
a heavy chain variable region CDR1 of SEQ ID NO: 20; CDR2 of SEQ ID NO: 21; CDR3 of SEQ ID NO: 22; a light chain variable region CDR1 of SEQ ID NO: 23; CDR2 of SEQ ID NO: 24; and CDR3 of SEQ ID NO: 25; or
a heavy chain variable region CDR1 of SEQ ID NO: 38; CDR2 of SEQ ID NO: 39; CDR3 of SEQ ID NO: 40; a light chain variable region CDR1 of SEQ ID NO: 41; CDR2 of SEQ ID NO: 42; and CDR3 of SEQ ID NO: 43; or
a heavy chain variable region CDR1 of SEQ ID NO: 56; CDR2 of SEQ ID NO: 57; CDR3 of SEQ ID NO: 58; a light chain variable region CDR1 of SEQ ID NO: 59; CDR2 of SEQ ID NO: 60; and CDR3 of SEQ ID NO: 61; or
a heavy chain variable region CDR1 of SEQ ID NO: 74; CDR2 of SEQ ID NO: 75; CDR3 of SEQ ID NO: 76; a light chain variable region CDR1 of SEQ ID NO: 77; CDR2 of SEQ ID NO: 78; and CDR3 of SEQ ID NO: 79; or
a heavy chain variable region CDR1 of SEQ ID NO: 92; CDR2 of SEQ ID NO: 93; CDR3 of SEQ ID NO: 94; a light chain variable region CDR1 of SEQ ID NO: 95; CDR2 of SEQ ID NO: 96; and CDR3 of SEQ ID NO: 97; or
a heavy chain variable region CDR1 of SEQ ID NO: 110; CDR2 of SEQ ID NO: 111; CDR3 of SEQ ID NO: 112; a light chain variable region CDR1 of SEQ ID NO: 113; CDR2 of SEQ ID NO: 114; and CDR3 of SEQ ID NO: 115; or
a heavy chain variable region CDR1 of SEQ ID NO: 128; CDR2 of SEQ ID NO: 129; CDR3 of SEQ ID NO: 130; a light chain variable region CDR1 of SEQ ID NO: 131; CDR2 of SEQ ID NO: 132; and CDR3 of SEQ ID NO: 133; or
a heavy chain variable region CDR1 of SEQ ID NO: 146; CDR2 of SEQ ID NO: 147; CDR3 of SEQ ID NO: 148; a light chain variable region CDR1 of SEQ ID NO: 149; CDR2 of SEQ ID NO: 150; and CDR3 of SEQ ID NO: 151; or
a heavy chain variable region CDR1 of SEQ ID NO: 164; CDR2 of SEQ ID NO: 165; CDR3 of SEQ ID NO: 166; a light chain variable region CDR1 of SEQ ID NO: 167; CDR2 of SEQ ID NO: 168; and CDR3 of SEQ ID NO: 169; or
a heavy chain variable region CDR1 of SEQ ID NO: 182; CDR2 of SEQ ID NO: 183; CDR3 of SEQ ID NO: 184; a light chain variable region CDR1 of SEQ ID NO: 185; CDR2 of SEQ ID NO: 186; and CDR3 of SEQ ID NO: 187; or
a heavy chain variable region CDR1 of SEQ ID NO: 200; CDR2 of SEQ ID NO: 201; CDR3 of SEQ ID NO: 202; a light chain variable region CDR1 of SEQ ID NO: 203; CDR2 of SEQ ID NO: 204; and CDR3 of SEQ ID NO: 205; or
a heavy chain variable region CDR1 of SEQ ID NO: 218; CDR2 of SEQ ID NO: 219; CDR3 of SEQ ID NO: 220; a light chain variable region CDR1 of SEQ ID NO: 221; CDR2 of SEQ ID NO: 222; and CDR3 of SEQ ID NO: 223; or
a heavy chain variable region CDR1 of SEQ ID NO: 236; CDR2 of SEQ ID NO: 237; CDR3 of SEQ ID NO: 238; a light chain variable region CDR1 of SEQ ID NO: 239; CDR2 of SEQ ID NO: 240; and CDR3 of SEQ ID NO: 241; or
a heavy chain variable region CDR1 of SEQ ID NO: 254; CDR2 of SEQ ID NO: 255; CDR3 of SEQ ID NO: 256; a light chain variable region CDR1 of SEQ ID NO: 257; CDR2 of SEQ ID NO: 258; and CDR3 of SEQ ID NO: 259; or
a heavy chain variable region CDR1 of SEQ ID NO: 272; CDR2 of SEQ ID NO: 273; CDR3 of SEQ ID NO: 274; a light chain variable region CDR1 of SEQ ID NO: 275; CDR2 of SEQ ID NO: 276; and CDR3 of SEQ ID NO: 277; or
a heavy chain variable region CDR1 of SEQ ID NO: 290; CDR2 of SEQ ID NO: 291; CDR3 of SEQ ID NO: 292; a light chain variable region CDR1 of SEQ ID NO: 293; CDR2 of SEQ ID NO: 294; and CDR3 of SEQ ID NO: 295; or
a heavy chain variable region CDR1 of SEQ ID NO: 308; CDR2 of SEQ ID NO: 309; CDR3 of SEQ ID NO: 310; a light chain variable region CDR1 of SEQ ID NO: 311; CDR2 of SEQ ID NO: 312; and CDR3 of SEQ ID NO: 313; or
a heavy chain variable region CDR1 of SEQ ID NO: 326; CDR2 of SEQ ID NO: 327; CDR3 of SEQ ID NO: 328; a light chain variable region CDR1 of SEQ ID NO: 329; CDR2 of SEQ ID NO: 330; and CDR3 of SEQ ID NO: 331; or
a heavy chain variable region CDR1 of SEQ ID NO: 344; CDR2 of SEQ ID NO: 345; CDR3 of SEQ ID NO: 346; a light chain variable region CDR1 of SEQ ID NO: 347; CDR2 of SEQ ID NO: 348; and CDR3 of SEQ ID NO: 349; or
a heavy chain variable region CDR1 of SEQ ID NO: 362; CDR2 of SEQ ID NO: 363; CDR3 of SEQ ID NO: 364; a light chain variable region CDR1 of SEQ ID NO: 365; CDR2 of SEQ ID NO: 366; and CDR3 of SEQ ID NO: 367.

2. The agent for use according to claim 1, wherein the antibody or fragment thereof comprises a heavy chain variable region CDR1 of SEQ ID NO: 128; CDR2 of SEQ ID NO: 129; CDR3 of SEQ ID NO: 130; and a light chain variable region CDR1 of SEQ ID NO: 131; CDR2 of SEQ ID NO: 132; and CDR3 of SEQ ID NO: 133.

3. The agent for use according to claim 1 or 2, wherein the antibody or fragment thereof comprises a variable heavy chain (VH) sequence of SEQ ID NO: 141 and a variable light chain (VL) sequence of SEQ ID NO: 140.

4. The agent for use according to any one of claims 1-3, wherein the antibody or fragment thereof comprises a heavy chain (HC) sequence of SEQ ID NO: 145 and a light chain (LC) sequence of SEQ ID NO: 144.

5. The agent for use according to any one of claims 1-4, wherein the EGFR family member comprises HER3, further comprising an additional agent selected from cetuximab or trastuzumab or cetuximab and trastuzumab.

6. The agent for use according to any one of claims 1-5, wherein the antibody or fragment thereof is administered by a route selected from the group consisting of oral, subcutaneous, intraperitoneal, intramuscular, intracerebroventricular, intraparenchymal, intrathecal, intracranial, buccal, mucosal, nasal, and rectal administration.

7. The agent for use according to any one of claims 1-6, wherein the antibody or fragment is formulated into a pharmaceutical composition comprising a physiologically acceptable carrier, excipient, or diluent.

8. The agent for use according to claim 7, further comprising an additional therapeutic agent.

9. The agent for use according to claim 8, wherein the additional therapeutic agent is selected from the group consisting of an HER1 inhibitor, a HER2 inhibitor, a HER3 inhibitor, a HER4 inhibitor, an mTOR inhibitor and a PI3 Kinase inhibitor.

10. The agent for use according to claim 9, wherein the additional therapeutic agent is a HER1 inhibitor selected from the group consisting of Matuzumab (EMD72000), Cetuximab, Panitumumab, mAb 806, Nimotuzumab, Gefitinib, CI-1033 (PD183805), Lapatinib (GW-572016), Lapatinib Ditosylate, Erlotinib HCL (OSI- 774), PKI-166, and N-[4-[(3-Chloro-4-fluorophenyl)amino]-7-[[(3"S")-tetrahydro-3-furanyl]oxy]- 6-quinazolinyl]-4(dimethylamino)-2-butenamide; a HER2 inhibitor selected from the group consisting of Pertuzumab, Trastuzumab, MM-111, neratinib, lapatinib or lapatinib ditosylate; a HER3 inhibitor selected from the group consisting of, MM- 121, MM- 111, IB4C3, 2DID 12 (U3 Pharma AG), AMG888 (Amgen), AV-203(Aveo), MEHD7945A (Genentech), MOR10703 (Novartis) and small molecules that inhibit HER3; and/or a HER4 inhibitor.

11. The agent for use according to claim 9, wherein the additional therapeutic agent is an mTOR inhibitor selected from the group consisting of Temsirolimus, ridaforolimus, AP23573, MK8669, everolimus.

12. The agent for use according to claim 9, wherein the additional therapeutic agent is a PI3 Kinase inhibitor selected from the group consisting of GDC 0941, BEZ235, BMK120 and BYL719.

13. The agent for use according to claim 9, wherein the additional therapeutic agent is selected from the group consisting of gemcitabine, paclitaxel, imatinib mesylate, sunitinib malate, adriamycin, daunomycin, cisplatin, etoposide, vinblastine, vincristine, and methotrexate.

## Patentansprüche

1. Agens zur Verwendung bei der Behandlung von niedriggradigem serösem Ovarialkarzinom, wobei das Agens einen Antikörper bzw. ein Fragment davon umfasst, der bzw. das an ein Mitglied der EGFR(Epidermal Growth Factor Receptor)-Familie spezifisch bindet, wobei der Antikörper bzw. das Fragment davon Folgendes umfasst:
eine Schwere-Kette-variable-Region-CDR1 unter SEQ ID NO: 2; -CDR2 unter SEQ ID NO: 3; -CDR3 unter SEQ ID NO: 4; eine Leichte-Kette-variable-Region-CDR1 unter SEQ ID NO: 5; -CDR2 unter SEQ ID NO: 6; und -CDR3 unter SEQ ID NO: 7; oder
eine Schwere-Kette-variable-Region-CDR1 unter SEQ ID NO: 20; -CDR2 unter SEQ ID NO: 21; -CDR3 unter SEQ ID NO: 22; eine Leichte-Kette-variable-Region-CDR1 unter SEQ ID NO: 23; -CDR2 unter SEQ ID NO: 24; und -CDR3 unter SEQ ID NO: 25; oder
eine Schwere-Kette-variable-Region-CDR1 unter SEQ ID NO: 38; -CDR2 unter SEQ ID NO: 39; -CDR3 unter SEQ ID NO: 40; eine Leichte-Kette-variable-Region-CDR1 unter SEQ ID NO: 41; -CDR2 unter SEQ ID NO: 42; und -CDR3 unter SEQ ID NO: 43; oder
eine Schwere-Kette-variable-Region-CDR1 unter SEQ ID NO: 56; -CDR2 unter SEQ ID NO: 57; -CDR3 unter SEQ ID NO: 58; eine Leichte-Kette-variable-Region-CDR1 unter SEQ ID NO: 59; -CDR2 unter SEQ ID NO: 60; und -CDR3 unter SEQ ID NO: 61; oder
eine Schwere-Kette-variable-Region-CDR1 unter SEQ ID NO: 74; -CDR2 unter SEQ ID NO: 75; -CDR3 unter SEQ ID NO: 76; eine Leichte-Kette-variable-Region-CDR1 unter SEQ ID NO: 77; -CDR2 unter SEQ ID NO: 78; und -CDR3 unter SEQ ID NO: 79; oder
eine Schwere-Kette-variable-Region-CDR1 unter SEQ ID NO: 92; -CDR2 unter SEQ ID NO: 93; -CDR3 unter SEQ ID NO: 94; eine Leichte-Kette-variable-Region-CDR1 unter SEQ ID NO: 95; -CDR2 unter SEQ ID NO: 96; und -CDR3 unter SEQ ID NO: 97; oder
eine Schwere-Kette-variable-Region-CDR1 unter SEQ ID NO: 110; -CDR2 unter SEQ ID NO: 111; -CDR3 unter SEQ ID NO: 112; eine Leichte-Kette-variable-Region-CDR1 unter SEQ ID NO: 113; -CDR2 unter SEQ ID NO: 114; und -CDR3 unter SEQ ID NO: 115; oder
eine Schwere-Kette-variable-Region-CDR1 unter SEQ ID NO: 128; -CDR2 unter SEQ ID NO: 129; -CDR3 unter SEQ ID NO: 130; eine Leichte-Kette-variable-Region-CDR1 unter SEQ ID NO: 131; -CDR2 unter SEQ ID NO: 132; und -CDR3 unter SEQ ID NO: 133; oder
eine Schwere-Kette-variable-Region-CDR1 unter SEQ ID NO: 146; -CDR2 unter SEQ ID NO: 147; -CDR3 unter SEQ ID NO: 148; eine Leichte-Kette-variable-Region-CDR1 unter SEQ ID NO: 149; -CDR2 unter SEQ ID NO: 150; und -CDR3 unter SEQ ID NO: 151; oder
eine Schwere-Kette-variable-Region-CDR1 unter SEQ ID NO: 164; -CDR2 unter SEQ ID NO: 165; -CDR3 unter SEQ ID NO: 166; eine Leichte-Kette-variable-Region-CDR1 unter SEQ ID NO: 167; -CDR2 unter SEQ ID NO: 168; und -CDR3 unter SEQ ID NO: 169; oder
eine Schwere-Kette-variable-Region-CDR1 unter SEQ ID NO: 182; -CDR2 unter SEQ ID NO: 183; -CDR3 unter SEQ ID NO: 184; eine Leichte-Kette-variable-Region-CDR1 unter SEQ ID NO: 185; -CDR2 unter SEQ ID NO: 186; und -CDR3 unter SEQ ID NO: 187; oder
eine Schwere-Kette-variable-Region-CDR1 unter SEQ ID NO: 200; -CDR2 unter SEQ ID NO: 201; -CDR3 unter SEQ ID NO: 202; eine Leichte-Kette-variable-Region-CDR1 unter SEQ ID NO: 203; -CDR2 unter SEQ ID NO: 204; und -CDR3 unter SEQ ID NO: 205; oder
eine Schwere-Kette-variable-Region-CDR1 unter SEQ ID NO: 218; -CDR2 unter SEQ ID NO: 219; -CDR3 unter SEQ ID NO: 220; eine Leichte-Kette-variable-Region-CDR1 unter SEQ ID NO: 221; -CDR2 unter SEQ ID NO: 222; und -CDR3 unter SEQ ID NO: 223; oder
eine Schwere-Kette-variable-Region-CDR1 unter SEQ ID NO: 236; -CDR2 unter SEQ ID NO: 237; -CDR3 unter SEQ ID NO: 238; eine Leichte-Kette-variable-Region-CDR1 unter SEQ ID NO: 239; -CDR2 unter SEQ ID NO: 240; und -CDR3 unter SEQ ID NO: 241; oder
eine Schwere-Kette-variable-Region-CDR1 unter SEQ ID NO: 254; -CDR2 unter SEQ ID NO: 255; -CDR3 unter SEQ ID NO: 256; eine Leichte-Kette-variable-Region-CDR1 unter SEQ ID NO: 257; -CDR2 unter SEQ ID NO: 258; und -CDR3 unter SEQ ID NO: 259; oder
eine Schwere-Kette-variable-Region-CDR1 unter SEQ ID NO: 272; -CDR2 unter SEQ ID NO: 273; -CDR3 unter SEQ ID NO: 274; eine Leichte-Kette-variable-Region-CDR1 unter SEQ ID NO: 275; -CDR2 unter SEQ ID NO: 276; und -CDR3 unter SEQ ID NO: 277; oder
eine Schwere-Kette-variable-Region-CDR1 unter SEQ ID NO: 290; -CDR2 unter SEQ ID NO: 291; -CDR3 unter SEQ ID NO: 292; eine Leichte-Kette-variable-Region-CDR1 unter SEQ ID NO: 293; -CDR2 unter SEQ ID NO: 294; und -CDR3 unter SEQ ID NO: 295; oder
eine Schwere-Kette-variable-Region-CDR1 unter SEQ ID NO: 308; -CDR2 unter SEQ ID NO: 309; -CDR3 unter SEQ ID NO: 310; eine Leichte-Kette-variable-Region-CDR1 unter SEQ ID NO: 311; -CDR2 unter SEQ ID NO: 312; und -CDR3 unter SEQ ID NO: 313; oder
eine Schwere-Kette-variable-Region-CDR1 unter SEQ ID NO: 326; -CDR2 unter SEQ ID NO: 327; -CDR3 unter SEQ ID NO: 328; eine Leichte-Kette-variable-Region-CDR1 unter SEQ ID NO: 329; -CDR2 unter SEQ ID NO: 330; und -CDR3 unter SEQ ID NO: 331; oder
eine Schwere-Kette-variable-Region-CDR1 unter SEQ ID NO: 344; -CDR2 unter SEQ ID NO: 345; -CDR3 unter SEQ ID NO: 346; eine Leichte-Kette-variable-Region-CDR1 unter SEQ ID NO: 347; -CDR2 unter SEQ ID NO: 348; und -CDR3 unter SEQ ID NO: 349; oder
eine Schwere-Kette-variable-Region-CDR1 unter SEQ ID NO: 362; -CDR2 unter SEQ ID NO: 363; -CDR3 unter SEQ ID NO: 364; eine Leichte-Kette-variable-Region-CDR1 unter SEQ ID NO: 365; -CDR2 unter SEQ ID NO: 366; und -CDR3 unter SEQ ID NO: 367.

2. Agens zur Verwendung nach Anspruch 1, wobei der Antikörper bzw. das Fragment davon eine Schwere-Kette-variable-Region-CDR1 unter SEQ ID NO: 128; -CDR2 unter SEQ ID NO: 129; -CDR3 unter SEQ ID NO: 130; und eine Leichte-Kette-variable-Region-CDR1 unter SEQ ID NO: 131; -CDR2 unter SEQ ID NO: 132; und -CDR3 unter SEQ ID NO: 133 umfasst.

3. Agens zur Verwendung nach Anspruch 1 oder 2, wobei der Antikörper bzw. das Fragment davon eine Variable-schwere-Kette(VH)-Sequenz unter SEQ ID NO: 141 und eine Variable-leichte-Kette(VL)-Sequenz unter SEQ ID NO: 140 umfasst.

4. Agens zur Verwendung nach einem der Ansprüche 1-3, wobei der Antikörper bzw. das Fragment davon eine Schwere-Kette(HC)-Sequenz unter SEQ ID NO: 145 und eine Leichte-Kette(LC)-Sequenz unter SEQ ID NO: 144 umfasst.

5. Agens zur Verwendung nach einem der Ansprüche 1-4, wobei das Mitglied der EGFR-Familie HER3 umfasst, ferner umfassend ein zusätzliches Agens ausgewählt aus Cetuximab oder Trastuzumab oder Cetuximab und Trastuzumab.

6. Agens zur Verwendung nach einem der Ansprüche 1-5, wobei der Antikörper bzw. das Fragment davon über einen Weg verabreicht wird, der ausgewählt ist aus der Gruppe bestehend aus oraler, subkutaner, intraperitonealer, intramuskulärer, intracerebroventrikulärer, intraparenchymaler, intrathekaler, intrakranialer, bukkaler, mukosaler, nasaler und rektaler Verabreichung.

7. Agens zur Verwendung nach einem der Ansprüche 1-6, wobei der Antikörper bzw. das Fragment zu einer pharmazeutischen Zusammensetzung formuliert wird, die einen physiologisch akzeptablen Träger, Exzipienten bzw. ein physiologisch akzeptables Verdünnungsmittel umfasst.

8. Agens zur Verwendung nach Anspruch 7, ferner umfassend ein zusätzliches Therapeutikum.

9. Agens zur Verwendung nach Anspruch 8, wobei das zusätzliche Therapeutikum ausgewählt ist aus der Gruppe bestehend aus einem HER1-Inhibitor, einem HER2-Inhibitor, einem HER3-Inhibitor, einem HER4-Inhibitor, einem mTOR-Inhibitor und einem PI3-Kinase-Inhibitor.

10. Agens zur Verwendung nach Anspruch 9, wobei es sich bei dem zusätzlichen Therapeutikum um einen HER1-Inhibitor ausgewählt aus der Gruppe bestehend aus Matuzumab (EMD72000), Cetuximab, Panitumumab, mAb 806, Nimotuzumab, Gefitinib, CI-1033 (PD183805), Lapatinib (GW-572016), Lapatinib-Ditosylat, Erlotinib-HCL (OSI-774), PKI-166 und N-[4-[(3-Chlor-4-fluorphenyl)amino]-7-[[(3"S")-tetrahydro-3-furanyl]oxy]-6-chinazolinyl]-4(dimethylamino)-2-butenamid, einen HER2-Inhibitor ausgewählt aus der Gruppe bestehend aus Pertuzumab, Trastuzumab, MM-111, Neratinib, Lapatinib oder Lapatinib-Ditosylat; einen HER3-Inhibitor ausgewählt aus der Gruppe bestehend aus MM-121, MM-111, IB4C3, 2DID 12 (U3 Pharma AG), AMG888 (Amgen), AV-203 (Aveo), MEHD7945A (Genentech), MOR10703 (Novartis) und Kleinmolekülen, die HER3 hemmen; und/oder einen HER4-Inhibitor handelt.

11. Agens zur Verwendung nach Anspruch 9, wobei es sich bei dem zusätzlichen Therapeutikum um einen mTOR-Inhibitor ausgewählt aus der Gruppe bestehend aus Temsirolimus, Ridaforolimus, AP23573, MK8669, Everolimus handelt.

12. Agens zur Verwendung nach Anspruch 9, wobei es sich bei dem zusätzlichen Therapeutikum um einen PI3-Kinase-Inhibitor ausgewählt aus der Gruppe bestehend aus GDC 0941, BEZ235, BMK120 und BYL719 handelt.

13. Agens zur Verwendung nach Anspruch 9, wobei das zusätzliche Therapeutikum ausgewählt ist aus der Gruppe bestehend aus Gemcitabin, Paclitaxel, Imatinibmesylat, Sunitinibmalat, Adriamycin, Daunomycin, Cisplatin, Etoposid, Vinblastin, Vincristin und Methotrexat.

## Revendications

1. Agent pour utilisation dans le traitement d'un cancer séreux de l'ovaire de bas grade, l'agent comprenant un anticorps ou un fragment de celui-ci qui se lie spécifiquement à un membre de la famille de récepteurs de facteur de croissance épidermique (EGFR), l'anticorps ou le fragment de celui-ci comprenant :
une région variable de chaîne lourde CDR1 de SEQ ID NO : 2 ; CDR2 de SEQ ID NO : 3 ; CDR3 de SEQ ID NO : 4 ; une région variable de chaîne légère CDR1 de SEQ ID NO : 5 ; CDR2 de SEQ ID NO : 6 ; et CDR3 de SEQ ID NO : 7 ; ou
une région variable de chaîne lourde CDR1 de SEQ ID NO : 20 ; CDR2 de SEQ ID NO : 21 ; CDR3 de SEQ ID NO : 22 ;
une région variable de chaîne légère CDR1 de SEQ ID NO : 23 ; CDR2 de SEQ ID NO : 24 ; et CDR3 de SEQ ID NO : 25 ; ou
une région variable de chaîne lourde CDR1 de SEQ ID NO : 38 ; CDR2 de SEQ ID NO : 39 ; CDR3 de SEQ ID NO : 40 ;
une région variable de chaîne légère CDR1 de SEQ ID NO : 41 ; CDR2 de SEQ ID NO : 42 ; et CDR3 de SEQ ID NO : 43 ; ou
une région variable de chaîne lourde CDR1 de SEQ ID NO : 56 ; CDR2 de SEQ ID NO : 57 ; CDR3 de SEQ ID NO : 58 ;
une région variable de chaîne légère CDR1 de SEQ ID NO : 59 ; CDR2 de SEQ ID NO : 60 ; et CDR3 de SEQ ID NO : 61 ; ou
une région variable de chaîne lourde CDR1 de SEQ ID NO : 74 ; CDR2 de SEQ ID NO : 75 ; CDR3 de SEQ ID NO : 76 ;
une région variable de chaîne légère CDR1 de SEQ ID NO : 77 ; CDR2 de SEQ ID NO : 78 ; et CDR3 de SEQ ID NO : 79 ; ou
une région variable de chaîne lourde CDR1 de SEQ ID NO : 92 ; CDR2 de SEQ ID NO : 93 ; CDR3 de SEQ ID NO : 94 ;
une région variable de chaîne légère CDR1 de SEQ ID NO : 95 ; CDR2 de SEQ ID NO : 96 ; et CDR3 de SEQ ID NO : 97 ; ou
une région variable de chaîne lourde CDR1 de SEQ ID NO : 110 ; CDR2 de SEQ ID NO : 111 ; CDR3 de SEQ ID NO : 112 ;
une région variable de chaîne légère CDR1 de SEQ ID NO : 113 ; CDR2 de SEQ ID NO : 114 ; et CDR3 de SEQ ID NO : 115 ; ou
une région variable de chaîne lourde CDR1 de SEQ ID NO : 128 ; CDR2 de SEQ ID NO : 129 ; CDR3 de SEQ ID NO : 130 ;
une région variable de chaîne légère CDR1 de SEQ ID NO : 131 ; CDR2 de SEQ ID NO : 132 ; et CDR3 de SEQ ID NO : 133 ; ou
une région variable de chaîne lourde CDR1 de SEQ ID NO : 146 ; CDR2 de SEQ ID NO : 147 ; CDR3 de SEQ ID NO : 148 ;
une région variable de chaîne légère CDR1 de SEQ ID NO : 149 ; CDR2 de SEQ ID NO : 150 ; et CDR3 de SEQ ID NO : 151 ; ou
une région variable de chaîne lourde CDR1 de SEQ ID NO : 164 ; CDR2 de SEQ ID NO : 165 ; CDR3 de SEQ ID NO : 166 ;
une région variable de chaîne légère CDR1 de SEQ ID NO : 167 ; CDR2 de SEQ ID NO : 168 ; et CDR3 de SEQ ID NO : 169 ; ou
une région variable de chaîne lourde CDR1 de SEQ ID NO : 182 ; CDR2 de SEQ ID NO : 183 ; CDR3 de SEQ ID NO : 184 ;
une région variable de chaîne légère CDR1 de SEQ ID NO : 185 ; CDR2 de SEQ ID NO : 186 ; et CDR3 de SEQ ID NO : 187 ; ou
une région variable de chaîne lourde CDR1 de SEQ ID NO : 200 ; CDR2 de SEQ ID NO : 201 ; CDR3 de SEQ ID NO : 202 ;
une région variable de chaîne légère CDR1 de SEQ ID NO : 203 ; CDR2 de SEQ ID NO : 204 ; et CDR3 de SEQ ID NO : 205 ; ou
une région variable de chaîne lourde CDR1 de SEQ ID NO : 218 ; CDR2 de SEQ ID NO : 219 ; CDR3 de SEQ ID NO : 220 ;
une région variable de chaîne légère CDR1 de SEQ ID NO : 221 ; CDR2 de SEQ ID NO : 222 ; et CDR3 de SEQ ID NO : 223 ; ou
une région variable de chaîne lourde CDR1 de SEQ ID NO : 236 ; CDR2 de SEQ ID NO : 237 ; CDR3 de SEQ ID NO : 238 ;
une région variable de chaîne légère CDR1 de SEQ ID NO : 239 ; CDR2 de SEQ ID NO : 240 ; et CDR3 de SEQ ID NO : 241 ; ou
une région variable de chaîne lourde CDR1 de SEQ ID NO : 254 ; CDR2 de SEQ ID NO : 255 ; CDR3 de SEQ ID NO : 256 ;
une région variable de chaîne légère CDR1 de SEQ ID NO : 257 ; CDR2 de SEQ ID NO : 258 ; et CDR3 de SEQ ID NO : 259 ; ou
une région variable de chaîne lourde CDR1 de SEQ ID NO : 272 ; CDR2 de SEQ ID NO : 273 ; CDR3 de SEQ ID NO : 274 ;
une région variable de chaîne légère CDR1 de SEQ ID NO : 275 ; CDR2 de SEQ ID NO : 276 ; et CDR3 de SEQ ID NO : 277 ; ou
une région variable de chaîne lourde CDR1 de SEQ ID NO : 290 ; CDR2 de SEQ ID NO : 291 ; CDR3 de SEQ ID NO : 292 ;
une région variable de chaîne légère CDR1 de SEQ ID NO : 293 ; CDR2 de SEQ ID NO : 294 ; et CDR3 de SEQ ID NO : 295 ; ou
une région variable de chaîne lourde CDR1 de SEQ ID NO : 308 ; CDR2 de SEQ ID NO : 309 ; CDR3 de SEQ ID NO : 310 ;
une région variable de chaîne légère CDR1 de SEQ ID NO : 311 ; CDR2 de SEQ ID NO : 312 ; et CDR3 de SEQ ID NO : 313 ; ou
une région variable de chaîne lourde CDR1 de SEQ ID NO : 326 ; CDR2 de SEQ ID NO : 327 ; CDR3 de SEQ ID NO : 328 ;
une région variable de chaîne légère CDR1 de SEQ ID NO : 329 ; CDR2 de SEQ ID NO : 330 ; et CDR3 de SEQ ID NO : 331 ; ou
une région variable de chaîne lourde CDR1 de SEQ ID NO : 344 ; CDR2 de SEQ ID NO : 345 ; CDR3 de SEQ ID NO : 346 ;
une région variable de chaîne légère CDR1 de SEQ ID NO : 347 ; CDR2 de SEQ ID NO : 348 ; et CDR3 de SEQ ID NO : 349 ; ou
une région variable de chaîne lourde CDR1 de SEQ ID NO : 362 ; CDR2 de SEQ ID NO : 363 ; CDR3 de SEQ ID NO : 364 ;
une région variable de chaîne légère CDR1 de SEQ ID NO : 365 ; CDR2 de SEQ ID NO : 366 ; et CDR3 de SEQ ID NO : 367.

2. Agent pour utilisation selon la revendication 1, dans lequel l'anticorps ou le fragment de celui-ci comprend une région variable de chaîne lourde CDR1 de SEQ ID NO : 128 ; CDR2 de SEQ ID NO : 129 ; CDR3 de SEQ ID NO : 130 ; et une région variable de chaîne légère CDR1 de SEQ ID NO : 131 ; CDR2 de SEQ ID NO : 132 ; et CDR3 de SEQ ID NO : 133.

3. Agent pour utilisation selon la revendication 1 ou 2, dans lequel l'anticorps ou le fragment de celui-ci comprend une séquence variable de chaîne lourde (VH) de SEQ ID NO : 141 et une séquence variable de chaîne légère (VL) de SEQ ID NO : 140.

4. Agent pour utilisation selon l'une quelconque des revendications 1 à 3, dans lequel l'anticorps ou le fragment de celui-ci comprend une séquence de chaîne lourde (HC) de SEQ ID NO : 145 et une séquence de chaîne légère (LC) de SEQ ID NO : 144.

5. Agent pour utilisation selon l'une quelconque des revendications 1 à 4, où le membre de la famille EGFR comprend HER3, comprenant en outre un agent supplémentaire choisi parmi le cétuximab ou le trastuzumab ou le cétuximab et le trastuzumab.

6. Agent pour utilisation selon l'une quelconque des revendications 1 à 5, dans lequel l'anticorps ou le fragment de celui-ci est administré par une voie choisie dans le groupe constitué de l'administration orale, sous-cutanée, intrapéritonéale, intramusculaire, intracérébroventriculaire, intraparenchymateuse, intrathécale, intracrânienne, buccale, transmuqueuse, nasale et rectale.

7. Agent pour utilisation selon l'une quelconque des revendications 1 à 6, où l'anticorps ou le fragment est formulé dans une composition pharmaceutique comprenant un véhicule, excipient ou diluant physiologiquement acceptable.

8. Agent pour utilisation selon la revendication 7, comprenant en outre un agent thérapeutique supplémentaire.

9. Agent pour utilisation selon la revendication 8, où l'agent thérapeutique supplémentaire est choisi dans le groupe constitué d'un inhibiteur de HER1, d'un inhibiteur de HER2, d'un inhibiteur de HER3, d'un inhibiteur de HER4, d'un inhibiteur de mTOR et d'un inhibiteur de PI3 kinase.

10. Agent pour utilisation selon la revendication 9, dans lequel l'agent thérapeutique supplémentaire est un inhibiteur de HER1 choisi dans le groupe constitué des suivants : matuzumab (EMD72000), cétuximab, panitumumab, mAb 806, nimotuzumab, géfitinib, CI-1033 (PD183805), lapatinib (GW-572016), lapatinib ditosylate, erlotinib HCL (OSI-774), PKI-166, et N-[4-[(3-chloro-4-fluorophényl)amino]-7-[[(3"S")-tétrahydro-3-furanyl]oxy]-6-quinazolinyl]-4(diméthylamino)-2-buténamide ; un inhibiteur de HER2 choisi dans le groupe constitué des suivants : pertuzumab, trastuzumab, MM-111, nératinib, lapatinib ou lapatinib ditosylate ; un inhibiteur de HER3 choisi dans le groupe constitué des suivants : MM-121, MM-111, IB4C3, 2DID 12 (U3 Pharma AG), AMG888 (Amgen), AV-203 (Aveo), MEHD7945A (Genentech), MOR10703 (Novartis) et des petites molécules qui inhibent HER3 ; et/ou un inhibiteur de HER4.

11. Agent pour utilisation selon la revendication 9, dans lequel l'agent thérapeutique supplémentaire est un inhibiteur de mTOR choisi dans le groupe constitué des suivants : temsirolimus, ridaforolimus, AP23573, MK8669, évérolimus.

12. Agent pour utilisation selon la revendication 9, dans lequel l'agent thérapeutique supplémentaire est un inhibiteur de PI3 kinase choisi dans le groupe constitué de GDC 0941, BEZ235, BMK120 et BYL719.

13. Agent pour utilisation selon la revendication 9, dans lequel l'agent thérapeutique supplémentaire est choisi dans le groupe constitué des suivants : gemcitabine, paclitaxel, imatinib mésylate, sunitinib malate, adriamycine, daunomycine, cisplatine, étoposide, vinblastine, vincristine et méthotrexate.
